# EUROPEAN PATENT APPLICATION

(11) **EP 3 928 615 A1**
(43) Date of publication of application: **29.12.2021**
(21) Application number: 20760031.3
(22) Date of filing: 21.02.2020
(51) Int. Cl.: A01H 1/00, C12N 5/14, C12N 15/82

(54) **NUCLEIC ACID FOR EDITING GENOME OF PLANT CELL AND USE THEREOF**

(30) Priority: 22.02.2019 JP 2019030971
(71) Applicant: Bio Palette Co., Ltd., Kobe-shi, Hyogo, 657-0013 (JP)
(72) Inventor: SHIMATANI, Zenpei, Kobe-shi, Hyogo 657-0013 (JP); TERADA, Rie, Nagoya-shi, Aichi 468-8502 (JP)
(74) Representative: Heubeck, Christian
(86) International application number: PCT/JP2020/006977
(87) International publication number: WO 2020/171192

(57) **Abstract**

The present invention provides a nucleic acid comprising: (1) a transposon; and (2) a sequence homologous to at least a part of a target region of a double-stranded DNA of a plant cell, wherein the transposon comprises a marker gene and a gene encoding a transposase operably linked to an inducible promoter.

SEQUENCE LISTING

<110>  Bio Palette Co., Ltd.

<120>  Nucleic acid for genome editing of plant cell and the use thereof

<130>  093006

<150>  JP 2019-030971
<151>  2019-02-22

<160>  168

<170>  PatentIn version 3.5

<210>  1
<211>  11
<212>  DNA
<213>  Artificial Sequence

<220>
<223>  terminal inverted repeat for AcTPase

<400>  1
cagggatgaa a                                                            11


<210>  2
<211>  11
<212>  DNA
<213>  Artificial Sequence

<220>
<223>  terminal inverted repeat for AcTPase

<400>  2
tagggatgaa a                                                            11


<210>  3
<211>  11
<212>  DNA
<213>  Artificial Sequence

<220>
<223>  terminal inverted repeat for AcTPase

<400>  3
gagggatgaa a                                                            11


<210>  4
<211>  11
<212>  DNA
<213>  Artificial Sequence

<220>
<223>  terminal inverted repeat for AcTPase

<400>  4
tagagatgaa a                                                            11

<210>  5
<211>  11
<212>  DNA
<213>  Artificial Sequence

<220>
<223>  terminal inverted repeat for AcTPase

<400>  5
gagctatgaa a                                                            11


<210>  6
<211>  11
<212>  DNA
<213>  Artificial Sequence

<220>
<223>  terminal inverted repeat for AcTPase

<400>  6
tttcatccct a                                                            11


<210>  7
<211>  11
<212>  DNA
<213>  Artificial Sequence

<220>
<223>  terminal inverted repeat for AcTPase

<400>  7
tttcatctga g                                                            11


<210>  8
<211>  11
<212>  DNA
<213>  Artificial Sequence

<220>
<223>  terminal inverted repeat for AcTPase

<400>  8
tttcatccct a                                                            11


<210>  9
<211>  11
<212>  DNA
<213>  Artificial Sequence

<220>
<223>  terminal inverted repeat for AcTPase

<400>  9
tttcatccct g                                                            11


<210>  10
<211>  11
<212>  DNA

<213>  Artificial Sequence

<220>
<223>  terminal inverted repeat for AcTPase

<400>  10
tttcatctct a                                                            11


<210>  11
<211>  13
<212>  DNA
<213>  Artificial Sequence

<220>
<223>  terminal inverted repeat for EnSpm/CACTA

<400>  11
cactacaaga aaa                                                          13


<210>  12
<211>  13
<212>  DNA
<213>  Artificial Sequence

<220>
<223>  terminal inverted repeat for EnSpm/CACTA

<400>  12
cactacaaca aaa                                                          13


<210>  13
<211>  13
<212>  DNA
<213>  Artificial Sequence

<220>
<223>  terminal inverted repeat for EnSpm/CACTA

<400>  13
cactacaaaa aaa                                                          13


<210>  14
<211>  13
<212>  DNA
<213>  Artificial Sequence

<220>
<223>  terminal inverted repeat for EnSpm/CACTA

<400>  14
cactataaga aaa                                                          13


<210>  15
<211>  13
<212>  DNA
<213>  Artificial Sequence

<220>

<223>  terminal inverted repeat for EnSpm/CACTA

<400>  15
cactacgcca aaa                                                          13


<210>  16
<211>  13
<212>  DNA
<213>  Artificial Sequence

<220>
<223>  terminal inverted repeat for EnSpm/CACTA

<400>  16
cactaccgga att                                                          13


<210>  17
<211>  13
<212>  DNA
<213>  Artificial Sequence

<220>
<223>  terminal inverted repeat for EnSpm/CACTA

<400>  17
ttttcttgta gtg                                                          13


<210>  18
<211>  13
<212>  DNA
<213>  Artificial Sequence

<220>
<223>  terminal inverted repeat for EnSpm/CACTA

<400>  18
ttttgttgta gtg                                                          13


<210>  19
<211>  13
<212>  DNA
<213>  Artificial Sequence

<220>
<223>  terminal inverted repeat for EnSpm/CACTA

<400>  19
   tttttttgta gtg                                                          13


<210>  20
<211>  13
<212>  DNA
<213>  Artificial Sequence

<220>
<223>  terminal inverted repeat for EnSpm/CACTA

<400>  20

ttttcttata gtg                                                          13


<210>  21
<211>  13
<212>  DNA
<213>  Artificial Sequence

<220>
<223>  terminal inverted repeat for EnSpm/CACTA

<400>  21
ttttggcgta gtg                                                          13


<210>  22
<211>  13
<212>  DNA
<213>  Artificial Sequence

<220>
<223>  terminal inverted repeat for EnSpm/CACTA

<400>  22
aattccggta gtg                                                          13


<210>  23
<211>  13
<212>  DNA
<213>  Artificial Sequence

<220>
<223>  terminal inverted repeat for PiggyBac

<400>  23
ccctagaaag ata                                                          13


<210>  24
<211>  35
<212>  DNA
<213>  Artificial Sequence

<220>
<223>  terminal inverted repeat for PiggyBac

<400>  24
ccctagaaag atagtctgcg taaaattgac gcatg                                  35


<210>  25
<211>  35
<212>  DNA
<213>  Artificial Sequence

<220>
<223>  terminal inverted repeat for PiggyBac

<400>  25
catgcgtcaa ttttacgcag actatctttc taggg                                  35

<210>  26
<211>  13
<212>  DNA
<213>  Artificial Sequence

<220>
<223>  terminal inverted repeat for PiggyBac

<400>  26
tatctttcta ggg                                                          13


<210>  27
<211>  35
<212>  DNA
<213>  Artificial Sequence

<220>
<223>  terminal inverted repeat for PiggyBac

<400>  27
catgcgtcaa ttttacgcag actatctttc taggg                                  35


<210>  28
<211>  35
<212>  DNA
<213>  Artificial Sequence

<220>
<223>  terminal inverted repeat for PiggyBac

<400>  28
ccctagaaag atagtctgcg taaaattgac gcatg                                  35


<210>  29
<211>  2118
<212>  DNA
<213>  Artificial Sequence

<220>
<223>  AcTPase 4x gene optimized for rice


<220>
<221>  CDS
<222>  (1)..(2118)

<400>  29
atg gcc atc gtg cat gag cca cag cca caa ccg caa cca cag cca gag      48
Met Ala Ile Val His Glu Pro Gln Pro Gln Pro Gln Pro Gln Pro Glu
1               5                   10                  15

cct cag cct caa cct cag cct gag cca gag gag gag gcc cca caa aag      96
Pro Gln Pro Gln Pro Gln Pro Glu Pro Glu Glu Glu Ala Pro Gln Lys
            20                  25                  30

cgc gct aag aag tgc acc tcc gac gtc tgg cag cac ttc acc aag aag      144
Arg Ala Lys Lys Cys Thr Ser Asp Val Trp Gln His Phe Thr Lys Lys
        35                  40                  45

gag atc gag gtc gag gtt gac ggc aag aag tac gtc cag gtc tgg ggc      192

Glu Ile Glu Val Glu Val Asp Gly Lys Lys Tyr Val Gln Val Trp Gly
    50                  55                  60

cac tgc aac ttc cca aac tgc aag gcc aag tac agg gcc gag ggc cat      240
His Cys Asn Phe Pro Asn Cys Lys Ala Lys Tyr Arg Ala Glu Gly His
65                  70                  75                  80

cat ggc aca tcc ggc ttt agg aac cac ctc agg acc tcc cac tct ctc      288
His Gly Thr Ser Gly Phe Arg Asn His Leu Arg Thr Ser His Ser Leu
                85                  90                  95

gtg aag ggc caa ctc tgc ctc aag tcc gag aag gac cac ggc aag gac      336
Val Lys Gly Gln Leu Cys Leu Lys Ser Glu Lys Asp His Gly Lys Asp
            100                 105                 110

atc aac ctc atc gag ccg tac aag tac gac gag gtg gtg tcc ctc aag      384
Ile Asn Leu Ile Glu Pro Tyr Lys Tyr Asp Glu Val Val Ser Leu Lys
        115                 120                 125

aag ctc cac ctc gcc atc att atg cac gag tac ccg ttc aac atc gtc      432
Lys Leu His Leu Ala Ile Ile Met His Glu Tyr Pro Phe Asn Ile Val
    130                 135                 140

gag cac gcc tac ttc gtc gag ttc gtg aag tcc ctc agg ccg cac ttc      480
Glu His Ala Tyr Phe Val Glu Phe Val Lys Ser Leu Arg Pro His Phe
145                 150                 155                 160

ccg atc aag tct aga gtg acc gcc cgc aag tac atc atg gac ctc tac      528
Pro Ile Lys Ser Arg Val Thr Ala Arg Lys Tyr Ile Met Asp Leu Tyr
                165                 170                 175

ctt gag gag aag gag aag ctc tac ggc aag ctc aag gac gtg cag tcc      576
Leu Glu Glu Lys Glu Lys Leu Tyr Gly Lys Leu Lys Asp Val Gln Ser
            180                 185                 190

agg ttc tcc acc acg atg gac atg tgg acc agc tgc cag aac aag tcc      624
Arg Phe Ser Thr Thr Met Asp Met Trp Thr Ser Cys Gln Asn Lys Ser
        195                 200                 205

tac atg tgc gtg acc atc cac tgg atc gac gac gac tgg tgc ctc cag      672
Tyr Met Cys Val Thr Ile His Trp Ile Asp Asp Asp Trp Cys Leu Gln
    210                 215                 220

aag agg atc gtc ggc ttc ttc cat gtg gcc ggc aga cat aca ggc cag      720
Lys Arg Ile Val Gly Phe Phe His Val Ala Gly Arg His Thr Gly Gln
225                 230                 235                 240

agg ctc tcc caa acc ttc acc gcg atc atg gtc aag tgg aac atc gag      768
Arg Leu Ser Gln Thr Phe Thr Ala Ile Met Val Lys Trp Asn Ile Glu
                245                 250                 255

aag aag ctg ttc gcc ctc tcc ctc gat aac gcc tcc gct aat gag gtg      816
Lys Lys Leu Phe Ala Leu Ser Leu Asp Asn Ala Ser Ala Asn Glu Val
            260                 265                 270

gcc gtg cac gac atc atc gag gac ctc cag gac acc gac tcc aac ctt      864
Ala Val His Asp Ile Ile Glu Asp Leu Gln Asp Thr Asp Ser Asn Leu
        275                 280                 285

gtg tgc gac ggc gcc ttc ttt cat gtt cgc tgc gcc tgc cac atc ctc      912
Val Cys Asp Gly Ala Phe Phe His Val Arg Cys Ala Cys His Ile Leu
    290                 295                 300

aac ctg gtt gct aag gat ggc ctc gcc gtg atc gcc ggc acc att gag 960
Asn Leu Val Ala Lys Asp Gly Leu Ala Val Ile Ala Gly Thr Ile Glu
305                 310                 315                 320

aag atc aag gcc atc gtc ctc gcc gtc aag tcc tct cca ctt cag tgg 1008
Lys Ile Lys Ala Ile Val Leu Ala Val Lys Ser Ser Pro Leu Gln Trp
                325                 330                 335

gag gag ctg atg aag tgc gcg tcc gag tgc gat ctc gac aag tcc aag 1056
Glu Glu Leu Met Lys Cys Ala Ser Glu Cys Asp Leu Asp Lys Ser Lys
            340                 345                 350

ggc atc agc tac gcc gtg tcc acc agg tgg aat agc acc tac ctc atg 1104
Gly Ile Ser Tyr Ala Val Ser Thr Arg Trp Asn Ser Thr Tyr Leu Met
        355                 360                 365

ctc cgc gac gcc ctc tac tac aag cca gcc ctc atc agg ctc aag acc 1152
Leu Arg Asp Ala Leu Tyr Tyr Lys Pro Ala Leu Ile Arg Leu Lys Thr
    370                 375                 380

tcc gat cca aga cgc tac gat gcc atc tgc cca aag gcc gag gag tgg 1200
Ser Asp Pro Arg Arg Tyr Asp Ala Ile Cys Pro Lys Ala Glu Glu Trp
385                 390                 395                 400

aag atg gcg ctc acc ctc ttc aag tgc ctg aag aag ttc ttc gac ctc 1248
Lys Met Ala Leu Thr Leu Phe Lys Cys Leu Lys Lys Phe Phe Asp Leu
                405                 410                 415

acc gag ctg ctc tcc ggc acc caa tac tct acc gcc aac ctc ttc tac 1296
Thr Glu Leu Leu Ser Gly Thr Gln Tyr Ser Thr Ala Asn Leu Phe Tyr
            420                 425                 430

aag ggc ttc tgc gag atc aag gac ctg atc gcc caa tgg tgc gtg cac 1344
Lys Gly Phe Cys Glu Ile Lys Asp Leu Ile Ala Gln Trp Cys Val His
        435                 440                 445

gag aag ttc gtg att cgc aga atg gcc gtg gcc atg agc gag aag ttt 1392
Glu Lys Phe Val Ile Arg Arg Met Ala Val Ala Met Ser Glu Lys Phe
    450                 455                 460

gag aag tac tgg aag gtg tcc aat atc gcc ctc gcg gtg gcc tgc ttc 1440
Glu Lys Tyr Trp Lys Val Ser Asn Ile Ala Leu Ala Val Ala Cys Phe
465                 470                 475                 480

ctc gat cca agg tac aag aag atc ctg atc gag ttc tac atg aag aag 1488
Leu Asp Pro Arg Tyr Lys Lys Ile Leu Ile Glu Phe Tyr Met Lys Lys
                485                 490                 495

ttt cac ggc gac tcc tac aag gtg cac gtc gac gat ttc gtg cgc gtg 1536
Phe His Gly Asp Ser Tyr Lys Val His Val Asp Asp Phe Val Arg Val
            500                 505                 510

atc cgc aag ctc tac cag ttc tac tcc tcc tgc tct cca tcc gcc cca 1584
Ile Arg Lys Leu Tyr Gln Phe Tyr Ser Ser Cys Ser Pro Ser Ala Pro
        515                 520                 525

aag acc aag acc acc acc aac gac agc atg gac gac acc ctc atg gag 1632
Lys Thr Lys Thr Thr Thr Asn Asp Ser Met Asp Asp Thr Leu Met Glu
    530                 535                 540

aac gag gac gac gag ttc cag aac tac ctc cac gag ctg aag gac tac 1680
Asn Glu Asp Asp Glu Phe Gln Asn Tyr Leu His Glu Leu Lys Asp Tyr
545                 550                 555                 560

gac cag gtc gag agc aac gag ctg gac aag tac atg tcc gag ccg ctc 1728
Asp Gln Val Glu Ser Asn Glu Leu Asp Lys Tyr Met Ser Glu Pro Leu
                565                 570                 575

ttg aag cac tcc ggc cag ttc gat atc ctc tca tgg tgg cgc ggc aga 1776
Leu Lys His Ser Gly Gln Phe Asp Ile Leu Ser Trp Trp Arg Gly Arg
            580                 585                 590

gtg gcc gag tac cca atc ctc aca cag atc gcc aga gat gtg ctc gcc 1824
Val Ala Glu Tyr Pro Ile Leu Thr Gln Ile Ala Arg Asp Val Leu Ala
        595                 600                 605

atc cag gtg tca aca gtg gct tct gag tct gcc ttt tcc gct ggc ggc 1872
Ile Gln Val Ser Thr Val Ala Ser Glu Ser Ala Phe Ser Ala Gly Gly
    610                 615                 620

agg gtg gtg gat cca tac aga aat aga ctc ggc tcc gag atc gtc gag 1920
Arg Val Val Asp Pro Tyr Arg Asn Arg Leu Gly Ser Glu Ile Val Glu
625                 630                 635                 640

gcc ctc atc tgc act aag gac tgg gtg gca gcc tct agg aag ggc gcc 1968
Ala Leu Ile Cys Thr Lys Asp Trp Val Ala Ala Ser Arg Lys Gly Ala
                645                 650                 655

aca tac ttc ccg acc atg att ggc gac ctt gag gtg ctc gac agc gtt 2016
Thr Tyr Phe Pro Thr Met Ile Gly Asp Leu Glu Val Leu Asp Ser Val
            660                 665                 670

atc gcc gct gcc acc aac cac gag aac cac atg gac gag gat gag gac 2064
Ile Ala Ala Ala Thr Asn His Glu Asn His Met Asp Glu Asp Glu Asp
        675                 680                 685

gcc atc gag ttc agc aag aac aat gag gac gtg gcc agc ggc tcc tcc 2112
Ala Ile Glu Phe Ser Lys Asn Asn Glu Asp Val Ala Ser Gly Ser Ser
    690                 695                 700

cca tga 2118
Pro
705

<210> 30
<211> 705
<212> PRT
<213> Artificial Sequence

<220>
<223> Synthetic Construct

<400> 30

Met Ala Ile Val His Glu Pro Gln Pro Gln Pro Gln Pro Gln Pro Glu
1               5                   10                  15

Pro Gln Pro Gln Pro Gln Pro Glu Pro Glu Glu Glu Ala Pro Gln Lys
            20                  25                  30

Arg Ala Lys Lys Cys Thr Ser Asp Val Trp Gln His Phe Thr Lys Lys
        35                  40                  45

Glu Ile Glu Val Glu Val Asp Gly Lys Lys Tyr Val Gln Val Trp Gly
    50                  55                  60

His Cys Asn Phe Pro Asn Cys Lys Ala Lys Tyr Arg Ala Glu Gly His
65                  70                  75                  80

His Gly Thr Ser Gly Phe Arg Asn His Leu Arg Thr Ser His Ser Leu
                85                  90                  95

Val Lys Gly Gln Leu Cys Leu Lys Ser Glu Lys Asp His Gly Lys Asp
            100                 105                 110

Ile Asn Leu Ile Glu Pro Tyr Lys Tyr Asp Glu Val Val Ser Leu Lys
        115                 120                 125

Lys Leu His Leu Ala Ile Ile Met His Glu Tyr Pro Phe Asn Ile Val
    130                 135                 140

Glu His Ala Tyr Phe Val Glu Phe Val Lys Ser Leu Arg Pro His Phe
145                 150                 155                 160

Pro Ile Lys Ser Arg Val Thr Ala Arg Lys Tyr Ile Met Asp Leu Tyr
                165                 170                 175

Leu Glu Glu Lys Glu Lys Leu Tyr Gly Lys Leu Lys Asp Val Gln Ser
            180                 185                 190

Arg Phe Ser Thr Thr Met Asp Met Trp Thr Ser Cys Gln Asn Lys Ser
        195                 200                 205

Tyr Met Cys Val Thr Ile His Trp Ile Asp Asp Asp Trp Cys Leu Gln
    210                 215                 220

Lys Arg Ile Val Gly Phe Phe His Val Ala Gly Arg His Thr Gly Gln
225                 230                 235                 240

Arg Leu Ser Gln Thr Phe Thr Ala Ile Met Val Lys Trp Asn Ile Glu
                245                 250                 255

Lys Lys Leu Phe Ala Leu Ser Leu Asp Asn Ala Ser Ala Asn Glu Val
            260                 265                 270

Ala Val His Asp Ile Ile Glu Asp Leu Gln Asp Thr Asp Ser Asn Leu
        275                 280                 285

Val Cys Asp Gly Ala Phe Phe His Val Arg Cys Ala Cys His Ile Leu
    290                 295                 300

Asn Leu Val Ala Lys Asp Gly Leu Ala Val Ile Ala Gly Thr Ile Glu
305                 310                 315                 320

Lys Ile Lys Ala Ile Val Leu Ala Val Lys Ser Ser Pro Leu Gln Trp
                325                 330                 335

Glu Glu Leu Met Lys Cys Ala Ser Glu Cys Asp Leu Asp Lys Ser Lys
            340                 345                 350

Gly Ile Ser Tyr Ala Val Ser Thr Arg Trp Asn Ser Thr Tyr Leu Met
        355                 360                 365

Leu Arg Asp Ala Leu Tyr Tyr Lys Pro Ala Leu Ile Arg Leu Lys Thr
    370                 375                 380

Ser Asp Pro Arg Arg Tyr Asp Ala Ile Cys Pro Lys Ala Glu Glu Trp
385                 390                 395                 400

Lys Met Ala Leu Thr Leu Phe Lys Cys Leu Lys Lys Phe Phe Asp Leu
                405                 410                 415

Thr Glu Leu Leu Ser Gly Thr Gln Tyr Ser Thr Ala Asn Leu Phe Tyr
            420                 425                 430

Lys Gly Phe Cys Glu Ile Lys Asp Leu Ile Ala Gln Trp Cys Val His
        435                 440                 445

Glu Lys Phe Val Ile Arg Arg Met Ala Val Ala Met Ser Glu Lys Phe
    450                 455                 460

Glu Lys Tyr Trp Lys Val Ser Asn Ile Ala Leu Ala Val Ala Cys Phe
465                 470                 475                 480

Leu Asp Pro Arg Tyr Lys Lys Ile Leu Ile Glu Phe Tyr Met Lys Lys
                485                 490                 495

Phe His Gly Asp Ser Tyr Lys Val His Val Asp Asp Phe Val Arg Val
            500                 505                 510

Ile Arg Lys Leu Tyr Gln Phe Tyr Ser Ser Cys Ser Pro Ser Ala Pro
        515                 520                 525

Lys Thr Lys Thr Thr Thr Asn Asp Ser Met Asp Asp Thr Leu Met Glu
    530                 535                 540

Asn Glu Asp Asp Glu Phe Gln Asn Tyr Leu His Glu Leu Lys Asp Tyr

545                 550                 555                 560

Asp Gln Val Glu Ser Asn Glu Leu Asp Lys Tyr Met Ser Glu Pro Leu
                565                 570                 575

Leu Lys His Ser Gly Gln Phe Asp Ile Leu Ser Trp Trp Arg Gly Arg
            580                 585                 590

Val Ala Glu Tyr Pro Ile Leu Thr Gln Ile Ala Arg Asp Val Leu Ala
        595                 600                 605

Ile Gln Val Ser Thr Val Ala Ser Glu Ser Ala Phe Ser Ala Gly Gly
    610                 615                 620

Arg Val Val Asp Pro Tyr Arg Asn Arg Leu Gly Ser Glu Ile Val Glu
625                 630                 635                 640

Ala Leu Ile Cys Thr Lys Asp Trp Val Ala Ala Ser Arg Lys Gly Ala
                645                 650                 655

Thr Tyr Phe Pro Thr Met Ile Gly Asp Leu Glu Val Leu Asp Ser Val
            660                 665                 670

Ile Ala Ala Ala Thr Asn His Glu Asn His Met Asp Glu Asp Glu Asp
        675                 680                 685

Ala Ile Glu Phe Ser Lys Asn Asn Glu Asp Val Ala Ser Gly Ser Ser
    690                 695                 700

Pro
705

<210>  31
<211>  25
<212>  DNA
<213>  Artificial Sequence

<220>
<223>  right border sequences of T-DNA

<400>  31
tggcaggata tataccgttg taatt                                          25

<210>  32
<211>  25
<212>  DNA
<213>  Artificial Sequence

<220>
<223>  left border sequences of T-DNA

<400>  32
cggcaggata tattcaattg taaat                                              25


<210>  33
<211>  21
<212>  DNA
<213>  Artificial Sequence

<220>
<223>  NLS-coding sequence derived from SV40

<400>  33
ccaaagaaga agagaaaggt c                                                  21


<210>  34
<211>  300
<212>  DNA
<213>  Artificial Sequence

<220>
<223>  subterminal region (Ds5)

<400>  34
cagggatgaa agtaggatgg gaaaatcccg taccgaccgt tatcgtataa ccgattttgt        60

tagttttatc ccgatcgatt tcgaacccga ggtaaaaaac gaaaacggaa cggaaacggg       120

atatacaaaa cggtaaacgg aaacggaaac ggtagagcta gtttcccgac cgtttcaccg       180

ggatcccgtt tttaatcggg atgatcccgt ttcgttaccg tattttctaa ttcgggatga       240

ctgcaatatg gccagctcca actcccatcc ataaccactg aggcccagcc catgtaagaa       300


<210>  35
<211>  300
<212>  DNA
<213>  Artificial Sequence

<220>
<223>  subterminal region (Ds3)

<400>  35
tgctccagat ttatatggat tttatctatg tttaattaag acttgtgttt acaatttttt        60

atatttgttt ttaagttttg aatatatgtt ttcatgtgtg attttaccga acaaaaatac       120

cggttcccgt ccgatttcga ctttaacccg accggatcgt atcggttttc gattaccgta       180

tttatcccgt tcgttttcgt taccggtata tcccgttttc gtttccgtcc cgcaagttaa       240

atatgaaaat gaaaacggta gaggtatttt accgaccgtt accgaccgtt ttcatcccta       300


<210>  36
<211>  29839
<212>  DNA
<213>  Artificial Sequence

<220>
<223>  Vector sequence of pClpP5 AcDsEG-D

<220>
<221>  promoter
<222>  (7)..(536)
<223>  p35S (CaMV 35S promoter)

<220>
<221>  gene
<222>  (553)..(1284)
<223>  DT-A gene with intron1 of Caster bean Cat1 gene

<220>
<221>  terminator
<222>  (1338)..(1536)
<223>  t35S (CaMV 35S terminator)

<220>
<221>  misc_feature
<222>  (1593)..(4592)
<223>  OsClpP5 5′ side homologous region for HR

<220>
<221>  misc_feature
<222>  (4593)..(4892)
<223>  5′ terminal region of Ds element (Ds5)

<220>
<221>  promoter
<222>  (4916)..(5753)
<223>  pAct1 (promoter region of rice Act1 gene)

<220>
<221>  5′UTR
<222>  (5754)..(6327)
<223>  5′UTR region of rice Act1 gene with intron

<220>
<221>  gene
<222>  (6328)..(7353)
<223>  hpt (Hygromycin phosphotransferase gene)

<220>
<221>  terminator
<222>  (7375)..(7574)
<223>  t35S (CaMV 35S terminator)

<220>
<221>  terminator
<222>  (7635)..(8611)
<223>  delta En/Spm

<220>
<221>  promoter
<222>  (8626)..(9394)
<223>  pHSP (Heat shock promoter)

<220>
<221>  gene
<222>  (9411)..(11626)
<223>  AcTPase4x gene with intron and SV40 NLS

<220>

<221>  terminator
<222>  (11665)..(11917)
<223>  tNOS (NOS terminator)

<220>
<221>  promoter
<222>  (11943)..(12472)
<223>  p35S (CaMV 35S promoter)

<220>
<221>  gene
<222>  (12489)..(13200)
<223>  EGFP gene

<220>
<221>  terminator
<222>  (13228)..(13526)
<223>  tE9 (pea rbcS E9 terminator)

<220>
<221>  misc_feature
<222>  (13615)..(13914)
<223>  3′ terminal region of Ds element (Ds3)

<220>
<221>  misc_feature
<222>  (13915)..(16912)
<223>  OsClpP5 3′ side homologous region for HR

<220>
<221>  terminator
<222>  (16962)..(17155)
<223>  t35S (CaMV 35S terminator) Complementary strand

<220>
<221>  gene
<222>  (17163)..(17750)
<223>  DT-A gene Complementary strand

<220>
<221>  promoter
<222>  (17751)..(19747)
<223>  pUbi (Maize Ubiquitin promoter)

<220>
<221>  misc_signal
<222>  (20298)..(20322)
<223>  T-DNA RB

<220>
<221>  promoter
<222>  (21040)..(21144)
<223>  AmpR promoter

<220>
<221>  gene
<222>  (21145)..(22006)
<223>  AmpR gene

<220>
<221>  rep_origin
<222>  (22177)..(22765)
<223>  Ori

<220>
<221>  rep_origin
<222>  (23435)..(23629)
<223>  pVS1 oriV

<220>
<221>  gene
<222>  (23695)..(24768)
<223>  pVS1 RepA

<220>
<221>  gene
<222>  (25197)..(25826)
<223>  pVS1 StaA

<220>
<221>  gene
<222>  (27730)..(28521)
<223>  SmR

<220>
<221>  misc_signal
<222>  (29079)..(29103)
<223>  T-DNA LB

<400>  36
gaattcccat ggagtcaaag attcaaatag aggacctaac agaactcgcc gtaaagactg      60

gcgaacagtt catacagagt ctcttacgac tcaatgacaa gaagaaaatc ttcgtcaaca     120

tggtggagca cgacacgctt gtctactcca aaaatatcaa agatacagtc tcagaagacc     180

aaagggcaat tgagactttt caacaaaggg taatatccgg aaacctcctc ggattccatt     240

gcccagctat ctgtcacttt attgtgaaga tagtggaaaa ggaaggtggc tcctacaaat     300

gccatcattg cgataaagga aaggccatcg ttgaagatgc ctctgccgac agtggtccca     360

aagatggacc cccacccacg aggagcatcg tggaaaaaga agacgttcca accacgtctt     420

caaagcaagt ggattgatgt gatatctcca ctgacgtaag ggatgacgca caatcccact     480

atccttcgca agacccttcc tctatataag gaagttcatt tcatttggag aggacagggt     540

acccggggat ctatggatcc cgacgatgta aatttctagt ttttctcctt cattttcttg     600

gttaggaccc ttttctcttt ttattttttt gagctttgat ctttctttaa actgatctat     660

tttttaattg attggttatg gtgtaaatat tacatagctt taactgataa tctgattact     720

ttatttcgtg tgtctatgat gatgatgata gttacaggtt gtcgactctt ctaaatcttt     780

tgtgatggaa aacttttctt cgtaccacgg gactaaacct ggttatgtag attccattca     840

aaaaggtata caaaagccaa aatctggtac acaaggaaat tatgacgatg attggaaagg     900

gttttatagt accgacaata aatacgacgc tgcgggatac tctgtagata atgaaaaccc     960

gctctctgga aaagctggag gcgtggtcaa agtgacgtat ccaggactga cgaaggttct    1020

cgcactaaaa gtggataatg ccgaaactat taagaaagag ttaggtttaa gtctcactga    1080

accgttgatg gagcaagtcg gaacggaaga gtttatcaaa aggttcggtg atggtgcttc 1140

gcgtgtagtg ctcagccttc ccttcgctga ggggagttct agcgttgaat atattaataa 1200

ctgggaacag gcgaaagcgt taagcgtaga acttgagatt aattttgaaa cccgtggaaa 1260

acgtggccaa gatgcgatgt atgagtatat ggctcaagcc tgtgcaggaa atcgtgtcag 1320

gcgatcttag catgcccgct gaaatcacca gtctctctct acaaatctat ctctctctat 1380

aataatgtgt gagtagttcc cagataaggg aattagggtt cttatagggt ttcgctcatg 1440

tgttgagcat ataagaaacc cttagtatgt atttgtattt gtaaaatact tctatcaata 1500

aaatttctaa ttcctaaaac caaaatccag gggtacctcg agtaactata acggtcctaa 1560

ggtagcgatt aattaaattt aaatcgtcga cgtgactgtt tagtgaaaat aactcatgaa 1620

atattttgca gatggatcct tatctaagaa ttggtgaaga cttgcaactc tatgtaaggt 1680

tgcaatctga tctaggtaac tatggttctg atagcgatca agaaatcgct agatctgtac 1740

tttctgactg caggacaaaa gtggggatta atgatcagcg agtacttgat gtagttgctt 1800

gtgcactgtg taatttaact gaggtaaagg ttgattttgc cttctatttg ctgttgacac 1860

tgcacgttgt gttcttattg aggtcacatg ctgttgacag caatggttga gtgcagttat 1920

ctaatattct tggaattgct attgccagat ggacaaggat gtactggtga aggagctcac 1980

agaaatgttt acacctgaag aggtgccctt gtttgggtca aattcagcat ttgactgggc 2040

caattttcat gttcaggcat tttctgatga atccctttct tttgatgagg taggtacatt 2100

aaaactggtg tctttaatga ttcatctggt ggaagctctt ataaaattct ttgatggtgc 2160

aatgatcctt gtaactgtac cttaccatta tgaaaattat tgttactttt tgtatcttct 2220

cagttgtctt ctgcattgta ggagtgttca agaacctctt cagtagatgg tgggttgcat 2280

gagtcaccaa ttacaaacac cggtagctcg atatcaaaga ctacgatgcc acaatctgtt 2340

cctcgtgtct taggtgttgg ccagttgctt gaatcggtga gtacttatca gctgcctttg 2400

cacatggttt gctagttgct gctactcagc tagctctagt tgccctaatc ttattaattg 2460

ttaaggtggc atggaaccat aacatgttgt gtttgtaccc ctttctgctg ctgtcaaaat 2520

agtatttatc acgtggtgga ttcggctaca aatatattat ggagcatttg aatctatgta 2580

acattttctc tactgtgaac tcatatcttt gtaatcaggc gttacatgta gccggccaag 2640

ttgcgggagc atctgtttcg acctccccgc tcccatacgg cacaatgacc agtcaatgcg 2700

aagccttggg atcaggcact aggaagaagc tctcaagctg gcttgtcaat ggccatgact 2760

ctacgccaga caatcctgct ccaagccttc catctgcgca gcatttcatc attcctaaag 2820

tacatgattt gtcctgtctt tctcccatgt cattctggaa tgttgcctat tctgcatctg 2880

tgtgtatcat ctggccctca cgatcgcaat cgacattcat ccaggtaaat tcatgcggtt 2940

tcgagagcag catccggacg actttggagc cttgctcggc agtgaagctc ccacccgcca 3000

gccccttcga caacttcctg aaggctgcat atcgtgccca gtagcagcca tgacaagcct 3060

tcgagattat gcctaaccat gtttagtagt gctaggatgt agtacctgag catcagaact 3120

agattatctt ttgaaagact tggccgatct ggcttatcct ttattatctg tagcactgtt 3180

agttgtagta gcttgtgtat caatgtatgg ctatgtctgt gtccctccag taaatgacaa 3240

tcgtatatgt actatgtagt atgaccatgt agcccactag taaatgtttg tagggtatgt 3300

atgacaatgt atgaccatat tgtttttttg agatgtagga ttctatttca gactcgagta 3360

atctgtgggg gacatgttcg tcgacctccg gtgctatttt tccttttatc actgcaagga 3420

ttagatggtt tcctgcaata tttgcatgct attcgcgcgg atgaaattgt gaagtcatgg 3480

gtttctcaag tccacaatca ttagttcaca ttattacggc gttcatgaat attacatttt 3540

ggaggatctt atttctatag tttttctata tagctctttg aatcaagaaa tgtttttcat 3600

gtgatctaat caaaaggtca tttctatgtt tgcaattatc tattttacat ttacatttct 3660

atcaaaatct tttttttgtt ttttttccta tttatctgtt ttttttattc ctgcgattca 3720

aagtgacgct taaaatttag ggaaaattgc aaaaaccatc ctataaatca cttgaaactt 3780

gacattccac tttataagcc attttattgc aaataccacc catctactct gtctaatcga 3840

atcaacatgt tttcatgaat tatgagctgt caatgaagct tttaccaaaa ttaaatattt 3900

atatagtaca gtcttgacac ttttgtattg agtttcgctg tattttaaaa aaaattaatt 3960

aaacatgaag aataaatctg atacaaaagc acttggacaa gattgactcg actagtttta 4020

acctgtggta tccctacccc agcgataatg ggttgattcg gttaaacacg ttcgcactag 4080

aatggttttt acaaaaaaaa agaactgagt aggggtggac atttacaata aaataactta 4140

tgagatgtaa tgtgaaattt caagtgattt atagggttgt ttttttttaa attccctaga 4200

atttaacggg tgtgtgtctg gtggacgtcc gtggtgcgcg cgcgcgcgcg agggaatgat 4260

caaaaacctt ttcggggcgt cacgtaagcc catgacccga cgtccaaagg cctaccaaca 4320

ccgtcggcat ggcccgcgtc ttctcctcgt cttcctctct ctcgagtctc gaagaagaca 4380

cacactattc tctcttcttc ttaccaccct ctcccggata agaggcgcaa ccaaagcccc 4440

caccctcgcc caaaaccccc acgagccgcg gccatggcga ccaccaccac caccccctcc 4500

tcctctctca ccgcccctct cctccgcccg agctcgaacg cgaaccccgc cccgagatct 4560

ctgccgctcc tcagctgcac tcccctcttc cgcagggatg aaagtaggat gggaaaatcc 4620

cgtaccgacc gttatcgtat aaccgatttt gttagtttta tcccgatcga tttcgaaccc 4680

gaggtaaaaa acgaaaacgg aacggaaacg ggatatacaa aacggtaaac ggaaacggaa 4740

acggtagagc tagtttcccg accgtttcac cgggatcccg tttttaatcg ggatgatccc 4800

gtttcgttac cgtattttct aattcgggat gactgcaata tggccagctc caactcccat 4860

ccataaccac tgaggcccag cccatgtaag aagcggccgc actagcatac tcgaggtcat 4920

tcatatgctt gagaagagag tcgggatagt ccaaaataaa acaaaggtaa gattacctgg 4980

tcaaaagtga aaacatcagt taaaaggtgg tataaagtaa aatatcggta ataaaaggtg 5040

gcccaaagtg aaatttactc ttttctacta ttataaaaat tgaggatgtt tttgtcggta 5100

ctttgatacg tcatttttgt atgaattggt ttttaagttt attcgctttt ggaaatgcat 5160

atctgtattt gagtcgggtt ttaagttcgt ttgcttttgt aaatacagag ggatttgtat 5220

aagaaatatc tttaaaaaaa cccatatgct aatttgacat aatttttgag aaaaatatat 5280

attcaggcga attctcacaa tgaacaataa taagattaaa atagctttcc cccgttgcag 5340

cgcatgggta ttttttctag taaaaataaa agataaactt agactcaaaa catttacaaa 5400

aacaacccct aaagttccta aagcccaaag tgctatccac gatccatagc aagcccagcc 5460

caacccaacc caacccaacc caccccagtc cagccaactg gacaatagtc tccacacccc 5520

cccactatca ccgtgagttg tccgcacgca ccgcacgtct cgcagccaaa aaaaaaaaaa 5580

gaaagaaaaa aaagaaaaag aaaaaacagc aggtgggtcc gggtcgtggg ggccggaaac 5640

gcgaggagga tcgcgagcca gcgacgaggc cggccctccc tccgcttcca aagaaacgcc 5700

ccccatcgcc actatataca tacccccccc tctcctccca tccccccaac cctaccacca 5760

ccaccaccac cacctccacc tcctcccccc tcgctgccgg acgacgagct cctcccccct 5820

ccccctccgc cgccgccgcg ccggtaacca ccccgcccct ctcctctttc tttctccgtt 5880

ttttttttcc gtctcggtct cgatctttgg ccttggtagt ttgggtgggc gagaggcggc 5940

ttcgtgcgcg cccagatcgg tgcgcgggag gggcgggatc tcgcggctgg ggctctcgcc 6000

ggcgtggatc cggcccggat ctcgcgggga atggggctct cggatgtaga tctgcgatcc 6060

gccgttgttg ggggagatga tggggggttt aaaatttccg ccatgctaaa caagatcagg 6120

aagaggggaa aagggcacta tggtttatat ttttatatat ttctgctgct tcgtcaggct 6180

tagatgtgct agatctttct ttcttctttt tgtgggtaga atttgaatcc ctcagcattg 6240

ttcatcggta gtttttcttt tcatgatttg tgacaaatgc agcctcgtgc ggagcttttt 6300

tgtaggtaga ccggggggca atgagatatg aaaaagcctg aactcaccgc gacgtctgtc 6360

gagaagtttc tgatcgaaaa gttcgacagc gtctccgacc tgatgcagct ctcggagggc 6420

gaagaatctc gtgctttcag cttcgatgta ggagggcgtg gatatgtcct gcgggtaaat 6480

agctgcgccg atggtttcta caaagatcgt tatgtttatc ggcactttgc atcggccgcg 6540

ctcccgattc cggaagtgct tgacattggg gaattcagcg agagcctgac ctattgcatc 6600

tcccgccgtg cacagggtgt cacgttgcaa gacctgcctg aaaccgaact gcccgctgtt 6660

ctgcagccgg tcgcggaggc catggatgcg atcgctgcgg ccgatcttag ccagacgagc 6720

gggttcggcc cattcggacc gcaaggaatc ggtcaataca ctacatggcg tgatttcata 6780

tgcgcgattg ctgatcccca tgtgtatcac tggcaaactg tgatggacga caccgtcagt 6840

gcgtccgtcg cgcaggctct cgatgagctg atgctttggg ccgaggactg ccccgaagtc 6900

cggcacctcg tgcacgcgga tttcggctcc aacaatgtcc tgacggacaa tggccgcata 6960

acagcggtca ttgactggag cgaggcgatg ttcggggatt cccaatacga ggtcgccaac 7020

atcttcttct ggaggccgtg gttggcttgt atggagcagc agacgcgcta cttcgagcgg 7080

aggcatccgg agcttgcagg atcgccgcgg ctccgggcgt atatgctccg cattggtctt 7140

gaccaactct atcagagctt ggttgacggc aatttcgatg atgcagcttg ggcgcagggt 7200

cgatgcgacg caatcgtccg atccggagcc gggactgtcg ggcgtacaca aatcgcccgc 7260

agaagcgcgg ccggccgtct ggaccgatgg ctgtgtagaa gtactcgccg atagtggaaa 7320

ccgacgcccc agcactcgtc cgggatcctc tagagtcgac ctgcaggcat gcccgctgaa 7380

atcaccagtc tctctctaca aatctatctc tctctataat aatgtgtgag tagttcccag 7440

ataagggaat tagggttctt atagggtttc gctcatgtgt tgagcatata agaaaccctt 7500

agtatgtatt tgtatttgta aaatacttct atcaataaaa tttctaattc ctaaaaccaa 7560

aatccagggg taccccctagt ctctcttaag gtagcatcac aagtttgtac aaaaaagcag 7620

gcttccctcg agggcaactt ctgtccaaga ccaattgatg ccattgggtg tgataggagg 7680

gcaaatgatg ccgtgggcac ctcgccagcc aggcatttgg ccaccgatgc aaacacagat 7740

gccaccgccg atgccgtggg gatttcctcc tcgtgggcag tcacaatcac caggattgcc 7800

ctcacactca ccaggatcag tacgttaagt tgatatcctt tgcatctcta tttgcttcgt 7860

tgtttaagca gttactagaa aacatgcatg tatatgttgc agtctatgta tatgtttaat 7920

tagttactcg gtaaactaac aaatgtttgt ttcttttaaa gggttcaggc tcacatcatg 7980

ctagtccgcc tccggatcag agcacgttta tggacttatt gatgaacaca agtggcggcg 8040

gctccaatga cccaccaaca gaatgaatta atatggaggc ttgtgtggaa cttactatga 8100

ttgcgttttg tatggacttt aacttgtttt agatggattt gaacttcttt cgtatggact 8160

tgaacttgta tgaatattga atatggtgct tgtgttatgt tatgttgaat atggtgcttg 8220

tgttgtgata tattgaatgt tgtgcttata ttgtgctgtt atggaggctt cccatccggg 8280

gagggagaaa aataaaattg gatattaaaa aaaattattc actaagagtg tcggccccca 8340

cactcttata tgcgcccagg tagcttactg atgtgcgcgc agtaagagtg acggccacgg 8400

tactggccga cacttttaac ataagagtgt cggttgcttg ttgaaccgac acttttaaca 8460

taagagcgtc ggtccccaca cttctatacg aataagagcg tccattttag agtgacggct 8520

aagagtgtcg gtcaaccgac actcttatac ttagagtgtc ggcttatttc agtaagagtg 8580

tggggttttg gccgaagctg ggcccgctag cgtttaaatg ctagcccagt gaaagcagtg 8640

aattgaagca ttcccgaaac ccactggaat gatctagtac tcactctacg atgtacagtg 8700

aagtaatact tcaaaactgg tgtaatttgg tatgccaaaa ggactccata gtttcacgac 8760

atatttccaa acggttcagg atcagtactg cccatctgcc tggggcccac actagcgggc 8820

aattggttct cgtagtttct cgttctcaat caatcattcc atactcgcta tcccctccat 8880

cacagaataa atgcaacaat gagtttccgt gtacaaattt aatcgttcgt cttatttaaa 8940

atatttttta aaaaactaaa aaacaaaagt cacgcataaa gtactattca tgttttataa 9000

tctaataaca gtataaatac taatcataaa aaaaaattca aataagatgg acgattaaag 9060

ttgaacactg aaattcatgg ctgcttttgt tttgagactg agggagtaca cgataagatt 9120

tgatcgcaat caaagtaacc tacatcaaag aagcaagata tgtgggggaa aaatgaatac 9180

tctagagcaa attaaggtga gccccgcttt gtagaggctg atggagtact ggagcgacgg 9240

aagcgaagca gatcgagtgt gctgtaaagc gaaacgagca agaaccagag aagtccagag 9300

atttcaggac agattagttg tgaacctata aatatcctgc ctcattcccc aacctccatc 9360

catcgagcca agactgaagc atttgatcga gctcctgcag aaccttggcc atggctccaa 9420

agaagaagag aaaggtcatg gccatcgtgc atgagccaca gccacaaccg caaccacagc 9480

cagagcctca gcctcaacct cagcctgagc cagaggagga ggccccacaa aagcgcgcta 9540

agaagtgcac ctccgacgtc tggcagcact tcaccaagaa ggagatcgag gtcgaggttg 9600

acggcaagaa gtacgtccag gtctggggcc actgcaactt cccaaactgc aaggccaagt 9660

acagggccga gggccatcat ggcacatccg gctttaggaa ccacctcagg acctcccact 9720

ctctcgtgaa gggccaactc tgcctcaagt ccgagaagga ccacggcaag gacatcaacc 9780

tcatcgagcc gtacaagtac gacgaggtgg tgtccctcaa gaagctccac ctcgccatca 9840

ttatgcacga gtacccgttc aacatcgtcg agcacgccta cttcgtcgag ttcgtgaagt 9900

ccctcaggcc gcacttcccg atcaagtcta gagtgaccgc ccgcaagtac atcatggacc 9960

tctaccttga ggagaaggag aagctctacg gcaagctcaa ggacgtgcag tccaggttct 10020

ccaccacgat ggacatgtgg accagctgcc agaacaagtc ctacatgtgc gtgaccatcc 10080

actggatcga cgacgactgg tgcctccaga agaggatcgt cggcttcttc catgtggccg 10140

gcagacatac aggccagagg ctctcccaaa ccttcaccgc gatcatggtc aagtggaaca 10200

tcgagaagaa gctgttcgcc ctctccctcg ataacgcctc cgctaatgag gtggccgtgc 10260

acgacatcat cgaggacctc caggacaccg actccaacct tgtgtgcgac ggcgccttct 10320

ttcatgttcg ctgcgcctgc cacatcctca acctggttgc taaggatggc ctcgccgtga 10380

tcgccggcac cattgagaag atcaaggcca tcgtcctcgc cgtcaagtcc tctccacttc 10440

agtgggagga gctgatgaag tgcgcgtccg agtgcgatct cgacaagtcc aagggcatca 10500

gctacgccgt gtccaccagg tggaatagca cctacctcat gctccgcgac gccctctact 10560

acaagccagc cctcatcagg ctcaagacct ccgatccaag acggtatgtt tgtctcaatt 10620

gttgtacatg tcatcattat aaattctcaa ttaatcaaat gtcaattatt gtagctacga 10680

tgccatctgc ccaaaggccg aggagtggaa gatggcgctc accctcttca agtgcctgaa 10740

gaagttcttc gacctcaccg agctgctctc cggcacccaa tactctaccg ccaacctctt 10800

ctacaagggc ttctgcgaga tcaaggacct gatcgcccaa tggtgcgtgc acgagaagtt 10860

cgtgattcgc agaatggccg tggccatgag cgagaagttt gagaagtact ggaaggtgtc 10920

caatatcgcc ctcgcggtgg cctgcttcct cgatccaagg tacaagaaga tcctgatcga 10980

gttctacatg aagaagtttc acggcgactc ctacaaggtg cacgtcgacg atttcgtgcg 11040

cgtgatccgc aagctctacc agttctactc ctcctgctct ccatccgccc caaagaccaa 11100

gaccaccacc aacgacagca tggacgacac cctcatggag aacgaggacg acgagttcca 11160

gaactacctc cacgagctga aggactacga ccaggtcgag agcaacgagc tggacaagta 11220

catgtccgag ccgctcttga agcactccgg ccagttcgat atcctctcat ggtggcgcgg 11280

cagagtggcc gagtacccaa tcctcacaca gatcgccaga gatgtgctcg ccatccaggt 11340

gtcaacagtg gcttctgagt ctgccttttc cgctggcggc agggtggtgg atccatacag 11400

aaatagactc ggctccgaga tcgtcgaggc cctcatctgc actaaggact gggtggcagc 11460

ctctaggaag ggcgccacat acttcccgac catgattggc gaccttgagg tgctcgacag 11520

cgttatcgcc gctgccacca accacgagaa ccacatggac gaggatgagg acgccatcga 11580

gttcagcaag aacaatgagg acgtggccag cggctcctcc ccatgaattg gtgaccagct 11640

cggagatctc acgtgaattt ccccgatcgt tcaaacattt ggcaataaag tttcttaaga 11700

ttgaatcctg ttgccggtct tgcgatgatt atcatataat ttctgttgaa ttacgttaag 11760

catgtaataa ttaacatgta atgcatgacg ttatttatga gatgggtttt tatgattaga 11820

gtcccgcaat tatacattta atacgcgata gaaaacaaaa tatagcgcgc aaactaggat 11880

aaattatcgc gcgcggtgtc atctatgtta ctagatcggg gcggccgcac tagtcctgca 11940

ggccatggag tcaaagattc aaatagagga cctaacagaa ctcgccgtaa agactggcga 12000

acagttcata cagagtctct tacgactcaa tgacaagaag aaaatcttcg tcaacatggt 12060

ggagcacgac acacttgtct actccaaaaa tatcaaagat acagtctcag aagaccaaag 12120

ggcaattgag acttttcaac aaagggtaat atccggaaac ctcctcggat tccattgccc 12180

agctatctgt cactttattg tgaagatagt ggaaaaggaa ggtggctcct acaaatgcca 12240

tcattgcgat aaaggaaagg ccatcgttga agatgcctct gccgacagtg gtcccaaaga 12300

tggaccccca cccacgagga gcatcgtgga aaaagaagac gttccaacca cgtcttcaaa 12360

gcaagtggat tgatgtgata tctccactga cgtaagggat gacgcacaat cccactatcc 12420

ttcgcaagac ccttcctcta tataaggaag ttcatttcat ttggagagga cagggtacct 12480
agggatctat ggtgagcaag ggcgaggagc tgttcaccgg ggtggtgccc atcctggtcg 12540
agctggacgg cgacgtaaac ggccacaagt tcagcgtgtc cggcgagggc gagggcgatg 12600
ccacctacgg caagctgacc ctgaagttca tctgcaccac cggcaagctg cccgtgccct 12660
ggcccaccct cgtgaccacc ctgacctacg gcgtgcagtg cttcagccgc taccccgacc 12720
acatgaagca gcacgacttc ttcaagtccg ccatgcccga aggctacgtc caggagcgca 12780
ccatcttctt caaggacgac ggcaactaca agacccgcgc cgaggtgaag ttcgagggcg 12840
acaccctggt gaaccgcatc gagctgaagg gcatcgactt caaggaggac ggcaacatcc 12900
tggggcacaa gctggagtac aactacaaca gccacaacgt ctatatcatg gccgacaagc 12960
agaagaacgg catcaaggtg aacttcaaga tccgccacaa catcgaggac ggcagcgtgc 13020
agctcgccga ccactaccag cagaacaccc ccatcggcga cggccccgtg ctgctgcccg 13080
acaaccacta cctgagcacc cagtccgccc tgagcaaaga ccccaacgag aagcgcgatc 13140
acatggtcct gctggagttc gtgaccgccg ccgggatcac tctcggcatg gacgagctgt 13200
acaagtaatc tagagttaac ctagcttgtt cgagtattat ggcattggga aaactgtttt 13260
tcttgtacca tttgttgtgc ttgtaattta ctgtgttttt tattcggttt tcgctatcga 13320
actgtgaaat ggaaatggat ggagaagagt taatgaatga tatggtcctt ttgttcattc 13380
tcaaattaat attatttgtt ttttctctta tttgttgtgt gttgaatttg aaattataag 13440
agatatgcaa acattttgtt ttgagtaaaa atgtgtcaaa tcgtggcctc taatgaccga 13500
agttaatatg aggagtaaaa cactagatcc ccaaacggta ccgtttaaac actagtgtca 13560
aagggcgaat tcgacccagc tttcttgtac aaagtggtga tgttaacctc gaggtgctcc 13620
agatttatat ggattttatc tatgtttaat taagacttgt gtttacaatt ttttatattt 13680
gtttttaagt tttgaatata tgttttcatg tgtgatttta ccgaacaaaa ataccggttc 13740
ccgtccgatt tcgactttaa cccgaccgga tcgtatcggt tttcgattac cgtatttatc 13800
ccgttcgttt tcgttaccgg tatatcccgt tttcgtttcc gtcccgcaag ttaaatatga 13860
aaatgaaaac ggtagaggta ttttaccgac cgttaccgac cgttttcatc cctagctccc 13920
tcctcactta ctctgtcgtg gctccactcg ctctacccta actgactcgg tcttgcagga 13980
gccggaggtg cgctcgggcc gtggcgaccg ccgccgccgc cgctggccac ggggccgctc 14040
atcagaggag cgggatttgg tcgatcaggt gggggggggg gttgattttg tgacgaacca 14100
tttgtagctc tgcatcgtct ggttggggga gggttgggtg gggatgcgag ggagctgatg 14160
atgctgtggt tgttgtgtgt ttcgtaggga tgatttggtg gtgccgaggt cgccctactt 14220
ccctgtggag tatgcgtcgg ggcaggaacg cgggccatcg cccatggtga tggagcggtt 14280
ccagagcgtc gtcagccagc tcttccagca cgtacgggag cactagattg ttgcacaagt 14340

ttatttgtgg agggttctgt gtgctgtctg actctgtgtg attgtggaac agaggattat 14400

ccggtgtggt ggacccgtgg aggatgatat ggcgaacatc atcgttgccc agctgctata 14460

tctcgatgcc atcgatccta acaaggtgaa aatgatgaat aagacgctat ccgacatcca 14520

attcctattt cctataccaa tagctgccaa agctttgtta cgaactgctt actctaaaaa 14580

tactgaggcg atgaagggag tggtgctcac tgtatttaca attttacatg aactatatgg 14640

tgtcttgttt taatggatga ttgttgttaa tccccagata aaatggtcct tttatctgtc 14700

aatactcaat accagacact aaagtaccat tgctactcac tttgggttat atatgagtcc 14760

ttgttagtcc tagcttaggc tgtgattcag ttatgtagtt gtgacctcaa accttatggt 14820

gaggcttgta ttgcgatttc atctaccagg acctgattcc atggcaccat atattcatga 14880

tttcgtactt acattcagta ctcgattctt gacttcctct caacattcct gtaggatatc 14940

attatgtatg tgaattctcc tggaggatca gtgacagctg gtaataactc atccattttt 15000

tttcatagct ccatctcact cttggaacat ttgtttcaca ccaaactgca ttttgtgttt 15060

agggatggcc atattcgaca cgatgaagca tatcagacct gatgtttcca cagtttgtat 15120

tggacttgct gcaaggtatt tagtactgaa aaactgatat ccttaattgg caattgattt 15180

ggatttgtga cgctctgcaa tttatgcatc ttataatgtt taattcttta gcattctgtt 15240

caattattca ataagggttt cagttgctat attctggtca acatgtaaac atgcacctgt 15300

ttccagttgt atatcccgcc taattccttc tcctttcata atgtgggaag cacaccaaat 15360

cattgattgt atcaggcatg ttctattcag tataatcagc ctcaatctag gattttgggt 15420

tgactgagat caaattttgt gctgcgcatg ttagcaaaga agctttgtac cctcatacct 15480

ccttaatcaa aatataattt ttcaaacttt atgtttgtga gaatccatat ggatgttgat 15540

aacaggagag tttcttcatt tcatgtactt tatcaatgtt tagccaacaa tgcaaattca 15600

ggagtgctta tcagtattcc aggcacacag tttattagta aaacatgata actgttcagc 15660

tgcaaatctg taaccatttc tttctcattg tacttttgga atgctctctt ttagctatcc 15720

atcttgcctt aagttgcatt gatttcggtt atgcactcat tttcactgtt ttgtttccct 15780

ttttggcagt atgggagctt ttctgcttag tgctgggaca aaaggtaatt gagtgattgt 15840

taatgcaagt actacattta ttctacaact agactgatct gaatccttga atactgtact 15900

tgtgaatgta acacagggaa gcgatacagc ttacctaact caagaataat gatccatcaa 15960

cctctcggag gagcccaagg acaagagact gatcttgaga tccaggtaaa tccacaaagt 16020

taaattgtgt ttttatcaag atcaggacga catctcaata gcaaataatt aaccatatca 16080

ttgtttctct cttgccggta tatggttcga tctacatgaa atacatttat ttccacaagc 16140

gaatagcaac tccgctggct ttctattgtt acttgtatca ccagaagatt tttctgtcac 16200

tgattttcca ttatttcttt taggctaatg agatgctgca tcacaaggct aacctgaatg 16260

gatacctagc ataccacact gggcagcccc tagataagat caacgtagat actgaccgtg 16320

attacttcat gagcgcgaag gaggcaaagg agtatggtct aattgatgga gttatcatga 16380

atccccttaa agcccttcaa ccgcttcctg cttctagtta gccatggagt gctcaatctc 16440

cacggagcat tttttggtta tcttttagaa ctgttattgc atccactgtt tttattagct 16500

tggcaagata gttttgcgat tccacaagca accacatcct gaggcttcaa agtttgtaca 16560

atacagatgt actactagga ggatatcttc tgcgatgaat attgcaactt atttgatgta 16620

ctattaggag gatatcttct gcgatgaata ttgcaactta tttgatactc cctctgtttc 16680

atgccaagtc gatttgattt tttttcctta gacaattttc tgtaggtttg actaaattta 16740

tcaaaaaatt agcataatct acaacactaa attagttcta ttaaaataac attgaatata 16800

ttttgataat atgtttgttg tattaaaatg ttgctatatt ttttatatac ttgatcaaat 16860

tttaaaaaag attatctata aaaaaattaa aacgatttat aatatgatga aaggcgcgcc 16920

tagggataac agggtaatct cgagaattcg agctcggtac cgctgaaatc accagtctct 16980

ctctacaaat ctatctctct ctataataat gtgtgagtag ttcccagata agggaattag 17040

ggttcttata gggtttcgct catgtgttga gcatataaga aacccttagt atgtatttgt 17100

atttgtaaaa tacttctatc aataaaattt ctaattccta aaaccaaaat ccagggggca 17160

tgctaagatc gcctgacacg atttcctgca caggcttgag ccatatactc atacatcgca 17220

tcttggccac gttttccacg ggtttcaaaa ttaatctcaa gttctacgct taacgctttc 17280

gcctgttccc agttattaat atattcaacg ctagaactcc cctcagcgaa gggaaggctg 17340

agcactacac gcgaagcacc atcaccgaac cttttgataa actcttccgt tccgacttgc 17400

tccatcaacg gttcagtgag acttaaacct aactctttct taatagtttc ggcattatcc 17460

acttttagtg cgagaacctt cgtcagtcct ggatacgtca ctttgaccac gcctccagct 17520

tttccagaga gcgggttttc attatctaca gagtatcccg cagcgtcgta tttattgtcg 17580

gtactataaa accctttcca atcatcgtca taatttcctt gtgtaccaga ttttggcttt 17640

tgtatacctt tttgaatgga atctacataa ccaggtttag tcccgtggta cgaagaaaag 17700

ttttccatca caaaagattt agaagaatca acaacatcat caggatccat ctgcagaagt 17760

aacaccaaac aacagggtga gcatcgacaa aagaaacagt accaagcaaa taaatagcgt 17820

atgaaggcag ggctaaaaaa atccacatat agctgctgca tatgccatca tccaagtata 17880

tcaagatcaa aataattata aaacatactt gtttattata atagataggt actcaaggtt 17940

agagcatatg aatagatgct gcatatgcca tcatgtatat gcatcagtaa aacccacatc 18000

aacatgtata cctatcctag atcgatattt ccatccatct taaactcgta actatgaaga 18060

tgtatgacac acacatacag ttccaaaatt aataaataca ccaggtagtt tgaaacagta 18120

ttctactccg atctagaacg aatgaacgac cgcccaacca caccacatca tcacaaccaa 18180

gcgaacaaaa agcatctctg tatatgcatc agtaaaaccc gcatcaacat gtatacctat 18240

cctagatcga tatttccatc catcatcttc aattcgtaac tatgaatatg tatggcacac 18300

acatacagat ccaaaattaa taaatccacc aggtagtttg aaacagaatt aattctactc 18360

cgatctagaa cgaccgccca accagaccac atcatcacaa ccaagacaaa aaaaagcatg 18420

aaaagatgac ccgacaaaca agtgcacggc atatattgaa ataaaggaaa agggcaaacc 18480

aaaccctatg caacgaaaca aaaaaaatca tgaaatcgat cccgtctgcg gaacggctag 18540

agccatccca ggattcccca aagagaaaca ctggcaagtt agcaatcaga acgtgtctga 18600

cgtacaggtc gcatccgtgt acgaacgcta gcagcacgga tctaacacaa acacggatct 18660

aacacaaaca tgaacagaag tagaactacc gggccctaac catggaccgg aacgccgatc 18720

tagagaaggt agagaggggg ggggggggag gacgagcggc gtaccttgaa gcggaggtgc 18780

cgacgggtgg atttggggga gatctggttg tgtgtgtgtg cgctccgaac aacacgaggt 18840

tggggaaaga gggtgtggag ggggtgtcta tttattacgg cgggcgagga agggaaagcg 18900

aaggagcggt gggaaaggaa tcccccgtag ctgccggtgc cgtgagagga ggaggaggcc 18960

gcctgccgtg ccggctcacg tctgccgctc cgccacgcaa tttctggatg ccgacagcgg 19020

agcaagtcca acggtggagc ggaactctcc agaggggtcc agaggcagcg acagagatgc 19080

cgtgccgtct gcttcgcttg gcccgacgcg acgctgctgg ttcgctggtt ggtgtccgtt 19140

agactcgtcg acggcgttta acaggctggc attatctact cgaaacaaga aaaatgtttc 19200

cttagttttt ttaatttctt aaagggtatt tgtttaattt ttagtcactt tattttattc 19260

tattttatat ctaaattatt aaataaaaaa actaaaatag agttttagtt ttcttaattt 19320

agaggctaaa atagaataaa atagatgtac taaaaaaatt agtctataaa aaccattaac 19380

cctaaaccct aaatggatgt actaataaaa tggatgaagt attatatagg tgaagctatt 19440

tgcaaaaaaa aaggagaaca catgcacact aaaaagataa aactgtagag tcctgttgtc 19500

aaaatactca attgtccttt agaccatgtc taactgttca tttatatgat tctctaaaac 19560

actgatatta ttgtagtact atagattata ttattcgtag agtaaagttt aaatatatgt 19620

ataaagatag ataaactgca cttcaaacaa gtgtgacaaa aaaaatatgt ggtaattttt 19680

tataacttag acatgcaatg ctcattatct ctagagaggg gcacgaccgg gtcacgctgc 19740

actgcagtct agagggggta ccgtcgacaa gctgggatcc cagctgggat cccagcttgg 19800

ctgcaggtcg acggatcccc cgatgagcta agctagctat atcatcaatt tatgtattac 19860

acataatatc gcactcagtc tttcatctac ggcaatgtac cagctgatat aatcagttat 19920

tgaaatattt ctgaatttaa acttgcatca ataaatttat gtttttgctt ggactataat 19980

acctgacttg ttattttatc aataaatatt taaactatat ttctttcaag atgggaatta 20040

acatctacaa attgcctttt cttatcgacc atgtacgtaa gcgcttacgt ttttggtgga 20100

cccttgagga aactggtagc tgttgtgggc ctgtggtctc aagatggatc attaatttcc 20160

accttcacct acgatggggg gcatcgcacc ggtgagtaat attgtacggc taagagcgaa 20220

tttggcctgt agacctcaat tgcgagcttt ctaatttcaa actattcggg cctaactttt 20280

ggtgtgatga tgctgactgg caggatatat accgttgtaa tttgagctcg tgtgaataag 20340

tcgctgtgta tgtttgtttg attgtttctg ttggagtgca gcccatttca ccggacaagt 20400

cggctagatt gatttagccc tgatgaactg ccgaggggaa gccatcttga gcgcggaatg 20460

ggaatggatt tcgttgtaca acgagacgac agaacaccca cgggaccgag cttcgaatta 20520

ttccggatga gcattcatca ggcgggcaag aatgtgaata aaggccggat aaaacttgtg 20580

cttatttttc tttacggtct ttaaaaaggc cgtaatatcc aggaacggtc tggttatagg 20640

tacattgagc aactgactga aatgcctcaa aatgttcttt acgatgccat tgggatatat 20700

caacggtggt atatccagtg atttttttct ccattttagc ttccttagct cctgaaaatc 20760

tcgataactc aaaaaatacg cccggtagtg atcttatttc attatggtga aagttggaac 20820

ctcttacgtg ccgatcaacg tctcattttc gccaaaagtt ggcccagggc ttcccggtat 20880

caacagggac accaggattt atttattctg cgaagtgatc ttccgtcaca ggtatttatt 20940

cgaagacgaa agggcctcgt gatacgccta tttttatagg ttaatgtcat gataataatg 21000

gtttcttaga cgtcaggtgg cacttttcgg ggaaatgtgc gcggaacccc tatttgttta 21060

tttttctaaa tacattcaaa tatgtatccg ctcatgagac aataaccctg ataaatgctt 21120

caataatatt gaaaaaggaa gagtatgagt attcaacatt tccgtgtcgc ccttattccc 21180

ttttttgcgg cattttgcct tcctgttttt gctcacccag aaacgctggt gaaagtaaaa 21240

gatgctgaag atcagttggg tgcacgagtg ggttacatcg aactggatct caacagcggt 21300

aagatccttg agagttttcg ccccgaagaa cgttttccaa tgatgagcac ttttaaagtt 21360

ctgctatgtg gcgcggtatt atcccgtgtt gacgccgggc aagagcaact cggtcgccgc 21420

atacactatt ctcagaatga cttggttgag tactcaccag tcacagaaaa gcatcttacg 21480

gatggcatga cagtaagaga attatgcagt gctgccataa ccatgagtga taacactgcg 21540

gccaacttac ttctgacaac gatcggagga ccgaaggagc taaccgcttt tttgcacaca 21600

catgggggat catgtaactc gccttgatcg ttgggaaccg gagctgaatg aagccatacc 21660

aaacgacgag cgtgacacca cgatgcctgt agcaatggca acaacgttgc gcaaactatt 21720

aactggcgaa ctacttactc tagcttcccg gcaacaatta atagactgga tggaggcgga 21780

taaagttgca ggaccacttc tgcgctcggc ccttccggct ggctggttta ttgctgataa 21840

atctggagcc ggtgagcgtg ggtctcgcgg tatcattgca gcactggggc cagatggtaa 21900

gccctcccgt atcgtagtta tctacacgac ggggagtcag gcaactatgg atgaacgaaa 21960

tagacagatc gctgagatag gtgcctcact gattaagcat tggtaactgt cagaccaagt 22020

ttactcatat atactttaga ttgatttaaa acttcatttt taatttaaaa ggatctaggt 22080

gaagatcctt tttgataatc tcatgaccaa aatcccttaa cgtgagtttt cgttccactg 22140

agcgtcagac cccgtagaaa agatcaaagg atcttcttga gatccttttt ttctgcgcgt 22200

aatctgctgc ttgcaaacaa aaaaaccacc gctaccagcg gtggtttgtt tgccggatca 22260

agagctacca actctttttc cgaaggtaac tggcttcagc agagcgcaga taccaaatac 22320

tgtccttcta gtgtagccgt agttaggcca ccacttcaag aactctgtag caccgcctac 22380

atacctcgct ctgctaatcc tgttaccagt ggctgctgcc agtggcgata agtcgtgtct 22440

taccgggttg gactcaagac gatagttacc ggataaggcg cagcggtcgg gctgaacggg 22500

gggttcgtgc acacagccca gcttggagcg aacgacctac accgaactga gatacctaca 22560

gcgtgagcta tgagaaagcg ccacgcttcc cgaagggaga aaggcggaca ggtatccggt 22620

aagcggcagg gtcggaacag gagagcgcac gagggagctt ccagggggaa acgcctggta 22680

tctttatagt cctgtcgggt ttcgccacct ctgacttgag cgtcgatttt tgtgatgctc 22740

gtcagggggg cggagcctat ggaaaaacgc cagcaacgcg gcctttttac ggttcctggc 22800

cttttgctgg ccttttgctc acatgttctt tcctgcgtta tcccctgatt ctgtggataa 22860

ccgtattacc gcctttgagt gagctgatac cgctcgccgc agccgaacga ccgagcgcag 22920

cgagtcagtg agcgaggaag cggaagagcg cctgatgcgg tattttctcc ttacgcatct 22980

gtgcggtatt tcacaccgca tatggtgcac tctcagtaca atctgctctg atgccgcata 23040

gttaagccag tatacactcc gctatcgcta cgtgactggg tcatggctgc gccccgacac 23100

ccgccaacac ccgctgacgc gccctgacgg gcttgtctgc tcccggcatc cgcttacaga 23160

caagctgtga ccgtctccgg gagctgcatg tgtcagaggt tttcaccgtc atcaccgaaa 23220

cgcgcgaggc agggtgcctt gatgtgggcg ccggcggtcg agtggcgacg gcgcggcttg 23280

tccgcgccct ggtagattgc ctggccgtag gccagccatt tttgagcggc cagcggccgc 23340

gataggccga cgcgaagcgg cggggcgtag ggagcgcagc gaccgaaggg taggcgcttt 23400

ttgcagctct tcggctgtgc gctggccaga cagttatgca caggccaggc gggttttaag 23460

agttttaata agttttaaag agttttaggc ggaaaaatcg ccttttttct cttttatatc 23520

agtcacttac atgtgtgacc ggttcccaat gtacggcttt gggttcccaa tgtacgggtt 23580

ccggttccca atgtacggct ttgggttccc aatgtacgtg ctatccacag gaaagagacc 23640

ttttcgacct ttttcccctg ctagggcaat ttgccctagc atctgctccg tacattagga 23700

accggcggat gcttcgccct cgatcaggtt gcggtagcgc atgactagga tcgggccagc 23760

ctgccccgcc tcctccttca aatcgtactc cggcaggtca tttgacccga tcagcttgcg 23820

cacggtgaaa cagaacttct tgaactctcc ggcgctgcca ctgcgttcgt agatcgtctt 23880

gaacaaccat ctggcttctg ccttgcctgc ggcgcggcgt gccaggcggt agagaaaacg 23940

gccgatgccg ggatcgatca aaaagtaatc ggggtgaacc gtcagcacgt ccgggttctt 24000

gccttctgtg atctcgcggt acatccaatc agctagctcg atctcgatgt actccggccg 24060

cccggtttcg ctctttacga tcttgtagcg gctaatcaag gcttcaccct cggataccgt 24120

caccaggcgg ccgttcttgg ccttcttcgt acgctgcatg gcaacgtgcg tggtgtttaa 24180

ccgaatgcag gtttctacca ggtcgtcttt ctgctttccg ccatcggctc gccggcagaa 24240

cttgagtacg tccgcaacgt gtggacggaa cacgcggccg ggcttgtctc ccttcccttc 24300

ccggtatcgg ttcatggatt cggttagatg ggaaaccgcc atcagtacca ggtcgtaatc 24360

ccacacactg gccatgccgg ccggccctgc ggaaacctct acgtgcccgt ctggaagctc 24420

gtagcggatc acctcgccag ctcgtcggtc acgcttcgac agacggaaaa cggccacgtc 24480

catgatgctg cgactatcgc gggtgcccac gtcatagagc atcggaacga aaaaatctgg 24540

ttgctcgtcg cccttgggcg gcttcctaat cgacggcgca ccggctgccg gcggttgccg 24600

ggattctttg cggattcgat cagcggccgc ttgccacgat tcaccggggc gtgcttctgc 24660

ctcgatgcgt tgccgctggg cggcctgcgc ggccttcaac ttctccacca ggtcatcacc 24720

cagcgccgcg ccgatttgta ccgggccgga tggtttgcga ccgctcacgc cgattcctcg 24780

ggcttggggg ttccagtgcc attgcagggc cggcagacaa cccagccgct tacgcctggc 24840

caaccgcccg ttcctccaca catggggcat tccacggcgt cggtgcctgg ttgttcttga 24900

ttttccatgc cgcctccttt agccgctaaa attcatctac tcatttattc atttgctcat 24960

ttactctggt agctgcgcga tgtattcaga tagcagctcg gtaatggtct tgccttggcg 25020

taccgcgtac atcttcagct tggtgtgatc ctccgccggc aactgaaagt tgacccgctt 25080

catggctggc gtgtctgcca ggctggccaa cgttgcagcc ttgctgctgc gtgcgctcgg 25140

acggccggca cttagcgtgt ttgtgctttt gctcattttc tctttacctc attaactcaa 25200

atgagttttg atttaatttc agcggccagc gcctggacct cgcgggcagc gtcgccctcg 25260

ggttctgatt caagaacggt tgtgccggcg gcggcagtgc ctgggtagct cacgcgctgc 25320

gtgatacggg actcaagaat gggcagctcg tacccggcca gcgcctcggc aacctcaccg 25380

ccgatgcgcg tgcctttgat cgcccgcgac acgacaaagg ccgcttgtag ccttccatcc 25440

gtgacctcaa tgcgctgctt aaccagctcc accaggtcgg cggtggccca tatgtcgtaa 25500

gggcttggct gcaccggaat cagcacgaag tcggctgcct tgatcgcgga cacagccaag 25560

tccgccgcct ggggcgctcc gtcgatcact acgaagtcgc gccggccgat ggccttcacg 25620

tcgcggtcaa tcgtcgggcg gtcgatgccg acaacggtta gcggttgatc ttcccgcacg 25680

gccgcccaat cgcgggcact gccctgggga tcggaatcga ctaacagaac atcggccccg 25740

gcgagttgca gggcgcgggc tagatgggtt gcgatggtcg tcttgcctga cccgcctttc 25800

tggttaagta cagcgataac cttcatgcgt tccccttgcg tatttgttta tttactcatc 25860

gcatcatata cgcagcgacc gcatgacgca agctgtttta ctcaaataca catcaccttt 25920

ttagacggcg gcgctcggtt tcttcagcgg ccaagctggc cggccaggcc gccagcttgg 25980

catcagacaa accggccagg atttcatgca gccgcacggt tgagacgtgc gcgggcggct 26040

cgaacacgta cccggccgcg atcatctccg cctcgatctc ttcggtaatg aaaaacggtt 26100

cgtcctggcc gtcctggtgc ggtttcatgc ttgttcctct tggcgttcat tctcggcggc 26160

cgccagggcg tcggcctcgg tcaatgcgtc ctcacggaag gcaccgcgcc gcctggcctc 26220

ggtgggcgtc acttcctcgc tgcgctcaag tgcgcggtac agggtcgagc gatgcacgcc 26280

aagcagtgca gccgcctctt tcacggtgcg gccttcctgg tcgatcagct cgcgggcgtg 26340

cgcgatctgt gccggggtga gggtagggcg ggggccaaac ttcacgcctc gggccttggc 26400

ggcctcgcgc ccgctccggg tgcggtcgat gattagggaa cgctcgaact cggcaatgcc 26460

ggcgaacacg gtcaacacca tgcggccggc cggcgtggtg gtgtcggccc acggctctgc 26520

caggctacgc aggcccgcgc cggcctcctg gatgcgctcg gcaatgtcca gtaggtcgcg 26580

ggtgctgcgg gccaggcggt ctagcctggt cactgtcaca acgtcgccag ggcgtaggtg 26640

gtcaagcatc ctggccagct ccgggcggtc gcgcctggtg ccggtgatct tctcggaaaa 26700

cagcttggtg cagccggccg cgtgcagttc ggcccgttgg ttggtcaagt cctggtcgtc 26760

ggtgctgacg cgggcatagc ccagcaggcc agcggcggcg ctcttgttca tggcgtaatg 26820

tctccggttc tagtcgcaag tattctactt tatgcgacta aaacacgcga caagaaaacg 26880

ccaggaaaag ggcagggcgg cagcctgtcg cgtaacttag gacttgtgcg acatgtcgtt 26940

ttcagaagac ggctgcactg aacgtcagaa gccgactgca ctatagcagc ggaggggttg 27000

gatcgatcct gctcgcgcag gctgggtgcc aagctctcgg gtaacatcaa ggcccgatcc 27060

ttggagccct tgccctcccg cacgatgatc gtgccgtgat cgaaatccag atccttgacc 27120

cgcagttgca aaccctcact gatccgcatg cccgttccat acagaagctg ggcgaacaaa 27180

cgatgctcgc cttccagaaa accgaggatg cgaaccactt catccggggt cagcaccacc 27240

ggcaagcgcc gcgacggccg aggtcttccg atctcctgaa gccagggcag atccgtgcac 27300

agcaccttgc cgtagaagaa cagcaaggcc gccaatgcct gacgatgcgt ggagaccgaa 27360

accttgcgct cgttcgccag ccaggacaga aatgcctcga cttcgctgct gcccaaggtt 27420

gccgggtgac gcacaccgtg gaaacggatg aaggcacgaa cccagtggac ataagcctgt 27480

tcggttcgta agctgtaatg caagtagcgt atgcgctcac gcaactggtc cagaaccttg 27540

accgaacgca gcggtggtaa cggcgcagtg gcggttttca tggcttgtta tgactgtttt 27600

tttggggtac agtctatgcc tcgggcatcc aagcagcaag cgcgttacgc cgtgggtcga 27660

tgtttgatgt tatggagcag caacgatgtt acgcagcagg gcagtcgccc taaaacaaag 27720

ttaaacatca tgagggaagc ggtgatcgcc gaagtatcga ctcaactatc agaggtagtt 27780

ggcgtcatcg agcgccatct cgaaccgacg ttgctggccg tacatttgta cggctccgca 27840

gtggatggcg gcctgaagcc acacagtgat attgatttgc tggttacggt gaccgtaagg 27900

cttgatgaaa caacgcggcg agctttgatc aacgaccttt tggaaacttc ggcttcccct 27960

ggagagagcg agattctccg cgctgtagaa gtcaccattg ttgtgcacga cgacatcatt 28020

ccgtggcgtt atccagctaa gcgcgaactg caatttggag aatggcagcg caatgacatt 28080

cttgcaggta tcttcgagcc agccacgatc gacattgatc tggctatctt gctgacaaaa 28140

gcaagagaac atagcgttgc cttggtaggt ccagcggcgg aggaactctt tgatccggtt 28200

cctgaacagg atctatttga ggcgctaaat gaaaccttaa cgctatggaa ctcgccgccc 28260

gactgggctg gcgatgagcg aaatgtagtg cttacgttgt cccgcatttg gtacagcgca 28320

gtaaccggca aaatcgcgcc gaaggatgtc gctgccgact gggcaatgga gcgcctgccg 28380

gcccagtatc agcccgtcat acttgaagct agacaggctt atcttggaca agaagaagat 28440

cgcttggcct cgcgcgcaga tcagttggaa gaatttgtcc actacgtgaa aggcgagatc 28500

accaaggtag tcggcaaata atgtctaaca attcgttcaa gccgacgccg cttcgcggcg 28560

cggcttaact caagcgttag atgcactaag cacataattg ctcacagcca aactatcagg 28620

tcaagtctgc ttttattatt tttaagcgtg cataataagc cctacacaaa ttgggagata 28680

tatcatgaaa ggctggcttt ttcttgttat cgcaatagtt ggcgaagtaa tcgcaacatc 28740

cgcattaaaa tctagcgagg gctttactaa gctagcttgc ttggtcgttc cggtaccgtg 28800

aacgtcggct cgattgtacc tgcgttcaaa tactttgcga tcgtgttgcg cgcctgcccg 28860

gtgcgtcggc tgatctcacg gatcgactgc ttctctcgca acgccatccg acggatgatg 28920

tttaaaagtc ccatgtggat cactccgttg ccccgtcgct caccgtgttg gggggaaggt 28980

gcacatggct cagttctcaa tggaaattat ctgcctaacc ggctcagttc tgcgtagaaa 29040

ccaacatgca agctccaccg ggtgcaaagc ggcagcggcg gcaggatata ttcaattgta 29100

aatggcttca tgtccgggaa atctacatgg atcagcaatg agtatgatgg tcaatatgga 29160

gaaaaagaaa gagtaattac caattttttt tcaattcaaa aatgtagatg tccgcagcgt 29220

tattataaaa tgaaagtaca ttttgataaa acgacaaatt acgatccgtc gtatttatag 29280

gcgaaagcaa taaacaaatt attctaattc ggaaatcttt atttcgacgt gtctacattc 29340

acgtccaaat gggggcttag atgagaaact tcacgatcga tgccttgatt tcgccattcc 29400

cagataccca tttcatcttc agattggtct gagattatgc gaaaatatac actcatatac 29460

ataaatactg acagtttgag ctaccaattc agtgtagccc attaccttac ataattcact 29520

caaatgctag gcagtctgtc aactcggcgt caatttgtcg gccactatac gatagttgcg 29580

caaattttca aagtcctggc ctaacatcac acctctgtcg gcggcgggtc ccatttgtga 29640

taaatccacc atcacaatag atagtctaat ggacgaaaaa ggcgaatatt tcgatgctga 29700

gattcgacgc aattaattcg agaaaaatcc cgtgattgat gctgttgagt taccaataat 29760

atgggcagcg aaggccattt aattataaga tctggtacca agctgggatc ccagcttgca 29820

tgcccatcga tgggtcgac 29839

<210> 37
<211> 530
<212> DNA
<213> Cauliflower mosaic virus

<400> 37
ccatggagtc aaagattcaa atagaggacc taacagaact cgccgtaaag actggcgaac 60

agttcataca gagtctctta cgactcaatg acaagaagaa aatcttcgtc aacatggtgg 120

agcacgacac gcttgtctac tccaaaaata tcaaagatac agtctcagaa gaccaaaggg 180

caattgagac ttttcaacaa agggtaatat ccggaaacct cctcggattc cattgcccag 240

ctatctgtca ctttattgtg aagatagtgg aaaaggaagg tggctcctac aaatgccatc 300

attgcgataa aggaaaggcc atcgttgaag atgcctctgc cgacagtggt cccaaagatg 360

gacccccacc cacgaggagc atcgtggaaa aagaagacgt tccaaccacg tcttcaaagc 420

aagtggattg atgtgatatc tccactgacg taagggatga cgcacaatcc cactatcctt 480

cgcaagaccc ttcctctata taaggaagtt catttcattt ggagaggaca 530

<210> 38
<211> 530
<212> DNA
<213> Cauliflower mosaic virus

<400> 38
ccatggagtc aaagattcaa atagaggacc taacagaact cgccgtaaag actggcgaac 60

agttcataca gagtctctta cgactcaatg acaagaagaa aatcttcgtc aacatggtgg 120

agcacgacac acttgtctac tccaaaaata tcaaagatac agtctcagaa gaccaaaggg 180

caattgagac ttttcaacaa agggtaatat ccggaaacct cctcggattc cattgcccag 240

ctatctgtca ctttattgtg aagatagtgg aaaaggaagg tggctcctac aaatgccatc 300

attgcgataa aggaaaggcc atcgttgaag atgcctctgc cgacagtggt cccaaagatg 360

gacccccacc cacgaggagc atcgtggaaa aagaagacgt tccaaccacg tcttcaaagc 420

aagtggattg atgtgatatc tccactgacg taagggatga cgcacaatcc cactatcctt 480

cgcaagaccc ttcctctata taaggaagtt catttcattt ggagaggaca 530

<210> 39
<211> 732
<212> DNA
<213> Artificial Sequence

<220>
<223> DT-A gene with intron1 of Caster bean Cat1 gene

<400> 39
atggatcccg acgatgtaaa tttctagttt ttctccttca ttttcttggt taggaccctt 60

ttctcttttt atttttttga gctttgatct ttctttaaac tgatctattt tttaattgat 120

tggttatggt gtaaatatta catagcttta actgataatc tgattacttt atttcgtgtg 180

tctatgatga tgatgatagt tacaggttgt cgactcttct aaatcttttg tgatggaaaa 240

cttttcttcg taccacggga ctaaacctgg ttatgtagat tccattcaaa aaggtataca 300

aaagccaaaa tctggtacac aaggaaatta tgacgatgat tggaaagggt tttatagtac 360

cgacaataaa tacgacgctg cgggatactc tgtagataat gaaaacccgc tctctggaaa 420

agctggaggc gtggtcaaag tgacgtatcc aggactgacg aaggttctcg cactaaaagt 480

ggataatgcc gaaactatta agaaagagtt aggtttaagt ctcactgaac cgttgatgga 540

gcaagtcgga acggaagagt ttatcaaaag gttcggtgat ggtgcttcgc gtgtagtgct 600

cagccttccc ttcgctgagg ggagttctag cgttgaatat attaataact gggaacaggc 660

gaaagcgtta agcgtagaac ttgagattaa ttttgaaacc cgtggaaaac gtggccaaga 720

tgcgatgtat ga 732

<210> 40
<211> 199
<212> DNA
<213> Cauliflower mosaic virus

<400> 40
gctgaaatca ccagtctctc tctacaaatc tatctctctc tataataatg tgtgagtagt 60

tcccagataa gggaattagg gttcttatag ggtttcgctc atgtgttgag catataagaa 120

acccttagta tgtatttgta tttgtaaaat acttctatca ataaaatttc taattcctaa 180

aaccaaaatc caggggtac 199

<210> 41
<211> 200
<212> DNA
<213> Cauliflower mosaic virus

<400> 41
gctgaaatca ccagtctctc tctacaaatc tatctctctc tataataatg tgtgagtagt 60

tcccagataa gggaattagg gttcttatag ggtttcgctc atgtgttgag catataagaa 120

acccttagta tgtatttgta tttgtaaaat acttctatca ataaaatttc taattcctaa 180

aaccaaaatc caggggtacc 200

<210> 42
<211> 3000
<212> DNA
<213> Oryza sativa

<400> 42
tgactgttta gtgaaaataa ctcatgaaat attttgcaga tggatcctta tctaagaatt 60

ggtgaagact tgcaactcta tgtaaggttg caatctgatc taggtaacta tggttctgat 120

agcgatcaag aaatcgctag atctgtactt tctgactgca ggacaaaagt ggggattaat 180

gatcagcgag tacttgatgt agttgcttgt gcactgtgta atttaactga ggtaaaggtt 240

gattttgcct tctatttgct gttgacactg cacgttgtgt tcttattgag gtcacatgct 300

gttgacagca atggttgagt gcagttatct aatattcttg gaattgctat tgccagatgg 360

acaaggatgt actggtgaag gagctcacag aaatgtttac acctgaagag gtgcccttgt 420

ttgggtcaaa ttcagcattt gactgggcca attttcatgt tcaggcattt tctgatgaat 480

ccctttcttt tgatgaggta ggtacattaa aactggtgtc tttaatgatt catctggtgg 540

aagctcttat aaaattcttt gatggtgcaa tgatccttgt aactgtacct taccattatg 600

aaaattattg ttactttttg tatcttctca gttgtcttct gcattgtagg agtgttcaag 660

aacctcttca gtagatggtg ggttgcatga gtcaccaatt acaaacaccg gtagctcgat 720

atcaaagact acgatgccac aatctgttcc tcgtgtctta ggtgttggcc agttgcttga 780

atcggtgagt acttatcagc tgcctttgca catggtttgc tagttgctgc tactcagcta 840

gctctagttg ccctaatctt attaattgtt aaggtggcat ggaaccataa catgttgtgt 900

ttgtacccct ttctgctgct gtcaaaatag tatttatcac gtggtggatt cggctacaaa 960

tatattatgg agcatttgaa tctatgtaac attttctcta ctgtgaactc atatctttgt 1020

aatcaggcgt tacatgtagc cggccaagtt gcgggagcat ctgtttcgac ctccccgctc 1080

ccatacggca caatgaccag tcaatgcgaa gccttgggat caggcactag gaagaagctc 1140

tcaagctggc ttgtcaatgg ccatgactct acgccagaca atcctgctcc aagccttcca 1200

tctgcgcagc atttcatcat tcctaaagta catgatttgt cctgtctttc tcccatgtca 1260

ttctggaatg ttgcctattc tgcatctgtg tgtatcatct ggccctcacg atcgcaatcg 1320

acattcatcc aggtaaattc atgcggtttc gagagcagca tccggacgac tttggagcct 1380

tgctcggcag tgaagctccc acccgccagc cccttcgaca acttcctgaa ggctgcatat 1440

cgtgcccagt agcagccatg acaagccttc gagattatgc ctaaccatgt ttagtagtgc 1500

taggatgtag tacctgagca tcagaactag attatctttt gaaagacttg gccgatctgg 1560

cttatccttt attatctgta gcactgttag ttgtagtagc ttgtgtatca atgtatggct 1620

atgtctgtgt ccctccagta aatgacaatc gtatatgtac tatgtagtat gaccatgtag 1680

cccactagta aatgtttgta gggtatgtat gacaatgtat gaccatattg tttttttgag 1740

atgtaggatt ctatttcaga ctcgagtaat ctgtggggga catgttcgtc gacctccggt 1800

gctatttttc cttttatcac tgcaaggatt agatggtttc ctgcaatatt tgcatgctat 1860

tcgcgcggat gaaattgtga agtcatgggt ttctcaagtc cacaatcatt agttcacatt 1920

attacggcgt tcatgaatat tacattttgg aggatcttat ttctatagtt tttctatata 1980

gctctttgaa tcaagaaatg tttttcatgt gatctaatca aaaggtcatt tctatgtttg 2040

caattatcta ttttacattt acatttctat caaaatcttt tttttgtttt ttttcctatt 2100

tatctgtttt ttttattcct gcgattcaaa gtgacgctta aaatttaggg aaaattgcaa 2160

aaaccatcct ataaatcact tgaaacttga cattccactt tataagccat tttattgcaa 2220

ataccaccca tctactctgt ctaatcgaat caacatgttt tcatgaatta tgagctgtca 2280

atgaagcttt taccaaaatt aaatatttat atagtacagt cttgacactt ttgtattgag 2340

tttcgctgta ttttaaaaaa aattaattaa acatgaagaa taaatctgat acaaaagcac 2400

ttggacaaga ttgactcgac tagttttaac ctgtggtatc cctaccccag cgataatggg 2460

ttgattcggt taaacacgtt cgcactagaa tggtttttac aaaaaaaaag aactgagtag 2520

gggtggacat ttacaataaa ataacttatg agatgtaatg tgaaatttca agtgatttat 2580

agggttgttt ttttttaaat tccctagaat ttaacgggtg tgtgtctggt ggacgtccgt 2640

ggtgcgcgcg cgcgcgcgag ggaatgatca aaaacctttt cggggcgtca cgtaagccca 2700

tgacccgacg tccaaaggcc taccaacacc gtcggcatgg cccgcgtctt ctcctcgtct 2760

tcctctctct cgagtctcga agaagacaca cactattctc tcttcttctt accaccctct 2820

cccggataag aggcgcaacc aaagccccca ccctcgccca aaacccccac gagccgcggc 2880

catggcgacc accaccacca ccccctcctc ctctctcacc gcccctctcc tccgcccgag 2940

ctcgaacgcg aaccccgccc cgagatctct gccgctcctc agctgcactc ccctcttccg 3000

<210> 43
<211> 838
<212> DNA
<213> Oryza sativa

<400> 43
gtcattcata tgcttgagaa gagagtcggg atagtccaaa ataaaacaaa ggtaagatta 60

cctggtcaaa agtgaaaaca tcagttaaaa ggtggtataa agtaaaatat cggtaataaa 120

aggtggccca aagtgaaatt tactcttttc tactattata aaaattgagg atgtttttgt 180

cggtactttg atacgtcatt tttgtatgaa ttggttttta agtttattcg cttttggaaa 240

tgcatatctg tatttgagtc gggttttaag ttcgtttgct tttgtaaata cagagggatt 300

tgtataagaa atatctttaa aaaaacccat atgctaattt gacataattt ttgagaaaaa 360

tatatattca ggcgaattct cacaatgaac aataataaga ttaaaatagc tttcccccgt 420

tgcagcgcat gggtattttt tctagtaaaa ataaaagata aacttagact caaaacattt 480

acaaaaacaa cccctaaagt tcctaaagcc caaagtgcta tccacgatcc atagcaagcc 540

cagcccaacc caacccaacc caacccaccc cagtccagcc aactggacaa tagtctccac 600

acccccccac tatcaccgtg agttgtccgc acgcaccgca cgtctcgcag ccaaaaaaaa 660

aaaaagaaag aaaaaaaaga aaaagaaaaa acagcaggtg ggtccgggtc gtgggggccg 720

gaaacgcgag gaggatcgcg agccagcgac gaggccggcc ctccctccgc ttccaaagaa 780

acgcccccca tcgccactat atacataccc ccccctctcc tcccatcccc ccaaccct 838

<210> 44
<211> 1026
<212> DNA
<213> Artificial Sequence

<220>
<223> hpt (Hygromycin phosphotransferase gene)

<400> 44
atgaaaaagc ctgaactcac cgcgacgtct gtcgagaagt ttctgatcga aaagttcgac 60

agcgtctccg acctgatgca gctctcggag ggcgaagaat ctcgtgcttt cagcttcgat 120

gtaggagggc gtggatatgt cctgcgggta aatagctgcg ccgatggttt ctacaaagat 180

cgttatgttt atcggcactt tgcatcggcc gcgctcccga ttccggaagt gcttgacatt 240

ggggaattca gcgagagcct gacctattgc atctcccgcc gtgcacaggg tgtcacgttg 300

caagacctgc ctgaaaccga actgcccgct gttctgcagc cggtcgcgga ggccatggat 360

gcgatcgctg cggccgatct tagccagacg agcgggttcg gcccattcgg accgcaagga 420

atcggtcaat acactacatg gcgtgatttc atatgcgcga ttgctgatcc ccatgtgtat 480

cactggcaaa ctgtgatgga cgacaccgtc agtgcgtccg tcgcgcaggc tctcgatgag 540

ctgatgcttt gggccgagga ctgccccgaa gtccggcacc tcgtgcacgc ggatttcggc 600

tccaacaatg tcctgacgga caatggccgc ataacagcgg tcattgactg gagcgaggcg 660

atgttcgggg attcccaata cgaggtcgcc aacatcttct tctggaggcc gtggttggct 720

tgtatggagc agcagacgcg ctacttcgag cggaggcatc cggagcttgc aggatcgccg 780

cggctccggg cgtatatgct ccgcattggt cttgaccaac tctatcagag cttggttgac 840

ggcaatttcg atgatgcagc ttgggcgcag ggtcgatgcg acgcaatcgt ccgatccgga 900

gccgggactg tcgggcgtac acaaatcgcc cgcagaagcg cggccggccg tctggaccga 960

tggctgtgta gaagtactcg ccgatagtgg aaaccgacgc cccagcactc gtccgggatc 1020

ctctag 1026

<210> 45
<211> 977
<212> DNA
<213> Artificial Sequence

<220>
<223> delta En/Spm

<400> 45
caacttctgt ccaagaccaa ttgatgccat tgggtgtgat aggagggcaa atgatgccgt 60

gggcacctcg ccagccaggc atttggccac cgatgcaaac acagatgcca ccgccgatgc 120

cgtggggatt tcctcctcgt gggcagtcac aatcaccagg attgccctca cactcaccag 180

gatcagtacg ttaagttgat atcctttgca tctctatttg cttcgttgtt taagcagtta 240

ctagaaaaca tgcatgtata tgttgcagtc tatgtatatg tttaattagt tactcggtaa 300

actaacaaat gtttgtttct tttaaagggt tcaggctcac atcatgctag tccgcctccg 360

gatcagagca cgtttatgga cttattgatg aacacaagtg gcggcggctc caatgaccca 420

ccaacagaat gaattaatat ggaggcttgt gtggaactta ctatgattgc gttttgtatg 480

gactttaact tgttttagat ggatttgaac ttctttcgta tggacttgaa cttgtatgaa 540

tattgaatat ggtgcttgtg ttatgttatg ttgaatatgg tgcttgtgtt gtgatatatt 600

gaatgttgtg cttatattgt gctgttatgg aggcttccca tccggggagg gagaaaaata 660

aaattggata ttaaaaaaaa ttattcacta agagtgtcgg cccccacact cttatatgcg 720

cccaggtagc ttactgatgt gcgcgcagta agagtgacgg ccacggtact ggccgacact 780

tttaacataa gagtgtcggt tgcttgttga accgacactt ttaacataag agcgtcggtc 840

cccacacttc tatacgaata agagcgtcca ttttagagtg acggctaaga gtgtcggtca 900

accgacactc ttatacttag agtgtcggct tatttcagta agagtgtggg gttttggccg 960

aagctgggcc cgctagc 977

<210> 46
<211> 769
<212> DNA
<213> Oryza sativa

<400> 46
ccagtgaaag cagtgaattg aagcattccc gaaacccact ggaatgatct agtactcact 60

ctacgatgta cagtgaagta atacttcaaa actggtgtaa tttggtatgc caaaaggact 120

ccatagtttc acgacatatt tccaaacggt tcaggatcag tactgcccat ctgcctgggg 180

cccacactag cgggcaattg gttctcgtag tttctcgttc tcaatcaatc attccatact 240

cgctatcccc tccatcacag aataaatgca acaatgagtt tccgtgtaca aatttaatcg 300

ttcgtcttat ttaaaatatt ttttaaaaaa ctaaaaaaca aaagtcacgc ataaagtact 360

attcatgttt tataatctaa taacagtata aatactaatc ataaaaaaaa attcaaataa 420

gatggacgat taaagttgaa cactgaaatt catggctgct tttgttttga gactgaggga 480

gtacacgata agatttgatc gcaatcaaag taacctacat caaagaagca agatatgtgg 540

gggaaaaatg aatactctag agcaaattaa ggtgagcccc gctttgtaga ggctgatgga 600

gtactggagc gacggaagcg aagcagatcg agtgtgctgt aaagcgaaac gagcaagaac 660

cagagaagtc cagagatttc aggacagatt agttgtgaac ctataaatat cctgcctcat 720

tccccaacct ccatccatcg agccaagact gaagcatttg atcgagctc 769

<210> 47
<211> 2216
<212> DNA
<213> Artificial Sequence

<220>
<223> AcTPase4x gene with intron and SV40 NLS

<400> 47
atggctccaa agaagaagag aaaggtcatg gccatcgtgc atgagccaca gccacaaccg 60

caaccacagc cagagcctca gcctcaacct cagcctgagc cagaggagga ggccccacaa 120

aagcgcgcta agaagtgcac ctccgacgtc tggcagcact tcaccaagaa ggagatcgag 180

gtcgaggttg acggcaagaa gtacgtccag gtctggggcc actgcaactt cccaaactgc 240

aaggccaagt acagggccga gggccatcat ggcacatccg gctttaggaa ccacctcagg 300

acctcccact ctctcgtgaa gggccaactc tgcctcaagt ccgagaagga ccacggcaag 360

gacatcaacc tcatcgagcc gtacaagtac gacgaggtgg tgtccctcaa gaagctccac 420

ctcgccatca ttatgcacga gtacccgttc aacatcgtcg agcacgccta cttcgtcgag 480

ttcgtgaagt ccctcaggcc gcacttcccg atcaagtcta gagtgaccgc ccgcaagtac 540

atcatggacc tctaccttga ggagaaggag aagctctacg gcaagctcaa ggacgtgcag 600

tccaggttct ccaccacgat ggacatgtgg accagctgcc agaacaagtc ctacatgtgc 660

gtgaccatcc actggatcga cgacgactgg tgcctccaga agaggatcgt cggcttcttc 720

catgtggccg gcagacatac aggccagagg ctctcccaaa ccttcaccgc gatcatggtc 780

aagtggaaca tcgagaagaa gctgttcgcc ctctccctcg ataacgcctc cgctaatgag 840

gtggccgtgc acgacatcat cgaggacctc caggacaccg actccaacct tgtgtgcgac 900

ggcgccttct ttcatgttcg ctgcgcctgc cacatcctca acctggttgc taaggatggc 960

ctcgccgtga tcgccggcac cattgagaag atcaaggcca tcgtcctcgc cgtcaagtcc 1020

tctccacttc agtgggagga gctgatgaag tgcgcgtccg agtgcgatct cgacaagtcc 1080

aagggcatca gctacgccgt gtccaccagg tggaatagca cctacctcat gctccgcgac 1140

gccctctact acaagccagc cctcatcagg ctcaagacct ccgatccaag acggtatgtt 1200

tgtctcaatt gttgtacatg tcatcattat aaattctcaa ttaatcaaat gtcaattatt 1260

gtagctacga tgccatctgc ccaaaggccg aggagtggaa gatggcgctc accctcttca 1320

agtgcctgaa gaagttcttc gacctcaccg agctgctctc cggcacccaa tactctaccg 1380

ccaacctctt ctacaagggc ttctgcgaga tcaaggacct gatcgcccaa tggtgcgtgc 1440

acgagaagtt cgtgattcgc agaatggccg tggccatgag cgagaagttt gagaagtact 1500

ggaaggtgtc caatatcgcc ctcgcggtgg cctgcttcct cgatccaagg tacaagaaga 1560

tcctgatcga gttctacatg aagaagtttc acggcgactc ctacaaggtg cacgtcgacg 1620

atttcgtgcg cgtgatccgc aagctctacc agttctactc ctcctgctct ccatccgccc 1680

caaagaccaa gaccaccacc aacgacagca tggacgacac cctcatggag aacgaggacg 1740

acgagttcca gaactacctc cacgagctga aggactacga ccaggtcgag agcaacgagc 1800

tggacaagta catgtccgag ccgctcttga agcactccgg ccagttcgat atcctctcat 1860

ggtggcgcgg cagagtggcc gagtacccaa tcctcacaca gatcgccaga gatgtgctcg 1920

ccatccaggt gtcaacagtg gcttctgagt ctgccttttc cgctggcggc agggtggtgg 1980

atccatacag aaatagactc ggctccgaga tcgtcgaggc cctcatctgc actaaggact 2040

gggtggcagc ctctaggaag ggcgccacat acttcccgac catgattggc gaccttgagg 2100

tgctcgacag cgttatcgcc gctgccacca accacgagaa ccacatggac gaggatgagg 2160

acgccatcga gttcagcaag aacaatgagg acgtggccag cggctcctcc ccatga 2216

<210> 48
<211> 253
<212> DNA
<213> Artificial Sequence

<220>
<223> tNOS (NOS terminator)

<400> 48
gatcgttcaa acatttggca ataaagtttc ttaagattga atcctgttgc cggtcttgcg 60

atgattatca tataatttct gttgaattac gttaagcatg taataattaa catgtaatgc 120

atgacgttat ttatgagatg ggtttttatg attagagtcc cgcaattata catttaatac 180

gcgatagaaa acaaaatata gcgcgcaaac taggataaat tatcgcgcgc ggtgtcatct 240

atgttactag atc 253

<210> 49
<211> 712
<212> DNA

<213>  Artificial Sequence

<220>
<223>  EGFP gene

<400>  49
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gt             712


<210>  50
<211>  2998
<212>  DNA
<213>  Oryza sativa

<400>  50
gctccctcct cacttactct gtcgtggctc cactcgctct accctaactg actcggtctt      60

gcaggagccg gaggtgcgct cgggccgtgg cgaccgccgc cgccgccgct ggccacgggg     120

ccgctcatca gaggagcggg atttggtcga tcaggtgggg gggggggttg attttgtgac     180

gaaccatttg tagctctgca tcgtctggtt gggggagggt tgggtgggga tgcgagggag     240

ctgatgatgc tgtggttgtt gtgtgtttcg tagggatgat ttggtggtgc cgaggtcgcc     300

ctacttccct gtggagtatg cgtcggggca ggaacgcggg ccatcgccca tggtgatgga     360

gcggttccag agcgtcgtca gccagctctt ccagcacgta cgggagcact agattgttgc     420

acaagtttat ttgtggaggg ttctgtgtgc tgtctgactc tgtgtgattg tggaacagag     480

gattatccgg tgtggtggac ccgtggagga tgatatggcg aacatcatcg ttgcccagct     540

gctatatctc gatgccatcg atcctaacaa ggtgaaaatg atgaataaga cgctatccga     600

catccaattc ctatttccta taccaatagc tgccaaagct ttgttacgaa ctgcttactc     660

taaaaatact gaggcgatga agggagtggt gctcactgta tttacaattt tacatgaact     720

atatggtgtc ttgttttaat ggatgattgt tgttaatccc cagataaaat ggtcctttta     780

tctgtcaata ctcaatacca gacactaaag taccattgct actcactttg ggttatatat 840

gagtccttgt tagtcctagc ttaggctgtg attcagttat gtagttgtga cctcaaacct 900

tatggtgagg cttgtattgc gatttcatct accaggacct gattccatgg caccatatat 960

tcatgatttc gtacttacat tcagtactcg attcttgact tcctctcaac attcctgtag 1020

gatatcatta tgtatgtgaa ttctcctgga ggatcagtga cagctggtaa taactcatcc 1080

attttttttc atagctccat ctcactcttg gaacatttgt ttcacaccaa actgcatttt 1140

gtgtttaggg atggccatat tcgacacgat gaagcatatc agacctgatg tttccacagt 1200

ttgtattgga cttgctgcaa ggtatttagt actgaaaaac tgatatcctt aattggcaat 1260

tgatttggat ttgtgacgct ctgcaattta tgcatcttat aatgtttaat tctttagcat 1320

tctgttcaat tattcaataa gggtttcagt tgctatattc tggtcaacat gtaaacatgc 1380

acctgtttcc agttgtatat cccgcctaat tccttctcct ttcataatgt gggaagcaca 1440

ccaaatcatt gattgtatca ggcatgttct attcagtata atcagcctca atctaggatt 1500

ttgggttgac tgagatcaaa ttttgtgctg cgcatgttag caaagaagct ttgtaccctc 1560

atacctcctt aatcaaaata taatttttca aactttatgt ttgtgagaat ccatatggat 1620

gttgataaca ggagagtttc ttcatttcat gtactttatc aatgtttagc caacaatgca 1680

aattcaggag tgcttatcag tattccaggc acacagttta ttagtaaaac atgataactg 1740

ttcagctgca aatctgtaac catttctttc tcattgtact tttggaatgc tctcttttag 1800

ctatccatct tgccttaagt tgcattgatt tcggttatgc actcattttc actgttttgt 1860

ttcccttttt ggcagtatgg gagcttttct gcttagtgct gggacaaaag gtaattgagt 1920

gattgttaat gcaagtacta catttattct acaactagac tgatctgaat ccttgaatac 1980

tgtacttgtg aatgtaacac agggaagcga tacagcttac ctaactcaag aataatgatc 2040

catcaacctc tcggaggagc ccaaggacaa gagactgatc ttgagatcca ggtaaatcca 2100

caaagttaaa ttgtgttttt atcaagatca ggacgacatc tcaatagcaa ataattaacc 2160

atatcattgt ttctctcttg ccggtatatg gttcgatcta catgaaatac atttatttcc 2220

acaagcgaat agcaactccg ctggctttct attgttactt gtatcaccag aagatttttc 2280

tgtcactgat tttccattat ttcttttagg ctaatgagat gctgcatcac aaggctaacc 2340

tgaatggata cctagcatac cacactgggc agcccctaga taagatcaac gtagatactg 2400

accgtgatta cttcatgagc gcgaaggagg caaaggagta tggtctaatt gatggagtta 2460

tcatgaatcc ccttaaagcc cttcaaccgc ttcctgcttc tagttagcca tggagtgctc 2520

aatctccacg gagcattttt tggttatctt ttagaactgt tattgcatcc actgttttta 2580

ttagcttggc aagatagttt tgcgattcca caagcaacca catcctgagg cttcaaagtt 2640

tgtacaatac agatgtacta ctaggaggat atcttctgcg atgaatattg caacttattt 2700

gatgtactat taggaggata tcttctgcga tgaatattgc aacttatttg atactccctc 2760

tgtttcatgc caagtcgatt tgattttttt tccttagaca attttctgta ggtttgacta 2820

aatttatcaa aaaattagca taatctacaa cactaaatta gttctattaa aataacattg 2880

aatatatttt gataatatgt ttgttgtatt aaaatgttgc tatatttttt atatacttga 2940

tcaaatttta aaaaagatta tctataaaaa aattaaaacg atttataata tgatgaaa 2998

<210> 51
<211> 35
<212> DNA
<213> Artificial Sequence

<220>
<223> PCR Primer

<400> 51
gctctccgcg ttttgggtac aggcaattca gacag 35

<210> 52
<211> 30
<212> DNA
<213> Artificial Sequence

<220>
<223> PCR Primer

<400> 52
acctgatcga ccaaatcccg ctcctctgat 30

<210> 53
<211> 50
<212> DNA
<213> Artificial Sequence

<220>
<223> PCR Primer

<400> 53
agtcatttaa atcgtcgacg tgactgttta gtgaaaataa ctcatgaaat 50

<210> 54
<211> 52
<212> DNA
<213> Artificial Sequence

<220>
<223> PCR Primer

<400> 54
cggaagaggg gagtgcagct gaggagcggc agagatctcg gggcggggtt cg 52

<210> 55
<211> 60
<212> DNA

<213>  Artificial Sequence

<220>
<223>  PCR Primer

<400>  55
gccgctcctc agctgcactc ccctcttccg cagggatgaa agtaggatgg gaaaatcccg        60


<210>  56
<211>  42
<212>  DNA
<213>  Artificial Sequence

<220>
<223>  PCR Primer

<400>  56
tgacgcggcc gcttcttaca tgggctgggc ctcagtggtt at                          42


<210>  57
<211>  50
<212>  DNA
<213>  Artificial Sequence

<220>
<223>  PCR Primer

<400>  57
agtcatttaa atcgtcgacg tattactgtt attcctgcat gcagtggaag                  50


<210>  58
<211>  30
<212>  DNA
<213>  Artificial Sequence

<220>
<223>  PCR Primer

<400>  58
ccctcgccca aaacccccac gagccgcggc                                        30


<210>  59
<211>  30
<212>  DNA
<213>  Artificial Sequence

<220>
<223>  PCR Primer

<400>  59
gctatactac cccattcggt gtctgagcgg                                        30


<210>  60
<211>  60
<212>  DNA
<213>  Artificial Sequence

<220>

<223>  PCR Primer

<400>  60
accgaccgtt accgaccgtt ttcatcccta gctccctcct cactctactc tgtcgtggct        60


<210>  61
<211>  49
<212>  DNA
<213>  Artificial Sequence

<220>
<223>  PCR Primer

<400>  61
tgacggcgcg cctttcatca tattataaat cgttttaatt tttttatag                    49


<210>  62
<211>  42
<212>  DNA
<213>  Artificial Sequence

<220>
<223>  PCR Primer

<400>  62
agtccctcga ggtgctccag atttatatgg attttatcta tg                           42


<210>  63
<211>  60
<212>  DNA
<213>  Artificial Sequence

<220>
<223>  PCR Primer

<400>  63
agccacgaca gagtagagtg aggagggagc tagggatgaa aacggtcggt aacggtcggt        60


<210>  64
<211>  42
<212>  DNA
<213>  Artificial Sequence

<220>
<223>  PCR Primer

<400>  64
tgacggcgcg cctgcagcta cgcactactg ttaatcaaag tt                           42


<210>  65
<211>  30
<212>  DNA
<213>  Artificial Sequence

<220>
<223>  PCR Primer

<400>  65

aggtgcttta catcttgcaa tgtaagtacc 30

<210> 66
<211> 30
<212> DNA
<213> Artificial Sequence

<220>
<223> PCR Primer

<400> 66
gtccagttgg ctggactggg gtgggttggg 30

<210> 67
<211> 30
<212> DNA
<213> Artificial Sequence

<220>
<223> PCR Primer

<400> 67
caccaaccac gagaaccaca tggacgagga 30

<210> 68
<211> 30
<212> DNA
<213> Artificial Sequence

<220>
<223> PCR Primer

<400> 68
ttctcccaca gtcccactaa tgtgaaatga 30

<210> 69
<211> 30
<212> DNA
<213> Artificial Sequence

<220>
<223> PCR Primer

<400> 69
catgctagtc cgcctccgga tcagagcacg 30

<210> 70
<211> 30
<212> DNA
<213> Artificial Sequence

<220>
<223> PCR Primer

<400> 70
gagccatggc caaggttctg caggagctcg 30

<210>  71
<211>  40
<212>  DNA
<213>  Artificial Sequence

<220>
<223>  PCR Primer

<400>  71
agtcaagctt tgtgtctggt ggacgtccgt ggtgcgcgcg                              40


<210>  72
<211>  45
<212>  DNA
<213>  Artificial Sequence

<220>
<223>  PCR Primer

<400>  72
atccgaattc gctgggcaac gatgatgttc gccatatcat cctcc                        45


<210>  73
<211>  30
<212>  DNA
<213>  Artificial Sequence

<220>
<223>  PCR Primer

<400>  73
ggacaaaagt ggggattaat gatcagcgag                                         30


<210>  74
<211>  30
<212>  DNA
<213>  Artificial Sequence

<220>
<223>  PCR Primer

<400>  74
gtagatggtg ggttgcatga gtcaccaatt                                         30


<210>  75
<211>  30
<212>  DNA
<213>  Artificial Sequence

<220>
<223>  PCR Primer

<400>  75
ttgtcaatgg ccatgactct acgccagaca                                         30


<210>  76
<211>  30
<212>  DNA

<213>  Artificial Sequence

<220>
<223>  PCR Primer

<400>  76
ctgtggggga catgttcgtc gacctccggt                                        30


<210>  77
<211>  30
<212>  DNA
<213>  Artificial Sequence

<220>
<223>  PCR Primer

<400>  77
cttgacactt ttgtattgag tttcgctgta                                        30


<210>  78
<211>  30
<212>  DNA
<213>  Artificial Sequence

<220>
<223>  PCR Primer

<400>  78
tgtgtctggt ggacgtccgt ggtgcgcgcg                                        30


<210>  79
<211>  30
<212>  DNA
<213>  Artificial Sequence

<220>
<223>  PCR Primer

<400>  79
ttgggtgggg atgcgaggga gctgatgatg                                        30


<210>  80
<211>  30
<212>  DNA
<213>  Artificial Sequence

<220>
<223>  PCR Primer

<400>  80
cttgttttaa tggatgattg ttgttaatcc                                        30


<210>  81
<211>  30
<212>  DNA
<213>  Artificial Sequence

<220>

<223>  PCR Primer

<400>  81
ccagttgtat atcccgccta attccttctc                                        30


<210>  82
<211>  30
<212>  DNA
<213>  Artificial Sequence

<220>
<223>  PCR Primer

<400>  82
tgaatgtaac acagggaagc gatacagctt                                        30


<210>  83
<211>  30
<212>  DNA
<213>  Artificial Sequence

<220>
<223>  PCR Primer

<400>  83
cccttcaacc gcttcctgct tctagttagc                                        30


<210>  84
<211>  30
<212>  DNA
<213>  Artificial Sequence

<220>
<223>  PCR Primer

<400>  84
gcaacttatt tgatactccc tctgtttcat                                        30


<210>  85
<211>  30
<212>  DNA
<213>  Artificial Sequence

<220>
<223>  PCR Primer

<400>  85
aaatttgatc aagtatataa aaaatatagc                                        30


<210>  86
<211>  30
<212>  DNA
<213>  Artificial Sequence

<220>
<223>  PCR Primer

<400>  86

ttagccttgt gatgcagcat ctcattagcc                                        30


<210>  87
<211>  30
<212>  DNA
<213>  Artificial Sequence

<220>
<223>  PCR Primer

<400>  87
ctggaatact gataagcact cctgaatttg                                        30


<210>  88
<211>  30
<212>  DNA
<213>  Artificial Sequence

<220>
<223>  PCR Primer

<400>  88
cactgatcct ccaggagaat tcacatacat                                        30


<210>  89
<211>  30
<212>  DNA
<213>  Artificial Sequence

<220>
<223>  PCR Primer

<400>  89
agatatagca gctgggcaac gatgatgttc                                        30


<210>  90
<211>  30
<212>  DNA
<213>  Artificial Sequence

<220>
<223>  PCR Primer

<400>  90
ccgcgttcct gccccgacgc atactccaca                                        30


<210>  91
<211>  30
<212>  DNA
<213>  Artificial Sequence

<220>
<223>  PCR Primer

<400>  91
gagaatagtg tgtgtcttct tcgagactcg                                        30

<210>  92
<211>  30
<212>  DNA
<213>  Artificial Sequence

<220>
<223>  PCR Primer

<400>  92
ccctaaattt taagcgtcac tttgaatcgc                                        30


<210>  93
<211>  30
<212>  DNA
<213>  Artificial Sequence

<220>
<223>  PCR Primer

<400>  93
ctacatccta gcactactaa acatggttag                                        30


<210>  94
<211>  30
<212>  DNA
<213>  Artificial Sequence

<220>
<223>  PCR Primer

<400>  94
ccataatata tttgtagccg aatccaccac                                        30


<210>  95
<211>  30
<212>  DNA
<213>  Artificial Sequence

<220>
<223>  PCR Primer

<400>  95
caataatttt cataatggta aggtacagtt                                        30


<210>  96
<211>  30
<212>  DNA
<213>  Artificial Sequence

<220>
<223>  PCR Primer

<400>  96
tgctgtcaac agcatgtgac ctcaataaga                                        30


<210>  97
<211>  30
<212>  DNA

<213>  Artificial Sequence

<220>
<223>  PCR Primer

<400>  97
gagcagccgc atcagtagtc atcagtgtgc                                        30


<210>  98
<211>  30
<212>  DNA
<213>  Artificial Sequence

<220>
<223>  PCR Primer

<400>  98
tcccttactt gttgtaagta gggctagacg                                        30


<210>  99
<211>  50
<212>  DNA
<213>  Artificial Sequence

<220>
<223>  PCR Primer

<400>  99
agtcatttaa atcgtcgacg ctccgccaag cccgccgccg acctctccgg                  50


<210>  100
<211>  60
<212>  DNA
<213>  Artificial Sequence

<220>
<223>  PCR Primer

<400>  100
agtctctttc aggggcatct cctgcgtctt tgcgcgcgtc aaggttcccg gcatacgtcc      60


<210>  101
<211>  76
<212>  DNA
<213>  Artificial Sequence

<220>
<223>  PCR Primer

<400>  101
gcaggagatg cccctgaaag agactgaaag gcagtgaaga aagttgcagg gatgaaagta      60

ggatgggaaa atcccg                                                       76


<210>  102
<211>  50
<212>  DNA
<213>  Artificial Sequence

<220>
<223>  PCR Primer

<400>  102
agtcatttaa atcgtcgacg atcagtgtgc ttgctctctc tcccatcgct                50


<210>  103
<211>  30
<212>  DNA
<213>  Artificial Sequence

<220>
<223>  PCR Primer

<400>  103
ctttctgctc ctccactcca gttacaggca                                      30


<210>  104
<211>  30
<212>  DNA
<213>  Artificial Sequence

<220>
<223>  PCR Primer

<400>  104
ggtgggacag ccgttgaggt agggccgaga                                      30


<210>  105
<211>  60
<212>  DNA
<213>  Artificial Sequence

<220>
<223>  PCR Primer

<400>  105
ccgaccgtta ccgaccgttt tcatccctaa tcagatcttt agacgtgtct cagcaaggtc      60


<210>  106
<211>  42
<212>  DNA
<213>  Artificial Sequence

<220>
<223>  PCR Primer

<400>  106
tgacggcgcg ccgggagatg aagcttcgga ggatccctat gc                        42


<210>  107
<211>  60
<212>  DNA
<213>  Artificial Sequence

<220>
<223>  PCR Primer

<400>  107
accttgctga gacacgtcta aagatctgat tagggatgaa aacggtcggt aacggtcggt      60


<210>  108
<211>  47
<212>  DNA
<213>  Artificial Sequence

<220>
<223>  PCR Primer

<400>  108
tgacggcgcg ccgccataat ttaatatgtg tgtgagactt ctctttc                    47


<210>  109
<211>  30
<212>  DNA
<213>  Artificial Sequence

<220>
<223>  PCR Primer

<400>  109
ccgccgcgac tagctagcta gctcggaggt                                       30


<210>  110
<211>  30
<212>  DNA
<213>  Artificial Sequence

<220>
<223>  PCR Primer

<400>  110
tgctagaaac atggatgtct agagtgcatg                                       30


<210>  111
<211>  40
<212>  DNA
<213>  Artificial Sequence

<220>
<223>  PCR Primer

<400>  111
agtcaagctt gacgttgggc agtccatact agagagttac                            40


<210>  112
<211>  45
<212>  DNA
<213>  Artificial Sequence

<220>
<223>  PCR Primer

<400>  112
atccgaattc tattggtgat ggaaattata gtcaaggagg atcac                      45

<210>  113
<211>  30
<212>  DNA
<213>  Artificial Sequence

<220>
<223>  PCR Primer

<400>  113
tttccagagg tggacatgat gaaggagcgc                                        30


<210>  114
<211>  30
<212>  DNA
<213>  Artificial Sequence

<220>
<223>  PCR Primer

<400>  114
aggcggccat ggcgattaac agcgatgtgc                                        30


<210>  115
<211>  30
<212>  DNA
<213>  Artificial Sequence

<220>
<223>  PCR Primer

<400>  115
cattcttacc aattatcagt ggaagctgac                                        30


<210>  116
<211>  30
<212>  DNA
<213>  Artificial Sequence

<220>
<223>  PCR Primer

<400>  116
gaggctgcca tccatgtgtg gaggttgaag                                        30


<210>  117
<211>  30
<212>  DNA
<213>  Artificial Sequence

<220>
<223>  PCR Primer

<400>  117
gacgttgggc agtccatact agagagttac                                        30


<210>  118

<211>  30
<212>  DNA
<213>  Artificial Sequence

<220>
<223>  PCR Primer

<400>  118
agccaagggt cgtctagccc tacttacaac                                        30


<210>  119
<211>  30
<212>  DNA
<213>  Artificial Sequence

<220>
<223>  PCR Primer

<400>  119
cctaatggac agatctgacg ctcttcagtg                                        30


<210>  120
<211>  30
<212>  DNA
<213>  Artificial Sequence

<220>
<223>  PCR Primer

<400>  120
cgaaggccgc cgggccgcag ctgaagctgc                                         30


<210>  121
<211>  30
<212>  DNA
<213>  Artificial Sequence

<220>
<223>  PCR Primer

<400>  121
gcatcgagcc catgtccacc atctccgccg                                        30


<210>  122
<211>  30
<212>  DNA
<213>  Artificial Sequence

<220>
<223>  PCR Primer

<400>  122
ctgctgaagg cgtctctgga gtcgacgacg                                        30


<210>  123
<211>  30
<212>  DNA
<213>  Artificial Sequence

<220>
<223>  PCR Primer

<400>  123
tgtaacccgg tcgaagcttc acccaatgca                                        30


<210>  124
<211>  30
<212>  DNA
<213>  Artificial Sequence

<220>
<223>  PCR Primer

<400>  124
ccctggatca agtagtctgg tacacacgaa                                        30


<210>  125
<211>  30
<212>  DNA
<213>  Artificial Sequence

<220>
<223>  PCR Primer

<400>  125
atatctctct gagttccaca catagtaacc                                        30


<210>  126
<211>  30
<212>  DNA
<213>  Artificial Sequence

<220>
<223>  PCR Primer

<400>  126
tgcacccaca cacagctaga gcactgacac                                        30


<210>  127
<211>  30
<212>  DNA
<213>  Artificial Sequence

<220>
<223>  PCR Primer

<400>  127
atcaacatat tgtcaccatc tcagttcatc                                        30


<210>  128
<211>  30
<212>  DNA
<213>  Artificial Sequence

<220>
<223>  PCR Primer

<400>  128
aaaatccact ccctccggat agagaagggt                                        30


<210>  129
<211>  30
<212>  DNA
<213>  Artificial Sequence

<220>
<223>  PCR Primer

<400>  129
aggaagaagg cgtcgtcgag ggcgcgcgcg                                        30


<210>  130
<211>  30
<212>  DNA
<213>  Artificial Sequence

<220>
<223>  PCR Primer

<400>  130
gctaagccta agctaatagc agaggcaacc                                        30


<210>  131
<211>  30
<212>  DNA
<213>  Artificial Sequence

<220>
<223>  PCR Primer

<400>  131
gattgaagat ggaaacagag tcagcagcgg                                        30


<210>  132
<211>  30
<212>  DNA
<213>  Artificial Sequence

<220>
<223>  PCR Primer

<400>  132
caacaaccca tcagctcttt gggacaaaac                                        30


<210>  133
<211>  30
<212>  DNA
<213>  Artificial Sequence

<220>
<223>  PCR Primer

<400>  133
ggttactatg tgtggaactc agagagatat                                        30

<210>  134
<211>  30
<212>  DNA
<213>  Artificial Sequence

<220>
<223>  PCR Primer

<400>  134
gtcagcttcc actgataatt ggtaagaatg                                        30


<210>  135
<211>  30
<212>  DNA
<213>  Artificial Sequence

<220>
<223>  PCR Primer

<400>  135
ctttggcagt gactccaagt acacttccgg                                        30


<210>  136
<211>  30
<212>  DNA
<213>  Artificial Sequence

<220>
<223>  PCR Primer

<400>  136
ccagcctcca cagctcgccg aacacagtgg                                        30


<210>  137
<211>  30
<212>  DNA
<213>  Artificial Sequence

<220>
<223>  PCR Primer

<400>  137
tgcctgtaac tggagtggag gagcagaaag                                        30


<210>  138
<211>  40
<212>  DNA
<213>  Artificial Sequence

<220>
<223>  PCR Primer

<400>  138
agtcaagctt ccggaaacct cctcggattc cattgcccag                             40


<210>  139

<211>  39
<212>  DNA
<213>  Artificial Sequence

<220>
<223>  PCR Primer

<400>  139
tgacgaattc gacgccattg aggtcgaaga cgccacggg                              39


<210>  140
<211>  30
<212>  DNA
<213>  Artificial Sequence

<220>
<223>  PCR Primer

<400>  140
atggtagatc tgagggtaaa tttctagttt                                        30


<210>  141
<211>  30
<212>  DNA
<213>  Artificial Sequence

<220>
<223>  PCR Primer

<400>  141
tcacacgtga tggtgatggt gatggctagc                                        30


<210>  142
<211>  30
<212>  DNA
<213>  Artificial Sequence

<220>
<223>  PCR Primer

<400>  142
gatatgtcct gcgggtaaat agctgcgccg                                        30


<210>  143
<211>  30
<212>  DNA
<213>  Artificial Sequence

<220>
<223>  PCR Primer

<400>  143
ggtacccctg gattttggtt ttaggaatta                                        30


<210>  144
<211>  30
<212>  DNA
<213>  Artificial Sequence

<220>
<223>  PCR Primer

<400>  144
cgtgctctga tccggaggcg gactagcatg                                        30


<210>  145
<211>  26
<212>  DNA
<213>  Oryza sativa

<400>  145
tgccgctcct caggttcact cccctc                                            26


<210>  146
<211>  26
<212>  DNA
<213>  Oryza sativa

<400>  146
tgccgctcct cagctgcact cccctc                                            26


<210>  147
<211>  50
<212>  DNA
<213>  Oryza sativa

<400>  147
ctctgccgct cctcaggttc actcccctct tccccctccc tcctcactct                  50


<210>  148
<211>  50
<212>  DNA
<213>  Oryza sativa

<400>  148
ctctgccgct cctcagctgc actcccctct tcccgcctcc ctcctcactc                  50


<210>  149
<211>  48
<212>  DNA
<213>  Oryza sativa

<400>  149
ctctgccgct cctcagctgc actcccctct tccctccctc ctcactct                    48


<210>  150
<211>  44
<212>  DNA
<213>  Oryza sativa

<400>  150
ctctgccgct cctcagctgc actcccctct tccctcctca ctct                        44


<210>  151

<211>  27
<212>  DNA
<213>  Oryza sativa


<220>
<221>  CDS
<222>  (1)..(27)

<400>  151
ggg aac ctt agc gca agg aaa gac gca                                    27
Gly Asn Leu Ser Ala Arg Lys Asp Ala
1               5


<210>  152
<211>  9
<212>  PRT
<213>  Oryza sativa

<400>  152

Gly Asn Leu Ser Ala Arg Lys Asp Ala
1               5


<210>  153
<211>  27
<212>  DNA
<213>  Oryza sativa


<220>
<221>  CDS
<222>  (1)..(27)

<400>  153
ggg aac ctt gac gcg cgc aaa gac gca                                    27
Gly Asn Leu Asp Ala Arg Lys Asp Ala
1               5


<210>  154
<211>  9
<212>  PRT
<213>  Oryza sativa

<400>  154

Gly Asn Leu Asp Ala Arg Lys Asp Ala
1               5


<210>  155
<211>  3000
<212>  DNA
<213>  Artificial Sequence

<220>
<223>  OsRacGEF1 5′ side homologous region for HR

<400>  155
ctccgccaag cccgccgccg acctctccgg tttgttcttc ctaccaaaca aactctcctc      60

tctctgctgt ttccttgttg cgtagatctc actccacccc tcacgagatg cttcggtttg 120

gggcttttgc tttccagagg tggacatgat gaaggagcgc ttcgccaagc tgctgctcgg 180

cgaggacatg tccgggagcg gcaagggcgt ctgcaccgcg ctcgccatct ccaacgccat 240

caccaacctc tccggtacgg tcatgaaccc ccacattgcc gtgatttgcg acgaggagag 300

tggtgaataa tgttgctgct gctgttgttg ttgtttccga atccgcagcc actgtgttcg 360

gcgagctgtg gaggctggag ccgatggctt cggcgaggaa ggcgatgtgg acgagggaga 420

tggactggct gctctccgtg gccgactcca tcgtcgagct caccccgtca atccaggagc 480

tccccgacgg cgggggacag ttcgaggtca tggtgccgcg cccccgcagc gacctctaca 540

tgaacctccc ggcgctcaag aagctcgatg cgatgctcct cgccatgatt gacgggttca 600

aggagaccga gttctggtat gtggataggg ggatcgtggt tgatgacagc ggcgggccgt 660

tctcgtcgtc gtcatcctcc tgcgggcggc cgtcggtgcg gcaggaggag aaatggtggc 720

tgccatgccc gcgggtgccg cccaaggggt tgtcagagga tgcgaggagg aagctgcaac 780

aggacaggga ttgtgcgaac cagatactga aggcggccat ggcgattaac agcgatgtgc 840

tcgcggagat ggagatcccg gaagtgtact tggagtcact gccaaaggta aacaatcatt 900

tcagattatc tactcattgg tactactagt tactaataac tggtgctcta tactagaaaa 960

aacaccactg gtgctggtaa aaatgggcca atgtgttaaa ttgcagtgaa caggacttct 1020

tctcaccata catgcttgta attgtagctc aactggtgaa agtgggagtg cctttatggc 1080

aatgtggtcc gttcgcttga gctagctgtg ttgctatcac actatcatct gtcttaaatc 1140

caatttattt ccattcttgt ttgtacagcc tgccattttt ttcacaagga atcaattcat 1200

gtgtagtaga gttaaagtca ctgtttttcc ttgttacaga aataagctaa ttaatgtctt 1260

tccagtgcaa ttctcatgta ttttgtgagg gggtattagc atattgcaca ctgtcttggt 1320

ttacttcttg ctgccttata tggcaagatg attttagcat ctgatctgga atcttgcaac 1380

cagtattcca ttttaatgaa aagttgaaaa cattcttacc aattatcagt ggaagctgac 1440

agttgatggc atttagaatg caaagaactt catcatataa attagcttat gatggagagt 1500

attgtaaaca ttaaacagat acctgggcga agctcaaatt cggcttcttt cttgtctgtg 1560

cttgtacacc atcacgcgca aaaataagct agtgatgcat agtattctag ttaaggcatt 1620

aatttcccaa atgtaattca tgtataggtt gctcaacctg gtcatgaata tggcatctag 1680

gcgttgactt ttaggcttgt tgtgtaatgt gctttagaag acgcaatatt gaaattccat 1740

ggcatctttt taacttttgt tctgtaattg aatataaatt atttgaactg tatctttaaa 1800

tgaatattca agtcgcttct gatatccttt actgtgtgtt tatgtgctta atgagtacct 1860

tcctaaaaga gttgttctta actggtaatg atatctctct gagttccaca catagtaacc 1920

tcttcttttt tttttctgca gagtggtaaa tcttgcttgg gtgagataat ttaccgatat 1980

ataacagctg aacaattctc accggagtgc ctcctcgatt gcttggatct gtcatcagaa 2040

caccacacac ttgaagtagc taacagaata gaggctgcca tccatgtgtg gaggttgaag 2100

ggccaaaaga aatccacgcc tcaagcaaaa tcaaagaaat cttggggtgg aaaagtgaag 2160

ggacttgttg gagatacaga aaagagtcat gttttgtccc aaagagctga tgggttgttg 2220

caaagcttaa gattgcgata ccctggtttg ccacaaacct ctctcgacat gaacaagatc 2280

cagtataaca aggttattcc tcacatttta tcttagttga atatgtcaaa caagttgggc 2340

acttgggctt agagttagca ttgtatggtt cattcacctt ctatttcatc aaaaccgagc 2400

atgctagcat gccatactaa gatctgaatt atatgaatag ttacattaga acctttctgt 2460

ttttaatatc acttaactta ttctcttcaa aaaaaatcag tttactgtca acataatcac 2520

aatattcatc tttttttctt gtgtttcttg tccaggacgt tgggcagtcc atactagaga 2580

gttactcaag ggttctggag agcttggcat ttaacataat tgccagaatt gatgatgtaa 2640

tttatgtaga tgatgcaaca aagaagtctg ccgctgctga ctctgtttcc atcttcaatc 2700

gtggcattgg cgtaccagtt cagaagagga tctccccaag cccgttctca attcagcata 2760

caccatatgc ctctccattt gctacaccca ctttctgctc ctccactcca gttacaggca 2820

gccctggaag agtacagcct ccattgaaca aagacaactt accgacaaaa caagaggtta 2880

aagttgagaa gctgttttct ggtgatatcg agaaggtctg gacgtatgcc gggaaccttg 2940

acgcgcgcaa agacgcagga gatgcccctg aaagagactg aaaggcagtg aagaaagttg 3000

<210> 156
<211> 3900
<212> DNA
<213> Artificial Sequence

<220>
<223> OsRacGEF1 3′ side homologous region for HR

<400> 156
atcagatctt tagacgtgtc tcagcaaggt caacaggttt caacacatcc tgttctggtc 60

tctagctaga ttttccctgt acagaagcaa catgattcat ttttttgtgc tttcttagcg 120

agccaagggt cgtctagccc tacttacaac aagtaaggga atgactcttt gagtcttgat 180

cgcatctgaa caagtcatag tttcacgtag ctgcctctgt cggacatgta ctcttcttgt 240

tcatgtttat actagctggt ttagttagca gcaagtgatt gaacttcaga tgctgcagtt 300

cttttttttt ttttttgtcc ctggaggcat cggttcccta acaatcaact gcacttgcag 360

tgcaatgtat catgattttc tgatgctgtt gtatatggca cctaatggac agatctgacg 420

ctcttcagtg actctgaaga ctttcttata tcaactgtga atctgtgatc gatcacaact 480

gctgaaggct tcacagtttt gttggtgatc ctccttgact ataatttcca tcaccaatat 540

tttcttctct gcagctgcaa cttgcaagca ggttgcctct gctattagct taggcttagc 600
tccatggaag aagcaaagaa taccttgttc cagaatcaca cggcgacatt gccagattgc 660
caactaaacc ctcaggtgaa agtaacttca tgtgcaaata taccatgcct aatttgatca 720
tcaagtccat ggcaacatct tgcttgatta cttctccaat cttgcaatca aatggctact 780
acaatataag ctgtgcattg caatgctgtt cgagacgtgt gctttaactg ttcgacagtg 840
acctgagaac tgagaagaga agactagagt tgtagccaca cgaccagacc ggcatggccg 900
ccgccgccgc ggcgaacgac acgacgccgg cgaaggccgc cgggccgcag ctgaagctgc 960
tggtggacaa gcggtcgcgg cgcgtgctgt acgcggaggc gcgcaaggac gcggtggact 1020
tcctcatcgg cctcctccgc gtgcccgcgg ggctcgccgc gcgcgtcctc gccagccacg 1080
gcgtcgccgc gccgggctcc ctcgccacgc tctacgccgc cgcgcgcgcc ctcgacgacg 1140
ccttcttcct cgcgtcgccg ccctccccgg gcccagaccg ccgcgacgcg gtgctcgccc 1200
ccgccgttct cccctccgcc gcgcttcccc tgctcggcga gcgccccccg ccgccgccgc 1260
cgccgccgcc gaagcggtac taccggtgca acgcgtacgc gatgccgtgc cggtcgaacc 1320
cgctgaacgt gacggacacg gcgggcctcg cgtgcccggg gtgccggcag ccgatgacgg 1380
tggagatgaa gtgggcggcc ggcggcggca gcaagccggc ggaggaggag gaggcggcgg 1440
ccggcggcga gggcgggtac gtgaaggagg tggtgacgta cctggtgatg gacgacctga 1500
gcatcgagcc catgtccacc atctccgccg tgatgctgct caagaagttc gacgtcaagg 1560
attgctccgc cctcgacgag atgaccgtcg acctgggccc caaagaggta aacttttgca 1620
acccttctct atccggaggg agtggatttt gatcgttatt cgcatttcac tcaaataatt 1680
atgaaataaa attaataaga tatattaata tgtgatacgt cactccacaa atatataagt 1740
tcaaattcaa cttctacata tcgtataaaa ataaaacaaa tttgactgtg aatatacatt 1800
aactagctgt agtttaactc aataagatta attatggtat gctcatttgg ctatggttcg 1860
tacatattag acaagttagt gcatcatgtt taatatgttt ttatagtagg ttgtaccccc 1920
atgttaagtt cttctatcac tgatataatt tattcattta tttattactc attgtgtacc 1980
atgatcatgt gtgttgtgca gtgtgtgaag ctgctgaagg cgtctctgga gtcgacgacg 2040
gcattgacgg atgtgttcag cggcggcgtc tccattgata ggcttgagtg agcaggcgag 2100
gtcgcggatg cacacgtgtg cgagctggct gatgaactga gatggtgaca atatgttgat 2160
atgcaatgga gaatccaatt catgttttca ccatgaaacc atggtacatg tatgcaaatg 2220
ttattctttg cacaaaaagt aacttaatga tcacaaagaa aacaggggtt aaagttcaaa 2280
agatcttctc tctctcaaca aaaaatattg caaaagatct ctctcgctac caatgattga 2340
agggtcgtgt ggacccccgt ttttataaca agaatcaatc gtgtaatcag taccattttc 2400

ctggatcaag tggtctggta cacacgaaca acaaaagagc aacaaaaggc gatcgatcca 2460

atgctgccat tagccaatac tctacacata tgtaacccgg tcgaagcttc acccaatgca 2520

atattattaa tcatatagtt gtgttattat attttgagat acgaataaat atactgtatt 2580

gtgtcagtgc tctagctgtg tgtgggtgca tgcatgatct gatgatatgc ttatttcccg 2640

tggaaaagtc tgcttatatt tgtatataat taaggaacca tgcaaatgac gtatataatt 2700

gcatcaaggt ccacaataga cagtatatac caatggtcac tagatcgatt cctgattctt 2760

gagtagtcaa caagaaaaat acaaagttgt gttcttacgg ggaaagaata tggcaggtca 2820

gtagtcgcct aatctcccgg tctctagcta gcacacaaca cgttctctac tactcaatca 2880

acacaggttg atgatgggtt agtgtggacc ccgtttttat aacaggaatc aatcgtgtaa 2940

acagtaccat ttccctggat caagtagtct ggtacacacg aactcatagc tgaaatatca 3000

agtgcacata cacgagagtc tagtacgaat tattacatca taagcccgca ggcacagtct 3060

taaatcatcg gaccgagcgg tccggaatac attccaaaaa cagaagtagg taaggcagcg 3120

gagttaaggc agcggtatgc cattgccaca ggcaacgacc gtactaagac ctactgagcg 3180

ccatcgtctt catctccctc ctggtagtag gcatagggat cctccgaagc ttcatctccc 3240

gcttctgatt atatttgcaa gggtgagtac caaccgtact cagcaagcca ccacagcaat 3300

gatgcacatg acagaggaat tcaaaggatg gctatggttc ttttgcgcaa agcaagtttt 3360

gtaattcttt tcacaagcct aagacctagc atagactgat caagttttaa taccagtgtt 3420

catattaaac aatgatggtt ctgtccacca tccattgtga tcccaaggat agcttcccgc 3480

cattgagtcg ttatggtttt ctgaggacgt ccatattccc gcctttcagg aagtggctcc 3540

atcagcataa aaatcatcat gcaatatccc atcccacaca agttaaagaa tttagagtct 3600

agccaagtgt aatacatgtc ccggtgctca ataaccgcga gcacggctat tcgaatagat 3660

ttggtttact cacactgcag tggatgtaca ctttacccgc actccgcgac tgcccaacac 3720

atgagcctcg tcccaacaca tgagacgcgc cacggtaaag ccttttgata acctcgcatt 3780

ggcagtaccc gctccatgaa cttaaatcct catgcactct agacatccat gtttctagca 3840

gtgagaggag ttctggcgct cccgggaaag agaagtctca cacacatatt aaattatggc 3900

<210> 157
<211> 30
<212> DNA
<213> Artificial Sequence

<220>
<223> PCR Primer

<400> 157
accaccctct cccggataag aggcgcaacc 30

<210>  158
<211>  30
<212>  DNA
<213>  Artificial Sequence

<220>
<223>  PCR Primer

<400>  158
tgtggaatcg caaaactatc ttgccaagct                                        30


<210>  159
<211>  83
<212>  DNA
<213>  Oryza sativa

<400>  159
gccgggaacc ttgacgcgcg caaagacgca ggagatgccc ctgaaagaga ctgaaaggca        60

gtgaagaaag ttcttcagat ctt                                               83


<210>  160
<211>  83
<212>  DNA
<213>  Oryza sativa

<400>  160
aagatctgaa gaactttctt cactgccttt cagtctcttt caggggcatc tcctgcgtct        60

ttgcgcgcgt caaggttccc ggc                                               83


<210>  161
<211>  80
<212>  DNA
<213>  Oryza sativa

<400>  161
gccgggaacc ttgacgcgcg caaagacgca ggagatgccc ctgaaagaga ctgaaaggca        60

gtgaagaaag ttcagatctt                                                   80


<210>  162
<211>  454
<212>  DNA
<213>  Oryza sativa

<400>  162
atggcgacca ccaccaccac cccctcctcc tctctcaccg cccctctcct ccgcccgagc        60

tcgaacgcga accccgcccc gagatctctg ccgctcctca ggagccggag gtgcgctcgg       120

gccgtggcga ccgccgccgc cgccgctggc cacggggccg ctcatcagag gagcgggatt       180

tggtcgatca gggatgattt ggtggtgccg aggtcgccct acttccctgt ggagtatgcg       240

tcggggcagg aacgcgggcc atcgcccatg gtgatggagc ggttccagag cgtcgtcagc       300

cagctcttcc agcacaggat tatccggtgt ggtggacccg tggaggatga tatggcgaac       360

atcatcgttg cccagctgct atatctcgat gccatcgatc ctaacaagga tatcattatg 420

tatgtgaatt ctcctggagg atcagtgaca gctg 454

<210> 163
<211> 537
<212> DNA
<213> Oryza sativa

<400> 163
atggcgacca ccaccaccac cccctcctcc tctctcaccg cccctctcct ccgcccgagc 60

tcgaacgcga accccgcccc gagatctctg ccgctcctca gctgcactcc cctcttcccc 120

ctccctcctc actctactct gtcgtggctc cactcgctct accctaactg actcggtctt 180

gcaggagccg gaggtgcgct cgggccgtgg cgaccgccgc cgccgccgct ggccacgggg 240

ccgctcatca gaggagcggg atttggtcga tcagggatga tttggtggtg ccgaggtcgc 300

cctacttccc tgtggagtat gcgtcggggc aggaacgcgg gccatcgccc atggtgatgg 360

agcggttcca gagcgtcgtc agccagctct tccagcacag gattatccgg tgtggtggac 420

ccgtggagga tgatatggcg aacatcatcg ttgcccagct gctatatctc gatgccatcg 480

atcctaacaa ggatatcatt atgtatgtga attctcctgg aggatcagtg acagctg 537

<210> 164
<211> 34
<212> DNA
<213> Oryza sativa

<400> 164
cctcaggttc actcccctct tccccctccc tcct 34

<210> 165
<211> 32
<212> DNA
<213> Oryza sativa

<400> 165
cctaactgac tcggtcttgc aggagccgga gg 32

<210> 166
<211> 10
<212> DNA
<213> Oryza sativa

<400> 166
gagccggagg 10

<210> 167
<211> 31
<212> DNA
<213> Oryza sativa

<400> 167

cctcagctgc actcccctct tcctccctcc t                                    31

<210>  168
<211>  32
<212>  DNA
<213>  Oryza sativa

<400>  168
cctaactgac tcggtcttgc aggagccgga gg                                   32

## Description

### [Technical Field]

The present invention relates to a nucleic acid for editing the genome of a plant cell comprising a transposon and a method for producing a plant cell having a mutation in a target region of a double-stranded DNA by using the nucleic acid.

### [Background Art]

There is a need for quick and highly versatile new plant breeding techniques in order to promote increased food production and improvement of the environment in a sudden change in the agriculture situation such as rapid growth of population or climate changes in recent years. A typical example of the techniques is molecular breeding utilizing random gene recombination. However, with the conventional plant gene recombination techniques, marker genes such as antibiotic-resistant genes need to be incorporated into a plant genome together with a gene of interest, wherein there is a problem of inability to control the region in the plant genome in which those foreign genes are incorporated and the number of copies of the foreign genes that are incorporated. In order to solve this problem, genome editing techniques utilizing artificial nucleases such as TALEN or CRISPR/Cas are attracting attention (e.g., Non Patent Literature 1). With these genome editing techniques, a double strand break (DSB) is mainly caused in a target DNA in a plant genome to induce a non homologous end joining (NHEJ) repairing mechanism in the plant cell. During the process, a mutation randomly occurs, which results in loss of the function of the target gene. In order to modify a target gene as designed, a template DNA is supplied to a region where a DSB has occurred due to an artificial nuclease to be incorporated into the plant genome side using homologous recombination (HR). However, there is still a high technical barrier because higher plants have an extremely low frequency of HR relative to NHEJ (Non Patent Literature 2), and both expression of an artificial nuclease and supply of a template DNA need to be achieved in the same cell. Further, when artificial nucleases are used, the off-target effect that causes a DSB in a place other than a target gene and the possibility of a large-scale structural mutation resulting therefrom cannot be avoided. Moreover, it is very difficult to introduce an artificial nuclease or the like to higher plant cells with an RNA and a protein. Thus, in order to utilize the genome editing techniques, it is necessary to incorporate a necessary gene group into a plant genome as a DNA in advance and express the gene group.

In order to develop a modified plant body obtained by the conventional gene recombination or genome editing techniques as a new variety, an artificial DNA sequence contained in the genome needs to be removed. One of the major measures is a technique utilizing gene segregation. However, this technique requires artificial crossing or the like and requires plant raising for a few years. Further, this technique cannot be applied to vegetative-propagation plants. Meanwhile, bacteriophage P1-derived Cre-loxP- or yeast-derived FLP-FRP-systems and the like are known as a method for deleting an artificial DNA sequence by utilizing a site-specific recombination technique (e.g., Non Patent Literature 3). However, these systems have a problem that a foreign DNA sequence having several tens of bp remains in the plant genome as a trace.

### [Citation List]

### [Non Patent Literature]

[NPL 1] Jung C. et al., Plant Breeding, 137:1-9 (2018)
[NPL 2] Paszkowski J. et al., EMBO J., 7(13):4021-4026 (1988)
[NPL 3] Dang T.T. et al., Plant Cell Physiol, 54(12):2058-2070 (2013)

### [Summary of Invention]

### [Technical Problem]

Thus, the problem to be solved by the present invention is to provide a minute and quick plant breeding technique that is able to introduce a mutation into a plant genome without leaving a trace of an exogenous artificial DNA sequence.

### [Solution to Problem]

The present inventors conceived of an idea that it may be possible to improve the frequency of successful target gene modification and further reduce necessary man-hours at the same time by improving a gene targeting method based on a positive-negative selection approach using Cre-loxP, and incorporating an autonomous marker deleting mechanism using a modified transposon instead of Cre-loxP. The present inventors advanced a research based on the idea to find that it is possible to autonomously perform two-stage target gene modification with one gene recombinant manipulation by using an Ac/Ds system, wherein an exogenous gene can be removed from the modified genome without leaving a trace of the exogenous gene, and that this method can create a new plant line having a useful phenotype in only about 10 months. The present inventors conducted further researches based on these findings to complete the present invention.

Specifically, the present invention is as follows.
[1] A nucleic acid comprising:
   (1) a transposon; and
   (2) a sequence homologous to at least a part of a target region of a double-stranded DNA of a plant cell,
   wherein the transposon comprises a marker gene and a gene encoding a transposase operably linked to an inducible promoter.
[2] The nucleic acid of [1], wherein the transposon is derived from a transposon selected from the group consisting of a Ds element, an Ac element, an Spm-s element, an En1 element, an Spm-I8 (dSpm) element, an Mu element, a Tam1 element, a Tam2 element, a Tam3 element, an nDart element, a Dart element, and a PiggyBac element.
[3] The nucleic acid of [1] or [2], wherein the transposon is derived from a Ds element or an Ac element, and the transposase is AcTPase or a variant thereof.
[4] The nucleic acid of any of [1] to [3], wherein the marker gene includes a gene encoding a fluorescent protein.
[5] The nucleic acid of any of [1] to [4], wherein the inducible promoter is a heat-shock inducible promoter.
[6] A method for producing plant cells that have a mutation in a target region of a double-stranded DNA and do not have any of an exogenous transposon and a gene encoding a transposase, the method comprising:
   (1) the step of selecting plant cells introduced with the nucleic acid of any of [1] to [5];
   (2) the step of culturing the plant cells selected in step (1) under a condition wherein the inducible promoter is activated; and
   (3) the step of selecting a cell in which expression of the marker protein is eliminated from a population comprising the plant cells cultured in step (2).
[7] The method of [6], wherein the plant cells are cells of a monocotyledon.
[8] The method of [7], wherein the monocotyledon is a gramineous plant.
[9] A plant cell obtained by the method of any of [6] to [8] or a plant body comprising the cell.

### [Advantageous Effects of Invention]

With the plant genome editing technique of the present invention, two-stage target gene modification can be autonomously materialized with one gene recombinant manipulation. Specifically, a wide range of a DNA sequence is modified as designed by gene targeting via homologous recombination in primary modification, and an artificial DNA sequence such as a transposon or a gene encoding a transposase is autonomously removed by a transposon system in secondary modification. A plant made in this manner in which the artificial DNA sequence is removed also exerts an advantageous effect that it can be excluded from the subject of the Cartagena Protocol. Since an exogenous transposon, a transposase and the like can be removed through the above-described secondary modification, it is not necessary to make a multiple transformant or perform backcross with a wild type to remove the above-described exogenous factors as in the conventional techniques. Thus, the present genome editing technique can minutely and quickly modify only a target gene, so that said technique is extremely useful as a practical plant breeding technique.

### [Brief Description of Drawings]

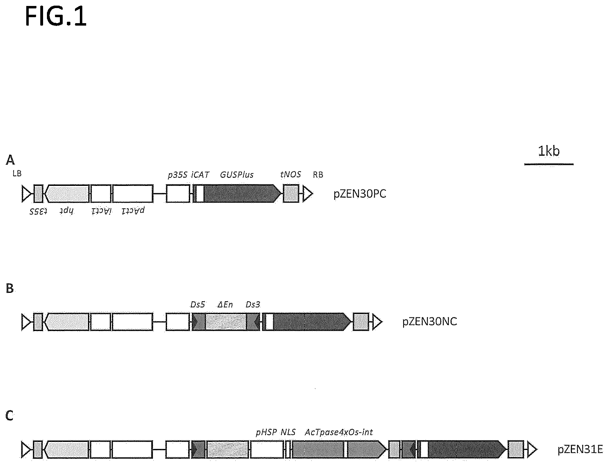

[Figure 1] Figure **1** shows the T-DNA structure of the binary vectors used for evaluation of a heat-shock inducible Ac/Ds-mediated marker excision system. (**A**) A schematic diagram of a pZEN30PC construct, wherein p35S shows a cauliflower mosaic virus (CaMV) 35S promoter (the sequence set forth in SEQ ID No: 37 or 38), GUSPlus shows a β-glucuronidase gene, iCAT shows intron 1 of a castor bean Catalase, tNOS shows a nopaline synthase terminator, pAct1 and iAct1 show a rice Actin1 promoter (the sequence set forth in SEQ ID NO: 43) having an intron of rice Actin1, hpt shows a hygromycin phosphotransferase gene, and t35S shows a CaMV 35S terminator (the sequence set forth in SEQ ID NO: 40 or 41). (**B**) A schematic diagram of a pZEN30NC construct, wherein ΔEn shows a functional transcription stop sequence derived from a maize En/Spm transposon, and Ds_5 and Ds_3 show a 300 bp terminal region of a maize Ac element. (**C**) A schematic diagram of a pZEN31E construct, wherein pHSP shows a promoter region of Oshsp16.9C, which is a heat-shock protein of rice, NLS shows a nuclear localization signal of an SV40 large T antigen, AcTPase4xOs-int shows a codon-optimized ORF of enhanced AcTPase having an intron, and RB and LB show a border sequence on the right side and a border sequence on the left side, respectively.

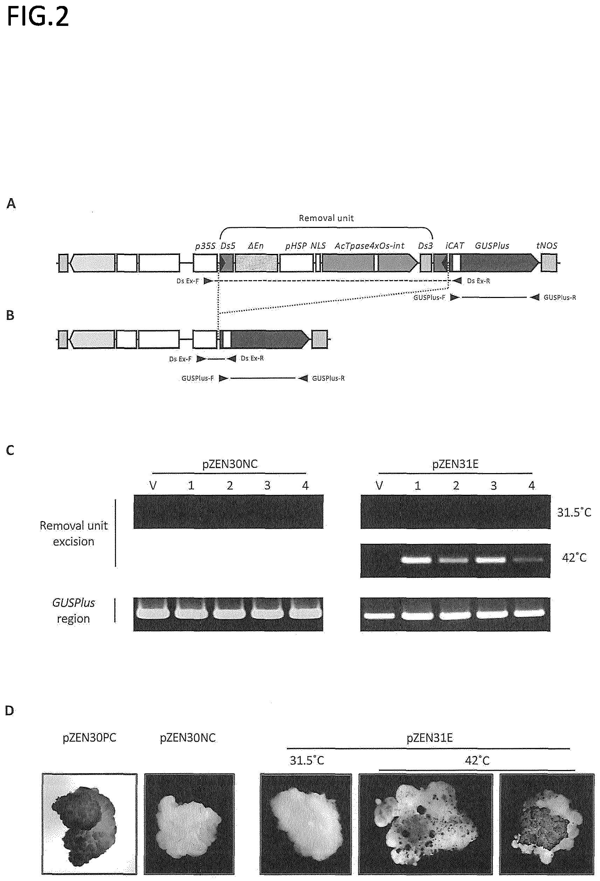

[Figure 2] Figure **2** shows a method for evaluating the heat-shock inducible Ac/Ds-mediated marker excision system in a callus of rice and the results thereof. (**A**) A schema of a GUS reporter assay using pZEN31E. ΔEn was inserted as a transcription terminator to functionally disrupt GUSPlus. Ds_5 and Ds_3 show a 300 bp terminal region of a maize Ac element. (**B**) The frequency and behaviors of heat-shock inducible Ac/Ds-mediated excision of a removal unit can be visualized by the GUS-reporter assay. Excision can be confirmed by PCR analysis using primers (Table 5) shown as horizontal arrows. (**C**) The PCR analysis results of a callus of transformed rice. Fragments amplified by PCR represent the removal unit being excised from the T-DNA region of pZEN30NC and 31E in a heat-shock inducible manner. (**D**) The results of the GUS-reporter assay of a genetically recombinant rice callus using histochemical staining with X-Gluc.

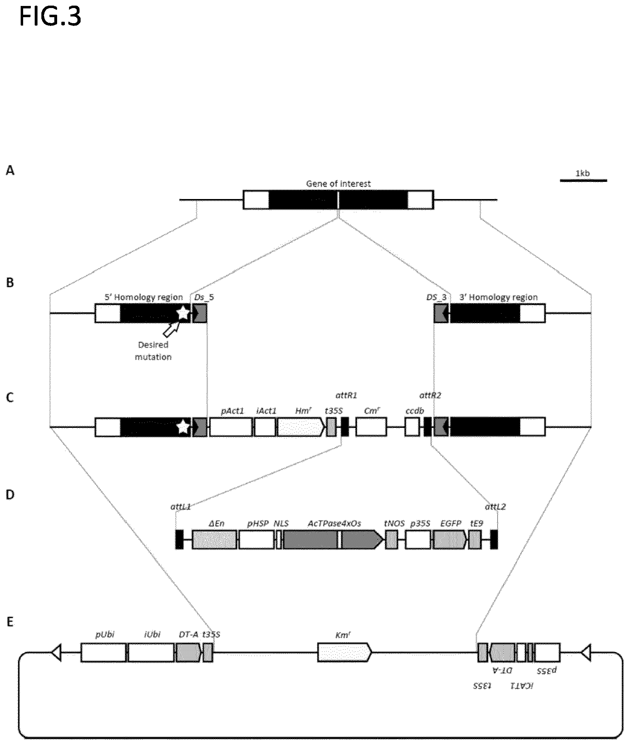

[Figure 3] Figure **3** shows the design and construct of a gene targeting vector having an autonomous marker excision system in a rice callus. (**A**) A model of a gene to be modified. (**B**) Cloning of a homology region. Ds_5 (the sequence set forth in SEQ ID NO: 34) and Ds_3 (the sequence set forth in SEQ ID NO: 35) are each a terminal region of an Ac element which is required for marker excision by an AcTpase4xOs transposase. (**C**) A homology region having a removal unit which comprises a positive marker gene (Hm^{r}). (**D**) Integration of heat-shock inducible AcTPase4xOs and constitutively active EGFP by a gateway LR reaction by a gateway LR reaction. (**E**) A figure of a complete gene targeting vector having an autonomous marker excision system. The constructed components are transferred between two diphtheria toxin A subunit genes (DT-A) (the sequence set forth in SEQ ID NO: 39) under the control of a constitutive promoter.

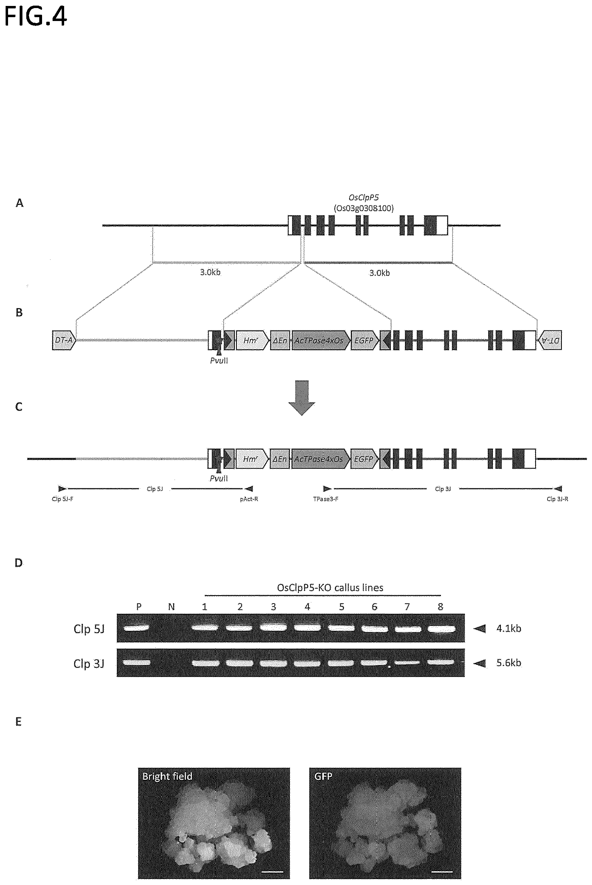

[Figure 4] Figure **4** shows primary modification of OsClpP5 by gene targeting mediated by homologous recombination. (**A**) An OsClpP5 gene in a rice genome. White boxes and black boxes show an untranslated region (UTR) and an exon, respectively. (**B**) The structure of pOsClpP5KO-AcDs, which is a gene targeting vector. The left and right bars represent a DNA sequence corresponding to a homology region carried by the vector. The star in the 5'-homology region shows the position of a new PvuII site and a desired mutation that results in disruption of a splice donor site of intron 1 of OsClpP5. (**C**) The structure of OsClpP5 modified by gene targeting. The horizontal lines marked with Clp 5J and Clp 3J show 5' and 3' junction fragments made by homologous recombination. The adjacent black arrows show primers that were used for PCR screening of callus lines of a target (Table 1). (**D**) The PCR screening results of gene-targeted (GT) callus lines. P shows a control plasmid comprising Clp 5J or Clp3J (Figure **4C**), N shows a callus of non-transformed rice variety "Nipponbare", and lanes 1 to 8 show a GT callus transformed with pOsClpP5KO-AcDs. (**E**) EGFP expression in the GT callus. Scale bar: 2.5 mm.

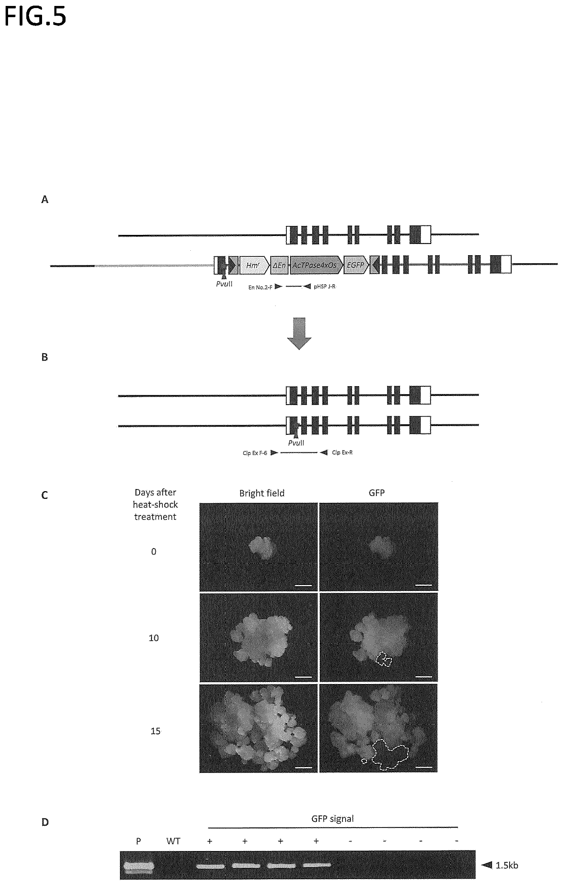

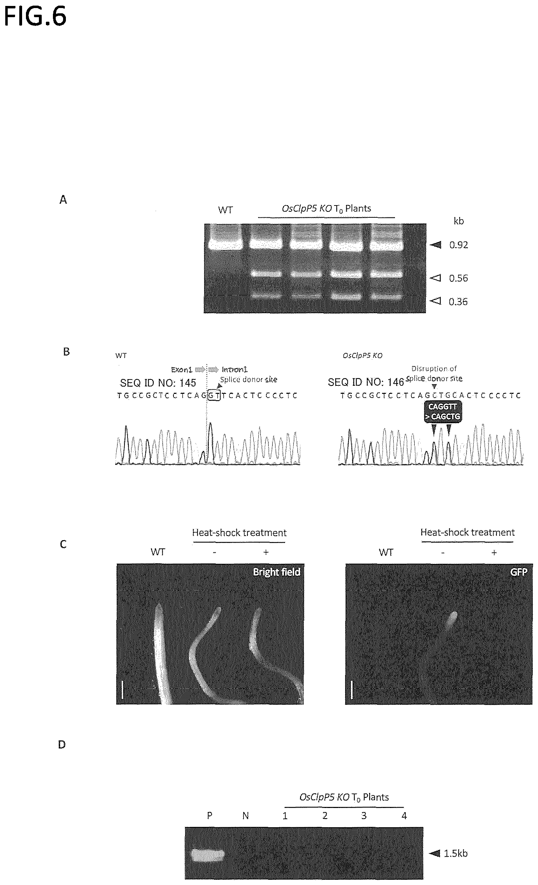

[Figure 5] Figure **5** shows excision of a positive marker from OsClpP5 modified by a heat-shock inducible Ac/Ds system. (**A**) The state of heterozygosis of an OsClpP5 gene of a target in the GT callus. The black arrows show primers that were used for PCR analysis to detect the presence of a removal unit (Table 1). (**B**) The modified OsClpP5 gene after excision of the removal unit mediated by Ac/Ds. The black arrows show primers that were used for CAPS and DNA sequencing analysis (Table 1 and Figure **6A**). (**C**) Proliferation of GT callus lines over time after heat-shock treatment. The proliferative cell lines without an EGFP signal were surrounded with a dotted line. Scale bar: 2.5 mm. (**D**) The presence of a removal unit in the GT callus lines. The arrow shows a DNA fragment amplified by PCR using a primer pair (En No.2-F/pHSP J-R) (Table 1 and Figure **5A**). Proliferative calli were used for analysis regardless of the presence or absence of an EGFP signal.
[Figure 6] Figure **6** shows transfer of the modified OsClpP5 gene to T₀ plants. (**A**) CAPS analysis of a target region in T₀ plants. A primer pair (Clp Ex F-6/Clp Ex-R) (Table 1 and Figure **5B**) amplifies a characteristic PCR fragment comprising a region (0.92 kb) of OsClpP5 intron 1. A PCR amplification product derived from the modified OsClpP5 KO gene was digested into two fragments (0.56 kb and 0.36 kb) by PvuII. WT shows a wild-type "Nipponbare" plant. (**B**) The spectra of Sanger sequencing of the OsClpP5 locus. The arrows show an overlapping heterogenic mutation. (**C**) EGFP expression in root tissue of T₀ plants. Regenerates derived from the wild type and the GT callus lines that were treated or not treated with heat shock are shown. Scale bar: 1.25 mm. (**D**) The presence of a removal unit in T₀ plants. P shows pOsClpP5KO-Con5, which is a control plasmid, while N shows a callus of non-transformed "Nipponbare".

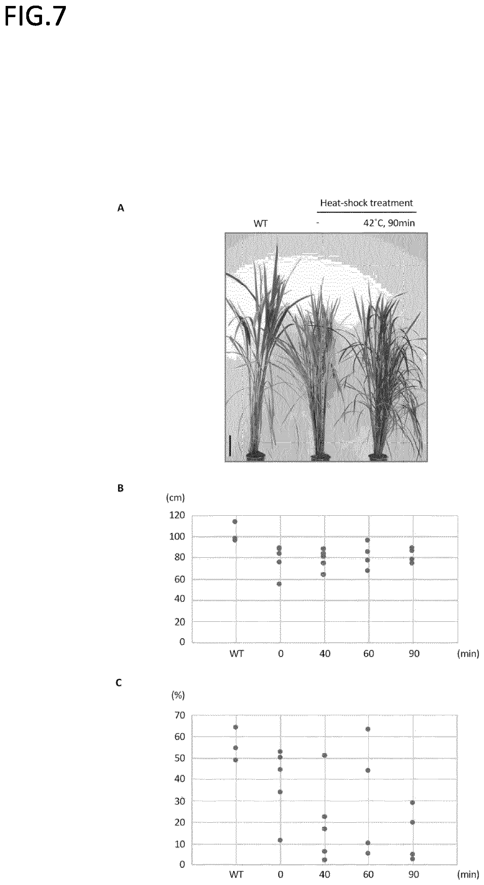

[Figure 7] Figure **7** shows the growth situation of T₀OsClpP5 KO plants. (**A**) T₀ plants in the ear emergence period. Left: Wild-type "Nipponbare". Middle: OsClpP5 KO T₀ plants derived from the callus lines that were not treated with heat shock. Right: OsClpP5 KO T₀ plants derived from the callus lines that were treated with heat shock. Scale bar: 12.5 cm. (**B**) The relative effect of the heat-shock treatment for the plant height of T₀ plants. The height of the wild-type plants which germinated from seeds and T₀ plants regenerated from marker-free callus lines were measured in their respective ear emergence period. The numbers on the horizontal axis show the length of time of the heat-shock treatment. 0 minute shows no heat-shock treatment, while 40 minutes, 60 minutes, and 90 minutes show the time during which the callus lines were maintained at 42°C for Ac/Ds-mediated marker excision. Each dot shows individual plants. (**C**) The relative effect of the heat-shock treatment for seed fertility of T₀ plants. The fertility of each plant was evaluated by averaging the fertility of two ears.

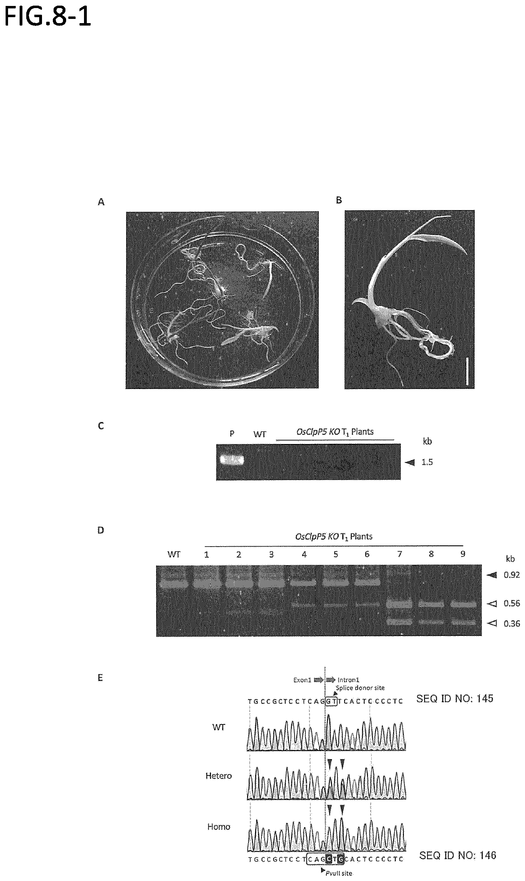

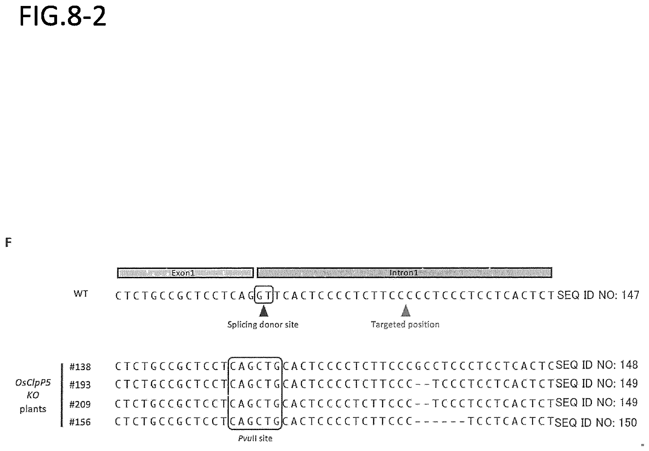

[Figure 8-1] Figure **8** shows heredity and segregation of the modified OsClpP5 gene in the T₁ generation. (**A**) Segregation of a phenotype in T₁ plants derived from T₀ plants having a heterozygosis-type OsClpP5 KO mutation. (**B**) A segregated albino plant having a homozygosis-type osclpp5 mutation. Scale bar: 5 mm. (**C**) The presence of a removal unit in the segregated albino plant. P shows pOsClpP5KO-Con5, which is a control plasmid, while WT shows a wild-type "Nipponbare" plant. (**D**) The results of CAPS analysis of a target region in the segregated plants. Lanes 1 to 3 show a segregated wild-type plant, lanes 4 to 6 show a heterozygosis-type plant, and lanes 7 to 9 show a homozygosis-type osclpp5 plant that exhibits an albino phonotype. The filled arrow indicates a PCR amplification product that does not have a PvuII site. The white arrows indicate a fragment amplified from an albino plant and digested with PvuII. (**E**) The spectra of Sanger sequencing of the OsClpP5 locus. The arrows show an introduced mutation.
[Figure 8-2] Figure **8** shows heredity and segregation of the modified OsClpP5 gene in the T₁ generation. (**F**) The footprint sequence of Ac/Ds-mediated marker excision that was detected among osclpp5 disruption strains that exhibit an albino phenotype.

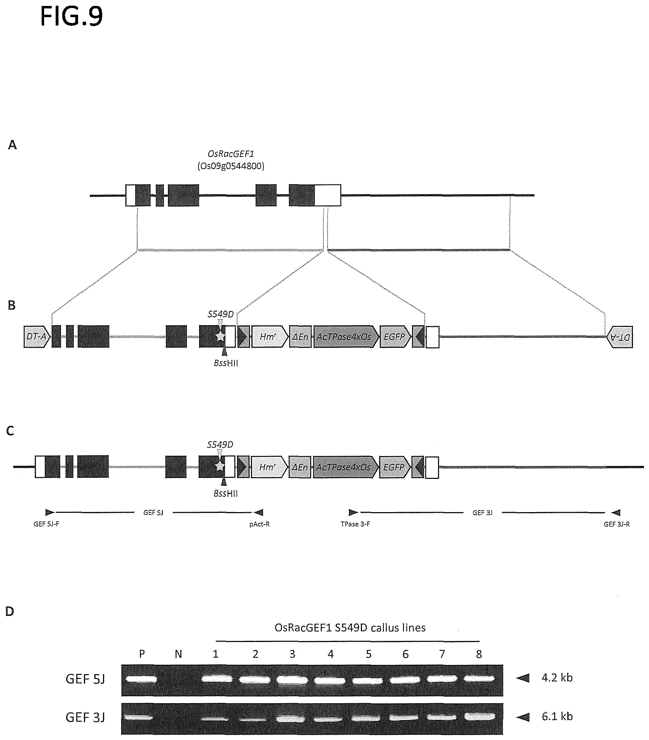

[Figure 9] Figure **9** shows primary modification of OsRacGEF1 by gene targeting which is mediated by homologous recombination. (**A**) An OsRacGEF1 gene in a rice genome. (**B**) The structure of pGEF1S549D-AcDs, which is a gene targeting vector. White boxes and black boxes show an untranslated region (UTR) and an exon, respectively. The left and right bars represent a DNA sequence corresponding to a homology region carried by the vector. The star in the 5'-homology region shows the position of a desired mutation that results in an S549D amino acid substitution of an OsRacGEF1 protein. The arrow indicates a new BssHII site adjacent to the S549D mutation. (**C**) The structure of OsRacGEF1 modified by gene targeting. The horizontal lines marked with GEF 5J and GEF 3J show 5' and 3' junction fragments made by homologous recombination. The adjacent black arrows show primers that were used for PCR screening of callus lines of a target (Table 2). (**D**) The PCR screening results of GT callus lines. P shows a control plasmid comprising GEF 5J or GEF 3J (Figure **9C**), N shows a callus of non-transformed "Kinmaze", and lanes 1 to 8 show a GT callus transformed with pGEF1S549D-AcDs.

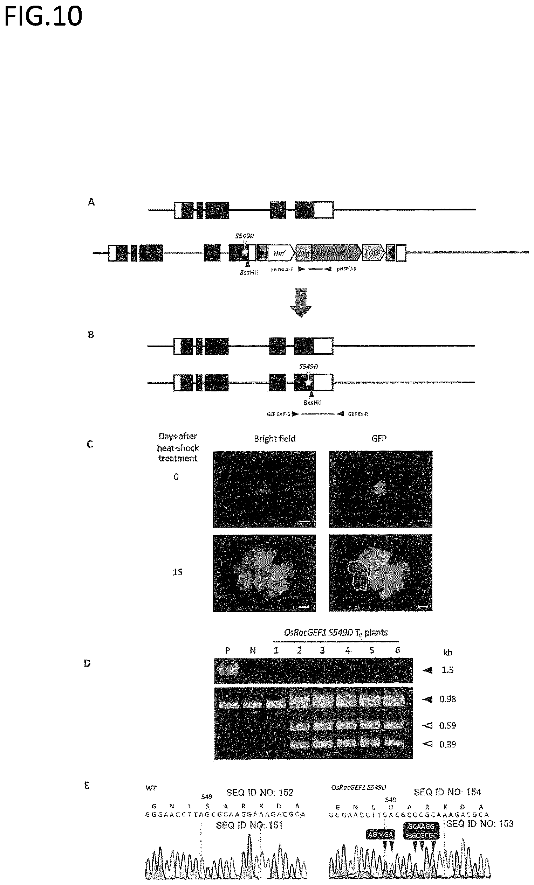

[Figure 10] Figure **10** shows excision of a positive marker from modified OsRacGEF1 by a heat-shock inducible Ac/Ds system. The black arrows show primers that were used for PCR analysis to detect the presence of a removal unit (Table 2). (**A**) The modified OsRacGEF1 gene before Ac/Ds-mediated removal unit excision. (**B**) The modified OsRacGEF1 gene after Ac/Ds-mediated removal unit excision. The black arrows show primers that were used for CAPS and DNA sequencing analysis (Table 2). (**C**) Proliferation of GT callus lines over time after heat-shock treatment. The proliferative cell lines without an EGFP signal were surrounded with a dotted line. Scale bar: 2.5 mm. (**D**) PCR and CAPS analysis of OsRacGEF1 S549D GT T₀ plants. The presence of a removal unit was analyzed by PCR using a primer pair (En No.2-F/pHSP J-R) (Table 2 and Figure **10A**). A DNA fragment comprising a desired mutation was amplified by PCR using a primer pair (GEF Ex F-5/GEF Ex-R) and digested with BssHII. The filled arrows indicate a PCR amplification product from unmodified OsRacGEF1. The white arrows indicate a fragment amplified from a modified target and digested with BssHII. P shows pGEF1S549D-Con, which is a control plasmid, while N shows a callus of non-transformed "Kinmaze". (**E**) The spectra of Sanger sequencing of the OsRacGEF1 locus. The arrows show an introduced mutation.

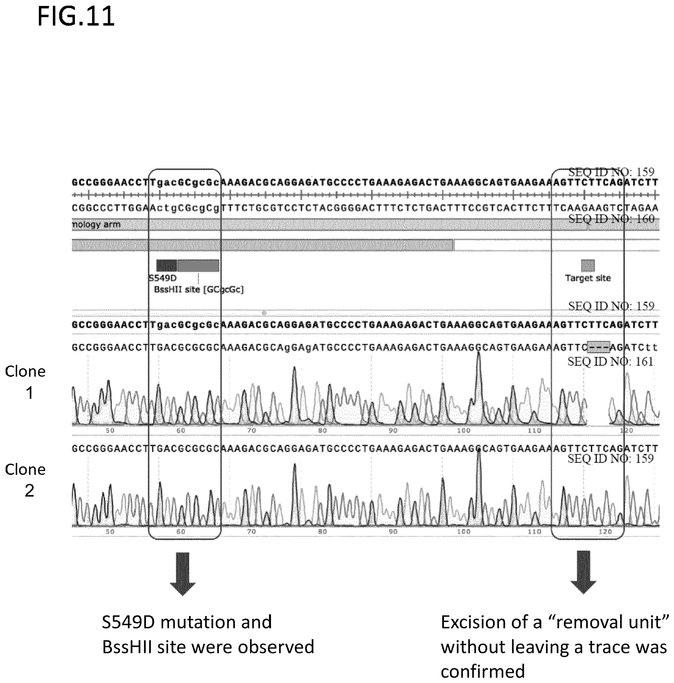

[Figure 11] Figure **11** shows the results of cloning sequence analysis of a target region of an OsRacGEF1 S549D variant callus.

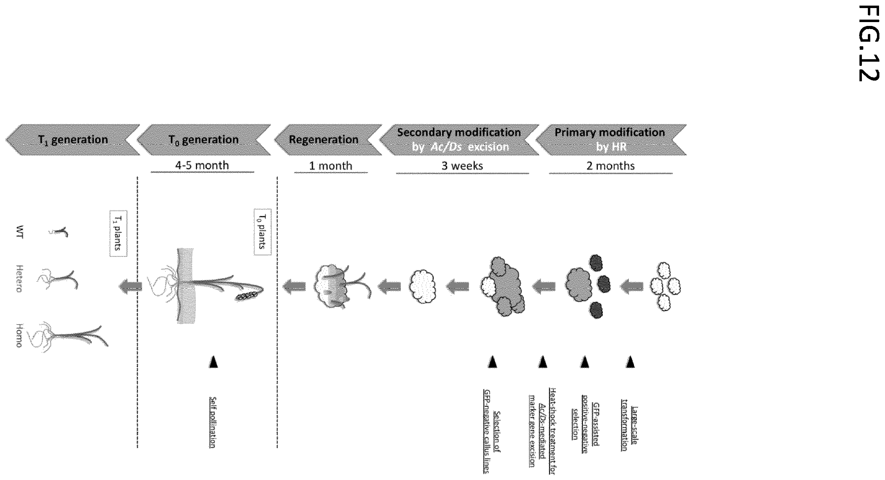

[Figure 12] Figure **12** shows a flow chart of making T₁ plants from calli introduced with the nucleic acid of the present invention.

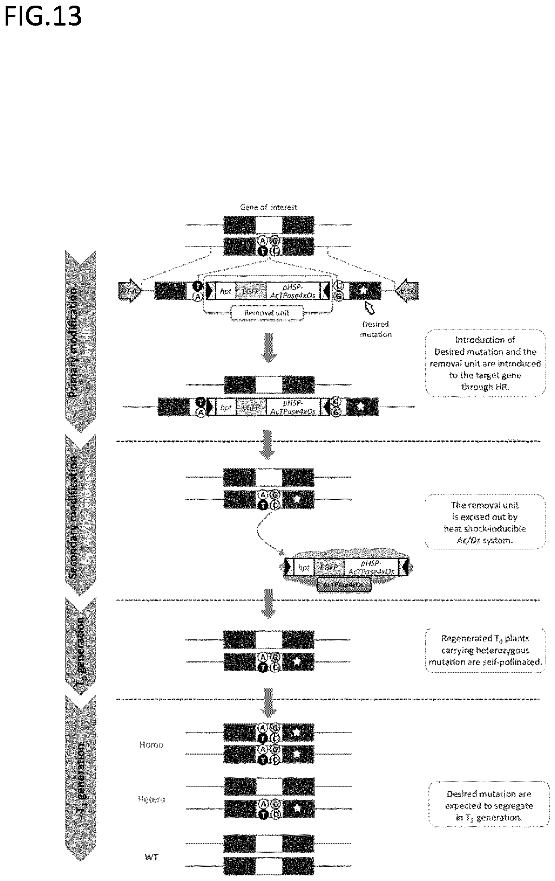

[Figure 13] Figure **13** shows a flow chart of gene modification that occurs while T₁ plants are made from calli introduced with the nucleic acid of the present invention.

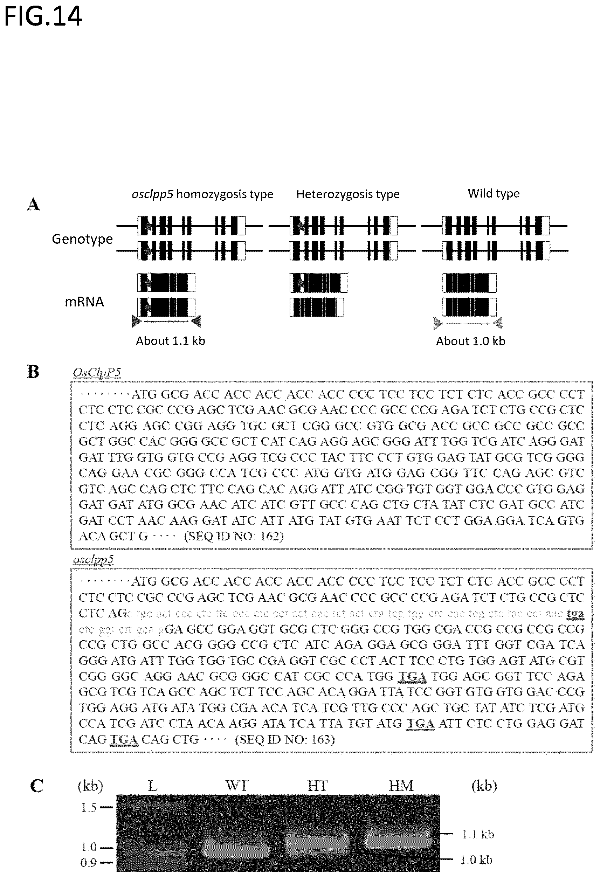

[Figure 14] Figure **14** shows the results of RNA expression analysis in a clpP5-Δspl secondary modification callus-derived T1 rice budding leaf. **A:** A schema of a genotype and an mRNA in the T1 generation of an OsClpP5 modification line. **B:** A part of the mRNA sequence of an OsClpP5 variant. TGA: shows a stop codon, and small letter: shows a first intron. **C:** An electrophoresis image of an OsClpP5 cDNA. WT: shows wild type, HT: shows hetero, and HM: shows homo.

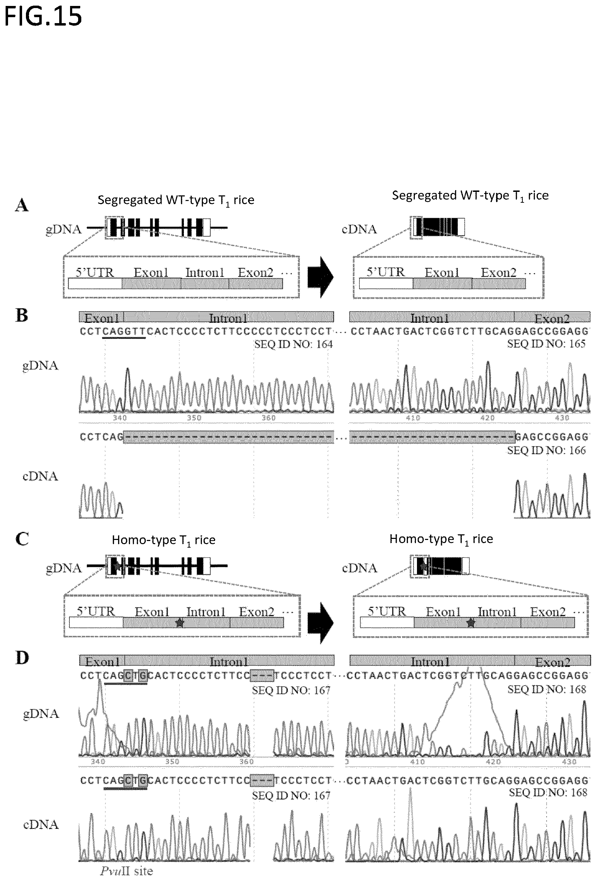

[Figure 15] Figure **15** shows the cDNA sequence of an OsClpP5 variant T1 plant body. **A:** A splicing schema of an OsClpP5 gene in a wild-type plant body segregated in the T1 generation. **B:** The base sequence of the OsClpP5 gene and a cDNA of a wild-type plant body segregated in the T1 generation. **C:** A splicing schema in the osclpP5 homozygosis type. **D:** The base sequence of a gene and a cDNA in the osclpP5 homozygosis type.

### [Description of Embodiments]

### 1. Nucleic acid for genome modification of a plant cell

The present invention provides a nucleic acid for genome modification of a plant cell, comprising a transposon (which may be hereinafter referred to as the "nucleic acid of the present invention"). The transposon comprises a marker gene and a gene (nucleotide sequence) encoding a transposase (a transposon comprising these sequences may be referred to as the "transposon of the present invention"). Further, the nucleic acid of the present invention comprises two types of regions that are homologous to at least a part of a target region in a plant cell for causing homologous recombination with the target region (specifically, the regions on the upstream side and the downstream side in the target region) (these regions may be hereinafter called "homology regions", and when each homology region is distinguished, they may be referred to as the "5' homology region" and/or "3' homology region"). A region consisting of the transposon and the homology regions may be hereinafter referred to as the "recombinant sequence". The definition of the plant cell, the type of the plant and the like are as described in 2. below.

As used herein, a "transposon" means a DNA sequence that is removed by a transposase. A terminal inverted repeat (TIR) which is recognized by a transposase is placed on both ends of the transposon. Examples of the transposon system used for the present invention include a DNA type transposon system although the transposon system used for the present invention is not particularly limited as long as it can remove the transposon introduced to a plant cell without leaving a trace in the genome of the plant cell. Examples of the DNA type transposon system include an Ac/Ds system derived from maize, Arabidopsis thaliana, tobacco, tomato or petunia, an EnSpm/CACTA system derived from maize, Arabidopsis thaliana or soy bean, an Mu system derived from maize, a Tam1/Tam2 system derived from snapdragon, a Tam3 system, a Dart/nDart system derived from rice, a PiggyBac system derived from Trichoplusia ni and the like. Thus, examples of the transposon used for the present invention include a sequence in which a marker gene and a gene encoding a transposase are inserted to a natural-type transposon such as a Ds (Dissociation) element (e.g., Ds1, Ds2, Ds6), an Ac (Activator) element of an Ac/Ds system; an Spm-s element, an En1 element, an Spm-I8 (dSpm) element or the like of an EnSpm/CACTA system; an Mu element (e.g., Mu1, Mu1.7, Mu7 or the like) of an Mu system; a Tam1 element, a Tam2 element or the like of a Tam-1/Tam-2 system; a Tam3 element of a Tam3 system or the like; a Dart element, nDart element of a Dart/nDart system; a PiggyBac element, or a transposon in which at least a part of the sequence of the natural-type transposon is modified (also referred to as an artificial-type transposon), and/or a sequence in which a part of the transposon is substituted with the sequence of these genes. Examples of the above-described artificial-type transposon include a combination of the above-described elements (e.g., a sequence in which a TIR and a subterminal region of a Ds element are substituted with a TIR and a subterminal region of an Ac element) and the like. As used herein, a transposon resulting from subjecting a natural-type or an artificial-type transposon to modification such as insertion or substitution of a sequence as described above, or any sequence comprising a TIR recognized by each transposase is referred to as transposon derived from each natural-type transposon. For example, a transposon resulting from subjecting a natural-type or an artificial-type Ds element to modification is referred to as transposon derived from a Ds element. Further, the transposase used for the present invention includes AcTPase (Genebank Accession No: X05424.1) that can remove a Ds element and Ac element, PBase (Genebank Accession No: EF587698.1) that can remove a PiggyBac element, a transposase encoded by a part of each natural-type transposon described above, and the like although the transposase used for the present invention is not particularly limited as long as it can remove the above-described transposon.

Further, a transposase may be a wild type, and may be a variant thereof having an activity to remove a transposon. The variant of a transposase includes a protein having a transposon removal activity and consisting of an amino acid sequence resulting from substitution, deletion, addition, and/or insertion of one or a few (e.g., two, three, four, five, six, seven, eight, nine, ten) amino acids in the amino acid sequence of a wild-type transposase, or a protein having a transposon removal activity and consisting of an amino acid sequence having 90% or greater (e.g., 91%, 92%, 93%, 94%, 95%, 96%, 97%, 98%, 99% or greater) identity to the above-described amino acid sequence. Examples of the variant of a transposase include AcTPase4x (Lazarow K. et al., Genetics, 191(3):747-756 (2012)) with an improved transposon removal activity due to substitution of four amino acid residues (specifically, E249A/E336A/D459A/D545A), which is a variant of AcTPase.

The amino acid sequence identity can be calculated using NCBI BLAST (National Center for Biotechnology Information Basic Local Alignment Search Tool) (https://blast.ncbi.nlm.nih.gov/Blast.cgi) of an identity calculation algorithm under the following condition (expectancy = 10; gap allowed; matrix = BLOSUM62; filtering = OFF). A sequence of the full length of a transposase is compared to another sequence in order to determine the identity.

The present inventors found that when AcTPase is used as a transposase, it is possible to remove a transposon without leaving a trace by replacing a base of one chain in contact with both ends of the region of the transposon with a base complementary thereto (e.g., if a base pair in contact with one end is A:T, replace it with T:A, and if a base pair in contact with one end is G:C, replace it with C:G). Thus, when AcTPase is used as a transposase, it is generally necessary to replace a base of one chain in contact with both ends of the region of a transposon with a base complementary thereto in order to remove the transposon without leaving a trace in the genome of a plant cell. Further, a gene encoding the transposase is desirably added with a sequence encoding a nuclear localization signal (NLS) (e.g., SV40-derived NLS encoding sequence (the sequence set forth in SEQ ID NO: 33)) that can function in the plant cell.

As used herein, a transposon not leaving a trace in the genome of a plant cell shall encompass not only the case where no trace of the transposon (e.g., the transposon, a sequence encoding a transposase, a footprint caused by removal of the transposon, or the like) is found in the genome but also the case where deletion, substitution, and/or insertion of 10 or less (e.g., 9, 8, 7, 6, 5, 4, 3, 2, or 1) nucleotide residues is found.

A TIR positioned on both ends of the transposon of the present invention can be appropriately selected depending on the type of the transposase. Specifically, when AcTPase is used, examples of the sequence of a TIR on one terminal include CAGGGATGAAA (SEQ ID NO: 1), TAGGGATGAAA (SEQ ID NO: 2), GAGGGATGAAA (SEQ ID NO: 3), TAGAGATGAAA (SEQ ID NO: 4), GAGCTATGAAA (SEQ ID NO: 5) and the like while the sequence of a TIR on the other terminal includes TTTCATCCCTA (SEQ ID NO: 6), TTTCATCTGAG (SEQ ID NO: 7), TTTCATCCCTA (SEQ ID NO: 8), TTTCATCCCTG (SEQ ID NO: 9), TTTCATCTCTA (SEQ ID NO: 10) and the like, wherein these sequences can be appropriately combined to be used. Examples of the above-described combination include the sequence set forth in SEQ ID NO: 1/the sequence set forth in SEQ ID NO: 6, the sequence set forth in SEQ ID NO: 1/the sequence set forth in SEQ ID NO: 7, the sequence set forth in SEQ ID NO: 2/the sequence set forth in SEQ ID NO: 8, the sequence set forth in SEQ ID NO: 2/the sequence set forth in SEQ ID NO: 9, the sequence set forth in SEQ ID NO: 2/the sequence set forth in SEQ ID NO: 10, the sequence set forth in SEQ ID NO: 3/the sequence set forth in SEQ ID NO: 9, the sequence set forth in SEQ ID NO: 4/the sequence set forth in SEQ ID NO: 8, the sequence set forth in SEQ ID NO: 5/the sequence set forth in SEQ ID NO: 8 and the like. When an EnSpm/CACTA system is used, examples of the sequence of a TIR on one terminal include CACTACAAGAAAA (SEQ ID NO: 11), CACTACAACAAAA (SEQ ID NO: 12), CACTACAAAAAAA (SEQ ID NO: 13), CACTATAAGAAAA (SEQ ID NO: 14), CACTACGCCAAAA (SEQ ID NO: 15), CACTACCGGAATT (SEQ ID NO: 16) and the like while the sequence of a TIR on the other terminal includes the complementary sequences (SEQ ID NO: 17 to 22) of each of the above-mentioned sequences. When a PiggyBac system is used, examples of the sequence of a TIR on one terminal include CCCTAGAAAGATA (SEQ ID NO: 23), CCCTAGAAAGATAGTCTGCGTAAAATTGACGCATG (SEQ ID NO: 24), CATGCGTCAATTTTACGCAGACTATCTTTCTAGGG (SEQ ID NO: 25) and the like while the sequence of a TIR on the other terminal includes the complementary sequences (SEQ ID NO: 26 to 28) of each of the above-mentioned sequences. The above-described specific sequences are exemplification, and a sequence exhibiting a high identity (e.g., 90% or greater) to the above-described sequences also can be used as a TIR as long as it is recognized by a transposase. Further, those skilled in the art can appropriately select a sequence even when a transposon system other than those mentioned above is used.

Further, one end or both ends of the transposon of the present invention may comprise a subterminal region (STR) adjacent to the above-described TIR which is joined to a transposase apart from the TIR. A STR generally consists of 100 to 300 bp. When AcTPase is used as a transposase, the STR preferably comprises, for example, at least one sequence (preferably three or more sequences) consisting of AACGG necessary for joining of AcTPase. In a preferred embodiment, a transposon comprising an IVR and a STR includes a transposon having the sequence set forth in SEQ ID NO: 34 (as used herein, it is referred to as "Ds_5" for convenience sake) on one terminal and having the sequence set forth in SEQ ID NO: 35 (as used herein, it is referred to as "Ds_3"for convenience sake) on the other terminal, or a transposon having a sequence having a high identity (e.g., 90% or greater, 95% or greater, 96%, 97%, 98%, 99% or greater) to these sequences, and the like. Those skilled in the art can appropriately select the sequence of the STR depending on the type of the transposase.

The number of nucleotides of the transposon of the present invention may be any number of nucleotides that can be excised out by a transposase. Although the number of nucleotides of the transposon of the present invention depends on the number of nucleotides of a marker gene, a transposase or the like described below, the number is generally 1500 to 10000 nucleotides and is preferably 3000 to 8000 nucleotides.

As used herein, a "target region" means a region on the genome of a plant cell in which homologous recombination with a recombinant sequence occurs. A desired mutation is introduced to at least one of the 5' homology region and the 3' homology region of a recombinant sequence in advance, and homologous recombination is caused between a sequence having the mutation and a target region, whereby the desired mutation can be introduced to the target region of a plant cell. A target region can be optionally selected from a region on the genome of a plant cell, may be a region encoding a protein, or may be a DNA encoding a noncoding RNA such as a functional RNA. Alternatively, a target region may be not only a region that does not encode a protein or a noncoding RNA (such as an UTR) but also a region that adjusts the expression and the chromosome structure of a transcriptional product encoding a protein or a noncoding RNA. Further, a target region is generally endogenous, but may be a DNA inserted to a genome DNA of a plant cell in a foreign manner.

The sequence of a homology region may be a sequence that is completely identical to the region of at least a part of a target region, but may be a sequence preferably having 80% or greater (e.g., 85% or greater, 90% or greater, 95% or greater, 96% or greater, 97% or greater, 98% or greater, 99% or greater) identity to the completely identical sequence as long as homologous recombination can occur in a plant cell.

Further, the number of nucleotides of a homology region may be any number that can cause homologous recombination between the homology region and a target region. Each of the both sides of a transposon is generally added with a homology region consisting of 500 to 7000 nucleotides (preferably 1000 to 5000 nucleotides, more preferably 1500 to 4000 nucleotides, and further preferably about 3000 nucleotides (e.g., 2500 to 3500 nucleotides)).

A "mutation" which is introduced to a target region is not particularly limited, and may be a null mutation such as a nonsense mutation, a frameshift mutation, an insertion mutation or a splice site mutation, or may be a silent mutation. Further, examples of a mutation in a target region include deletion, substitution, addition, and/or insertion of one or more (e.g., 2, 3, 4, 5, 6, 7, 8, 9, 10 or more) nucleotides in the region. Further, a mutation may be introduced to only one site or may be introduced to multiple sites (e.g., 2, 3, 4, 5 or more sites). When a mutation is introduced to multiple sites, the mutation introduction sites may be close to each other or may be about hundreds or thousands of bases away from each other.

A "marker gene" comprised in a transposon may be any gene whose expression serves as an indicator for selecting a few transformed cells introduced with a recombinant sequence from non-transformed cells. Examples of the marker gene include a reporter gene (e.g., a gene encoding a fluorescent protein, a gene encoding a luminescent protein, a gene encoding a protein that assists fluorescence, luminescence, or color development), a drug-resistant gene and the like. Only one type of marker gene may be used, or two or more types of marker gene (e.g., combination of a gene encoding a fluorescent protein and a drug-resistant gene) may be used. Use of two types or more can decrease false positivity more. As used herein, a protein encoded by a marker gene may be referred to as "marker protein".

Examples of a fluorescent protein include, but are not limited to, a blue fluorescent protein such as Sirius, TagBFP, or EBFP; a cyan fluorescent protein such as mTurquoise, TagCFP, AmCyan, mTFP1, MidoriishiCyan, or CFP; a green fluorescent protein such as TurboGFP, AcGFP, TagGFP, Azami-Green (e.g., hmAG1), ZsGreen, EmGFP, EGFP, GFP2, or HyPer; a yellow fluorescent protein such as TagYFP, EYFP, Venus, YFP, PhiYFP, PhiYFP-m, TurboYFP, ZsYellow, or mBanana; an orange fluorescent protein such as KusabiraOrange (e.g., hmKO2), or mOrange; a red fluorescent protein such as TurboRFP, DsRed-Express, DsRed2, TagRFP, DsRed-Monomer, AsRed2, or mStrawberry; a near infrared fluorescent protein such as TurboFP602, mRFP1, JRed, KillerRed, mCherry, HcRed, KeimaRed (e.g., hdKeimaRed), mRasberry, or mPlum.

Examples of a luminescent protein include, but are not limited to, Aequorin. Further, examples of a protein that assists fluorescence, luminescence, or color development include, but are not limited to, an enzyme that decomposes a fluorescent, luminescent, or color development precursor such as a luciferase, phosphatase, peroxidase, or β-lactamase.

Examples of a drug-resistant gene include a drug-resistant gene such as a hygromycin-resistant gene (hygromycin phosphotransferase gene, hpt), a kanamycin-resistant gene, or a neomycin-resistant gene, and an herbicide-resistant gene such as an ALS (AHAS) gene or PPO gene. Among them, a hygromycin-resistant gene is preferred from the viewpoint of high selection efficiency in transformation using a rice callus.

Further, the nucleic acid of the present invention preferably comprises a negative marker gene in a region other than a recombinant sequence in order to remove cells in which a part or the whole of a region of the nucleic acid is unintentionally inserted to a region other than a target region. Thus, in one embodiment of the present invention, a nucleic acid comprising (1) a transposon of the present invention, (2) a homology region, and (3) a negative marker gene is provided. Examples of the above-described negative marker gene include, but are not limited to, diphtheria toxin protein A chain gene (DT-A), Exotoxin A gene, Ricin toxin A gene, codA gene, Cytochrome P-450 gene, RNase T1 gene and barnase gene.

A control region for causing the above-described marker gene and/or negative marker gene to express a protein encoded by the gene in a plant cell is operably linked to the nucleic acid of the present invention. When the protein is constantly expressed, examples of the control region include a promoter such as a cauliflower mosaic virus (CaMV) 35S promoter, a promoter of a nopaline synthase gene, a polyubiquitin 1 promoter derived from maize, an actin promoter derived from rice, or an elongation factor 1α promoter derived from rice, a terminator sequence for terminating transcription of a gene induced by the promoter or the like (heat-shock protein 17.3 terminator derived from rice, heat-shock protein 16.9a terminator derived from rice, actin terminator derived from rice, terminator of a nopaline synthase gene, terminator of an octopine synthase (OCS) gene, CaMV 35S terminator and the like), and a ΔEn terminator derived from a maize En/Spm transposon (En element) (Terada R. et al., Nat Biotechnol, 20(10):1030-1034 (2002)). Furthermore, an enhancer such as intron 1 of a castor bean Catalase gene, a CaMV 35S enhancer, a transcription enhancer E12, or an omega sequence also may be comprised in the control region in order to enhance the gene expression efficiency.

Further, it is preferable that an inducible promoter is operably linked to the nucleic acid of the present invention in order to cause a gene encoding the above-described transposase to transiently express the protein in a plant cell. Examples of the inducible promoter include a drug inducible promoter wherein expression of a transposase is induced by a drug, a stimulus inducible promoter wherein expression of a transposase is induced by a stimulus (e.g., a light inducible promoter wherein expression of a transposase is induced by a light stimulus, a heat-shock inducible promoter wherein expression of a transposase is induced by heat shock and the like) and the like. The inducible promoter is preferably a heat-shock inducible promoter. Further, the above-described terminator or enhancer may be connected to the nucleic acid of the present invention in order to control expression of a transposase.

A drug inducible promoter includes a TRE promoter (wherein expression of a protein is induced by contact with Doxycycline (Dox), tetracyline, or a derivative thereof or cancellation of the contact), a metallothionein promoter (wherein expression of a protein is induced by contact with a heavy metal ion), a steroid responsive promoter (wherein expression of a protein is induced by contact with a steroid hormone or a derivative thereof) and the like. Examples of a light inducible promoter include, but are not limited to, a ribulose-bisphosphate carboxylase small subunit gene (Rubisco) promoter (R. Fluhret al., Proc. Natl. Acad. Sci. USA 83:2358, 1986), a promoter of a fructose-1,6-bisphosphatase gene (Japanese National Phase PCT Laid-Open Publication No. 7-501921), a promoter of a light harvesting chlorophyll a/b binding protein gene (Japanese Laid-Open Publication No. 5-89), and a LP2 leucine-rich repeat receptor kinase gene promoter of rice (Plant Biotechnology Journal

(2009)7, pp.1-16). Examples of a heat-shock inducible promoter include, but are not limited to, a promoter of a heat-shock protein (hsp) 16.9A gene derived from rice, a promoter of a hsp 16.9B gene, a promoter of a hsp 16.9C gene, a promoter of a hsp 18.2 gene derived from Arabidopsis thaliana, and a promoter of a hsp 70 gene derived from drosophila.

The nucleic acid of the present invention can be made by a method known per se. For example, it is possible to perform cloning by synthesizing, based on known DNA sequence information of a transposon, a transposase, a target region or the like, an oligo DNA primer to cover a desired part of the sequence and using a total RNA or an mRNA fraction prepared from a cell having the sequence as a template to perform amplification by RT-PCR method. Alternatively, it is also possible to chemically synthesize a DNA chain or connect a synthesized partially overlapping oligo DNA short chain using PCR method (overlapping PCR method) or Gibson Assembly method to thereby construct a DNA encoding the full length thereof. Construction of a full length DNA with chemical synthesis or combination with PCR method or Gibson Assembly method has an advantage in that a codon to be used can be designed over the CDS full length depending on the host introduced with the DNA. In expression of a heterologous DNA, converting the DNA sequence to a codon which is highly frequently used in a host organism can be expected to increase the protein expression level. For the data of the frequency of use of codons in hosts to be used, for example, the genetic code use frequency data base (http://www.kazusa.or.jp/codon/index.html) disclosed on the website of Kazusa DNA Research Institute can be used. Alternatively, documents containing the frequency of use of codons in each host may be referred to. With reference to obtained data and a DNA sequence which is intended to be introduced, a codon which is less frequently used in a host among the codons used for the DNA sequence may be converted to a codon which encodes the same amino acid and is highly frequently used. For example, when a host cell is a rice cell, a gene encoding a transposase optimized for codon use of monocotyledons such as rice or general angiosperms such as Arabidopsis thaliana can be used. For example, AcTPase4x having codon use suitable for expression in rice includes a DNA having the nucleotide sequence set forth in SEQ ID NO: 29 (the amino acid sequence is set forth in SEQ ID NO: 30).

The nucleic acid of the present invention may be in the form of a single-stranded DNA or may be in the form of a double-stranded DNA, and may be a linear DNA or may be a circular DNA. The nucleic acid of the present invention is preferably provided in the form of an expression vector placed under the control of the control region which is functional in a host cell. The nucleic acid of the present invention can be prepared in a suitable form depending on the type of the plant cell that is the subject of introduction.

An expression vector that can be replicated in a plant cell is not particularly limited as long as it has a replication origin (e.g., ori of Ti plasmid, Ri plasmid or the like) that functions in the plant cell, but the expression vector preferably has a replication origin of E. coli (e.g., ColE1 ori or the like) as well. When Agrobacterium method is used as a method for introducing a gene, it is generally necessary to further contain a T-DNA fragment from which a pathogenic gene of a Ti plasmid or a Ri plasmid is removed (including border sequences RB (right border) (e.g., the sequence set forth in SEQ ID NO: 31) and LB (left border) (e.g., the sequence set forth in SEQ ID NO: 32)). Examples of specific vectors include pBI-based vectors, pPZP-based vectors, or pSMA-based vectors and the like. Further, more suitable forms include binary vector-based vectors (pZHG, pKOD4, pBI121, pBI101, pBI101.2, pBI101.3, pBIG2113 and the like).

### 2. A method for producing plant cells with a modified genome

In another embodiment, the present invention provides a method for producing plant cells having a mutation in a target region of a double-stranded DNA by a two-stage selection step for plant cells introduced with the nucleic acid of the present invention of the above 1 (which may be hereinafter referred to as the "production method of the present invention"). The method comprises, for example: (1) the step of selecting plant cells introduced with the nucleic acid of the present invention; (2) the step of culturing the plant cells selected in step (1) under a condition wherein an inducible promoter is activated; and (3) the step of selecting a cell in which expression of a marker protein is eliminated from a population comprising the plant cells cultured in step (2). With this method, it is possible to introduce only a necessary mutation without leaving an unnecessary artificial DNA sequence such as an exogenous transposon or a gene encoding a transposase in the genome of the plant cells or while suppressing residual of the sequence as shown in the Examples described below. Thus, with the production method of the present invention, plant cells that have a mutation in a target region of a double-stranded DNA and do not have any of an exogenous transposon and a gene encoding a transposase, and further, more preferably, do not have a footprint caused by removal of the transposon, are produced. In other words, with the present invention, plant cells wherein no trace of a transposon is left in the genome are produced. That is, it is also possible to quickly modify the genome of plant cells without the need for a step of making a multiple transformant or performing backcross with a wild type in order to remove the exogenous factor as in the conventional techniques.

A plant from which cells to be introduced with the nucleic acid of the present invention are derived is not particularly limited, but is preferably a monocotyledon or a dicotyledon. Examples of a monocotyledon include a gramineous plant. The gramineous plant includes plants that belong to Oryza, Triticum, Hordeum, Secale, Saccharum, Sorghum, or Zea, which specifically include, but are not limited to, maize, sorghum, wheat, rice, oat, barley, rye, and millet. A preferable gramineous plant is maize, wheat, and rice. Wheat also includes wheat variety Nourin No. 61, from which a transformant was difficult to obtain with the conventional methods.

Examples of a dicotyledon include, but are not limited to, Brassicaceae plants, Leguminosae plants, Solanaceae plants, Cucurbitaceae plants, and Convolvulaceae plants. Brassicaceae plants include plants that belong to Raphanus, Brassica, Arabidopsis, Wasabia, or Capsella, which specifically include, but are not limited to, Brassica rapa var. pekinensis, rapeseed, cabbage, cauliflower, Raphanus sativus var. hortensis, Brassica rapa subsp. oleifera, Arabidopsis thaliana, Eutrema japonicum, and Capsella bursa-pastoris. A preferable Brassicaceae plant is Brassica rapa var. pekinensis and Capsella bursa-pastoris. Examples of Leguminosae plants include, but are not limited to, soy bean, Vigna angularis, Phaseolus vulgaris, and Vigna unguiculata. A preferable Leguminosae plant is soy bean. Examples of Solanaceae plants include, but are not limited to, tomato, eggplant, and potato. A preferable Solanaceae plant is tomato. Examples of Cucurbitaceae plants include, but are not limited to, oriental melon, cucumber, melon, and watermelon. A preferable Cucurbitaceae plant is oriental melon. Examples of Convolvulaceae plants include, but are not limited to, Ipomoea nil, Ipomoea batatas, and Calystegia japonica. A preferable Convolvulaceae plant is Ipomoea batatas.

Apart from the above plants, the examples of the plants further include plants of Rosaceae, Lamiaceae, Liliaceae, Chenopodiaceae, Apiaceae, Asteraceae and the like. Furthermore, any tree species, any fruit tree species, Moraceae plants (e.g., rubber), and Malvaceae plants (e.g., cotton) are included.

As used herein, a "plant body" encompasses all of a plant individual, a plant organ, a plant tissue, a plant cell, and a seed. Examples of a plant organ include a root, a leaf, a stem, and a flower and the like. Further, a plant cell also includes a cell in a plant body in addition to a cultured cell. Furthermore, a plant cell in various forms (e.g., a suspension cultured cell, a protoplast, a section of a leaf, a section of a root, a callus, an immature embryo, pollen and the like) is included.

The production method of the present invention may comprise the step of introducing the nucleic acid of the present invention to plant cells prior to the above-described step (1). Introduction of the nucleic acid of the present invention (e.g., a nucleic acid in the form of an expression vector) can be implemented for appropriate tissue (e.g., a callus, a root, a leaf, a seed, a growing point or the like) depending on the type of the plant cells in accordance with a known method (e.g., Agrobacterium method, PEG method, electroporation method, particle gun method or the like). For example, in the case of rice, Agrobacterium method, whisker direct introduction method or the like is generally used, but the used method is not limited thereto. For example, in the case of Agrobacterium method, a callus is induced from a rice seed in accordance with a common method, the callus is infected with Agrobacterium introduced with a T-DNA fragment of a vector for Agrobacterium expression which is incorporated with a part or the whole of the region of the nucleic acid of the present invention, and the bacterium is removed after a few days (e.g., three days). Meanwhile, in the case of whisker direct introduction method, an expression vector is mixed with polyornithine to form a complex, the complex is then added to a rice callus together with potassium titanate whiskers and mixed, followed by ultrasonic treatment.

In the case of wheat or maize, for example, an expression vector can be introduced by using Agrobacterium method in the same manner, with an immature embryo collected from an immature seed as a plant material.

When PEG method or electroporation method is used, a protoplast is prepared from an appropriate cell/tissue in accordance with a common method, and an expression vector is introduced to the protoplast. In the case of particle gun method, an expression vector adsorbed to a gold microparticle can be introduced to a callus, an immature embryo, a growing point present in a shoot apex or an axillary bud or the like by using a particle gun.

With particle gun method or Agrobacterium method, gene introduction often results in a chimera. Thus, sample cells wherein the above-described nucleic acid is highly frequently introduced to germ line cells need to be used for transformation. For example, an embryo, a hypocotyl section, an embryogenic callus, an isolated growing point and the like are included.

Culture of plant cells introduced with the nucleic acid of the present invention can be implemented in accordance with a known method depending on the type. A preferable medium used for culture is a solid medium (e.g., an agar medium, an agarose medium, a gellan gum medium or the like). Further, a medium preferably contains a carbon source, a nitrogen source, an inorganic substance or the like which is necessary for growth of a transformant. For example, an N6 medium, an MS medium, an LS medium, a B5 medium or the like is used as a basal medium. A plant growth substance (e.g., auxins, cytokinins or the like) or the like may be appropriately added to a medium. PH of a medium is preferably about 5 to about 8. Culture temperature can be appropriately selected within the range of about 20°C to about 35°C in general depending on the type of the plant cells. For example, a rice callus can be generally cultured at 28 to 33°C, and preferably at 30 to 33°C.

Those skilled in the art can perform step (1) of the production method of the present invention through appropriate selection by a known method depending on the type of the marker gene that is used. For example, when a drug-resistant gene is used, plant cells in which a mutation or the like is introduced to a target region can be selected by culturing plant cells introduced with the nucleic acid of the present invention in the presence of a corresponding drug. For example, when hygromycin is used as a drug, the concentration thereof in a medium is preferably 10 mg/L to 200 mg/L (e.g., 50 mg/L).

When a reporter gene is used, plant cells in which a mutation or the like is introduced to a target region can be selected by detecting a signal from a reporter protein encoded by the gene using a predetermined detection apparatus. The detection apparatus includes, but is not limited to, a flow cytometer, an imaging cytometer, a fluorescence microscope, a luminescence microscope, a CCD camera and the like. Those skilled in the art can use a suitable apparatus as said detection apparatus depending on the type of the reporter protein. For example, when the reporter protein is a fluorescent protein or a luminescent protein, selection is possible using a flow cytometer. When the reporter protein is a protein that assists fluorescence, luminescence, or color development, selection is possible by using a microscope, using a culture dish coated with a photoresponsive cell culture substrate, irradiating plant cells subjected to color development or the like with light, and utilizing the cells that were not irradiated being peeled from the culture dish.

Further, after performing the step of selection using expression of the marker protein as an indicator, PCR method, sequencing method, Southern blotting method, CAPS (cleaved amplified polymorphic sequence) method or the like may be used to confirm that a transposon has been introduced to a target region of plant cells by homologous recombination.

The "condition wherein an inducible promoter is activated" in step (2) of the production method of the present invention means a condition wherein, when the inducible promoter is a drug inducible promoter, continuous culture of plant cells in the presence of a substance described in the above 1. corresponding to each drug inducible promoter or continuous culture of plant cells in the absence of a substance that inhibits activation of the promoter activates the promoter to thereby induce expression of a transposase. When the inducible promoter is a stimulus inducible promoter, the above condition means a condition wherein culture of plant cells under a stimulus (e.g., light or heat) corresponding to each promoter activates the promoter to thereby induce expression of a transposase.

The culture time of step (2) is not particularly limited as long as a transposon is removed within the time, but the culture time is preferably 5 minutes or more (e.g., 5 minutes, 10 minutes or more). Further, the culture time is preferably 120 minutes or less (e.g., 120 minutes, 90 minutes or less) in order to suppress damage to plant cells due to drugs or stimuli. In a preferred embodiment, the time of step (1) is 40 minutes to 60 minutes, particularly preferably 40 minutes. When a drug response inducible vector is used, the concentration of a drug in a medium in step (1) is not particularly limited as long as a transposase is expressed in cells. For example, when Dox is used, about 0.4 µg/mL to 1.5 µg/mL is preferred. When a drug other than Dox is used, those skilled in the art can appropriately set the concentration. The culture temperature of when a heat-shock response inducible vector is used is not particularly limited as long as expression of a transposase is induced, but is preferably 32°C or greater (e.g., 35°C, 36°C, 37°C, 38°C, 39°C, 40°C or greater). Further, 47°C or below (e.g., 46°C, 45°C, 44°C, 43°C or below) is preferred in order to suppress damage to plant cells due to heat. In a preferred embodiment, the culture temperature is 42°C. The present inventors found that when a heat-shock responsive vector is used, a removal unit can be removed without applying a heat-shock stimulus in some cases (data not shown). Thus, even when a heat-shock responsive vector is used, cells may be cultured at normal culture temperature (e.g., about 20°C to about 35°C).

In step (3) of the production method of the present invention, when expression of a marker protein cannot be detected or when the expression level of a marker protein lowers to a background level, it can be evaluated that expression of the marker protein is eliminated. Detection of expression of a marker protein or measurement of the expression level can be appropriately performed by using, for example, the above-described detection apparatus.

Further, after performing the step of selection using expression of the marker protein as an indicator, PCR method, sequencing method, Southern blotting method, CAPS (cleaved amplified polymorphic sequence) method or the like may be used to confirm that a transposon has been removed from a target region.

### 3. A plant cell with a modified genome or a plant body comprising the cell

In another embodiment of the present invention, a plant cell (which may be hereinafter referred to as the "plant cell of the present invention") obtained by the production method of the present invention of the above 2. or a plant body comprising the cell is provided. A plant body comprising the plant cell of the present invention can be obtained by regenerating the cell. Since the plant cell of the present invention has a desired mutation in a target region, the phenotype of a plant body regenerated from such a plant cell can vary in association with the mutation. Thus, it is possible to efficiently analyze the function and the like of the target region by using the plant cell of the present invention. The definition of plant cell and a plant body, the type of plants and the like are as described in the above 2.

A transformant clone confirmed to be introduced with a mutation, which is obtained by the production method of the present invention, can be regenerated into a plant body by a regeneration method known per se. In the case of rice, examples of a regeneration method include a method described in Toki S. et al., Plant Physiol. 100 (3) :1503-1507 (1992), a method described in Christou P. et al., Bio/Technology 9:957-962 (1991), a method described in Hiei Y. et al., Plant J., 6:271-282, (1994) and the like.

Once a plant body comprising a cell in which a mutation is introduced to a target region is obtained in this manner, it is possible to obtain progeny from the plant body by sexual reproduction or asexual reproduction. Further, it is also possible to obtain a propagation material (e.g., seeds, fruits, cuttings, stubbles, calli, protoplasts or the like) from the plant body or progeny or clones thereof and mass-produce the plant body based on the propagation material. Thus, the present invention includes a plant body comprising the plant cell of the present invention, progeny and clones of the plant body, and a propagation material of the plant body and progeny and clones thereof. When a mutation is introduced to heterozygosity, an R1 plant obtained by self-pollination of a resulting plant body is further subjected to self-pollination to obtain an R2 plant, whereby a plant body in which a mutation is introduced to homozygosity can be obtained.

### 4. A method for modifying the genome of a plant cell

In another embodiment, the present invention provides a method for introducing a mutation to a target region of a double-stranded DNA of a plant cell for plant cells introduced with the nucleic acid of the present invention of above 1. The method comprises, for example: (1) the step of selecting plant cells introduced with the nucleic acid of the present invention; and (2) the step of culturing the plant cells selected in step (1) (preferably culturing the plant cells under a condition wherein an inducible promoter is activated), and may optionally comprise (3) the step of selecting a cell in which expression of a marker protein is eliminated from a population comprising the plant cells cultured in step (2). With this method, it is possible to introduce only a necessary mutation without leaving an unnecessary artificial DNA sequence such as an exogenous transposon or a gene encoding a transposase in the genome of the plant cells or while suppressing residual of the sequence. Thus, with the above-described method, only a mutation of interest can be introduced to the genome of a plant cell without introducing any of a plant cell exogenous transposon and a gene encoding a transposase, more preferably, and further a footprint caused by removal of the transposon. In other words, the present invention provides a method for modifying the genome of a plant cell wherein a trace of a transposon is not left in the genome. The above-described steps (1) to (3) can be performed in the same manner as the method described in above 2.

The present invention is described hereinafter based on Examples. However, the present invention is not limited to these Examples.

### [Examples]

Experiments were conducted in the following manner in the Examples described below.

### <Plant materials and growth conditions>

Japonica rice (Oryza sativa L.) (cultivar: "Nipponbare" and "Kinmaze") was used in the Examples. Rice was grown in a green house under long day conditions (16 hours:8 hours, light:dark, 28°C:22°C).

### <Construction of a marker excision system>

A DNA transposon Ac/Ds (Activator/Dissociation) system of artificially manipulated maize was used for heat-shock inducible marker excision. The DNA sequence (Muller-Neumann M. et al., Mol. Gen. Genet, 198(1):19-24 (1984)) of an Ac element isolated from a wx-m7 allele of maize was referred to.

An expression cassette of inducible-type AcTPase was synthesized based on the following design. An AcTPase gene of rice (AcTPase₄ₓOs) comprises a codon-optimized ORF (the sequence set forth in SEQ ID NO: 47) of enhanced AcTPase (Lazarow K. et al., Genetics 191:747-756 (2012)) in which a nuclear localization signal of an SV40 large T antigen and intron 2 of a wild-type AcTPase gene in a corresponding position are fused. A region adjacent to 5' of Oshsp16.9C (Chang P.L. et al., Botanical Bulletin- Academia Sinica Taipei, 42(2):85-92 (2001), Guan J.C. et al., Plant Mol Biol, 56(5):795-809 (2004), Itoh H. et al., Nat Genet, 42(7):635-638 (2010)) was used as a heat-shock promoter (the sequence set forth in SEQ ID NO: 46). AcTPase₄ₓOs was placed between the heat-shock promoter of rice and the NOS terminator (the sequence set forth in SEQ ID No: 48) (Figure **1C**).

An artificial Ds element was designed to have ΔEn (Terada R. et al., Nat Biotechnol, 20(10):1030-1034 (2002)) (the sequence set forth in SEQ ID No: 45) that is a transcription terminator in a region within 300 bp from both terminals of the Ac element. This terminal region comprises a terminal inverted repeat (TIR) and an adjacent subterminal region having an AcTPase binding motif essential for making transposition of the Ac/Ds element efficient (Becker H.A. and Kunze R., Mol Gen Genet. 254(3):219-230 (1997)).

Binary vectors for a GUS reporter assay were made based on pZEN11 (Shimatani Z. et al., Mol Genet Genomics. 281(3):329-344 (2009)). In order to construct pZEN30NC, a Ds element was inserted to the downstream of a p35S promoter that prevents expression of GUSPlus (Figure **1B**). In pZEN31E, an inducible AcTPase cassette was inserted adjacently to the ΔEn element of pZEN30NC (Figure **1C**). Further, a vector that imitates the result of Ds excision was constructed as pZEN30PC (Figure **1A**).

### <Evaluation of an Ac/Ds-mediated marker excision system>

Similarly to a prior report (Shimatani Z. et al., Mol Genet Genomics. 281(3):329-344 (2009)), binary vectors, i.e., pZEN30PC, pZEN30NC, and pZEN31E, were introduced to proliferative rice calli derived from a germ disc of "Nipponbare". After selection for four weeks, hygromycin-resistant callus lines were used for evaluation of an inducible Ac/Ds-mediated marker excision system. Each callus line was divided into two copies, wherein one of them was cultured at normal temperature (31.5°C) while the other was exposed to high temperature of 42°C for 90 minutes. After subculture for one week, excision of the artificial Ds element was studied by histochemical GUS reporter assay and PCR analysis in the same manner as the above description (Shimatani Z. et al., Mol Genet Genomics. 281(3):329-344

(2009)). In order to determine a footprint generated by Ds excision, the amplification product of PCR was cloned into pCR-BlutnII-TOPO (Thermo Fisher Scientific) and analyzed by Sanger sequencing.

### <A gene targeting vector having an autonomous marker excision system>

Modules as shown in Figure **3** and Figure **4** were combined to construct a gene targeting vector having an autonomous marker removal system. A homology region for homologous recombination (HR) was made by PCR using a Tks Gflex DNA polymerase (Takara Bio) and an appropriate primer listed in Table 1 and Table 2.

In order to construct vectors pOsClpP5KO-AcDs and pOsClpP5KO-Ds, 3 kb (the sequence set forth in SEQ ID NO: 42) of a 5' homology arm comprising a promoter region of OsClpP5 was prepared from the genome of "Nipponbare" by nested PCR, which was then fused with the 5' terminal region of a Ds element by overlapping PCR method (Figure **3B**). A PvuII site that results in a KO mutation was introduced with this process (Figure **4B**). Similarly, 3 kb (the sequence set forth in SEQ ID NO: 50) of a 3' homology arm comprising a sequence encoding OsClpP5 was amplified and fused to the 3' terminal region of the Ds element. Similarly, 3.55 kb and 3.66 kb fragments were cloned to construct pOsClpP5KO-Con5 and pOsClpP5KO-Con3, respectively, which are positive control vectors. These two vectors were used to make true 5' and 3' junction fragments in PCR screening of gene-targeted (GT) candidates. The full length sequence of the made targeting vector pOsClpP5KO-AcDs is indicated as SEQ ID NO: 36. The sequence of pOsClpP5KO-Ds corresponds to a sequence in which the region of position 8626 to position 9394 (the sequence of a heat-shock promoter), the region of position 9411 to position 11626 (the sequence of an AcTPase4x gene comprising an intron and an SV40 NLS), and the region of position 11665 to position 11917 (the sequence of an NOS terminator) are removed from SEQ ID NO: 36.

For vectors (pGEF1S549D-AcDs and pGEF1S549D-Ds) to introduce an S549D mutation to an OsRacGEF1 gene, 3 kb and 3.24 kb fragments were amplified from the "Kinmaze" genome for 5' and 3' homology arms, respectively. The terminal region of Ds was then fused by overlapping PCR (Figure **9B**). In this step, an S549D mutation and an adjacent BssHII site were introduced to the 5' homology arm. pGEF1S549D-Con, which is a control vector having true 5' and 3' junction fragments, was also constructed. The sequence of the made targeting vector pGEF1S549D-AcDs corresponds to a sequence in which the region of position 1593 to position 4592 (the sequence of a 5' homology arm) is substituted with the sequence set forth in SEQ ID NO: 155 and the region of position 13915 to position 16912 (the sequence of a 3' homology arm) is substituted with the sequence set forth in SEQ ID NO: 156 in SEQ ID NO: 36. The sequence of pGEF1S549D-Ds corresponds to a sequence in which the region of position 8626 to position 9394 (the sequence of a heat-shock promoter), the region of position 9411 to position 11626 (the sequence of an AcTPase4x gene comprising an intron and an SV40 NLS), and the region of position 11665 to position 11917 (the sequence of an NOS terminator) are removed from the sequence of pGEF1S549D-AcDs.

A DNA fragment comprising inducible AcTPase4xOs and a gene of a fluorescent protein was incorporated by site-specific recombination using LR clonaseII (Thermo Fisher Scientific) (Figure **3D**).

The DNA sequence of the homology arms was confirmed by Sanger sequencing using primers listed in Tables 1 and 2. [Table 1]

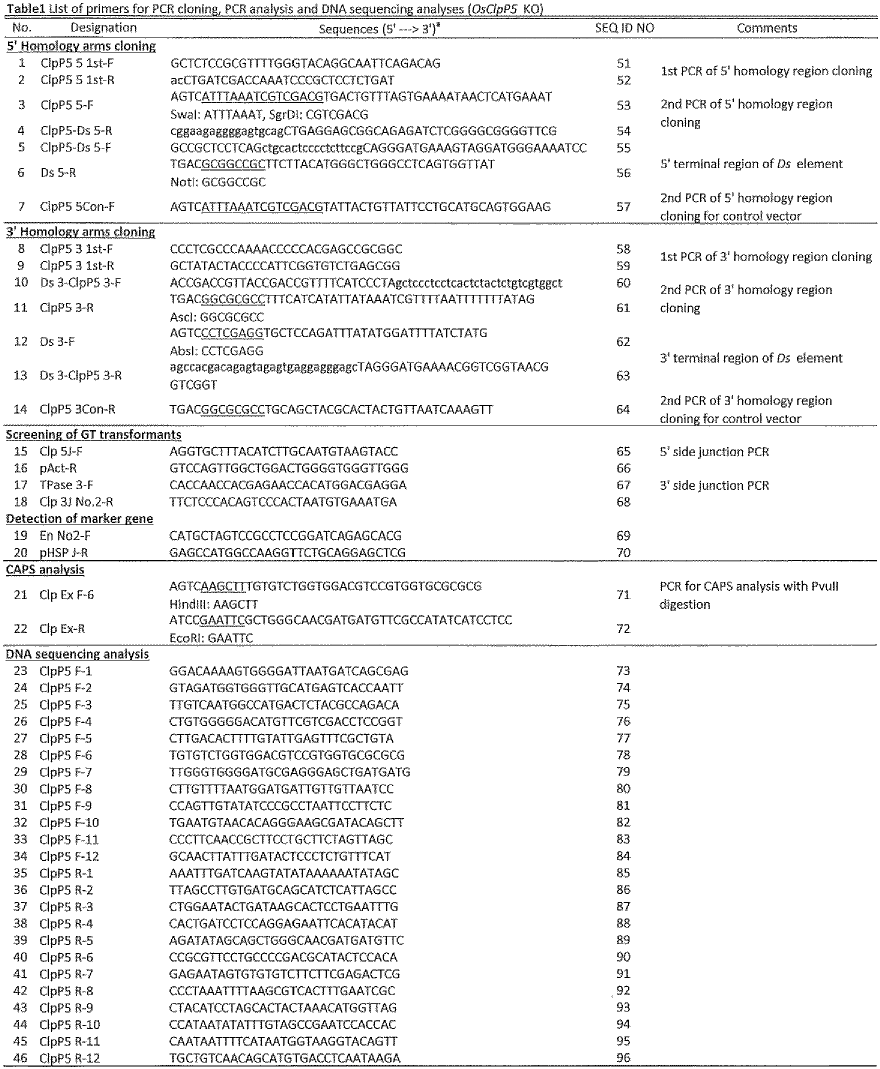

[Table 2]

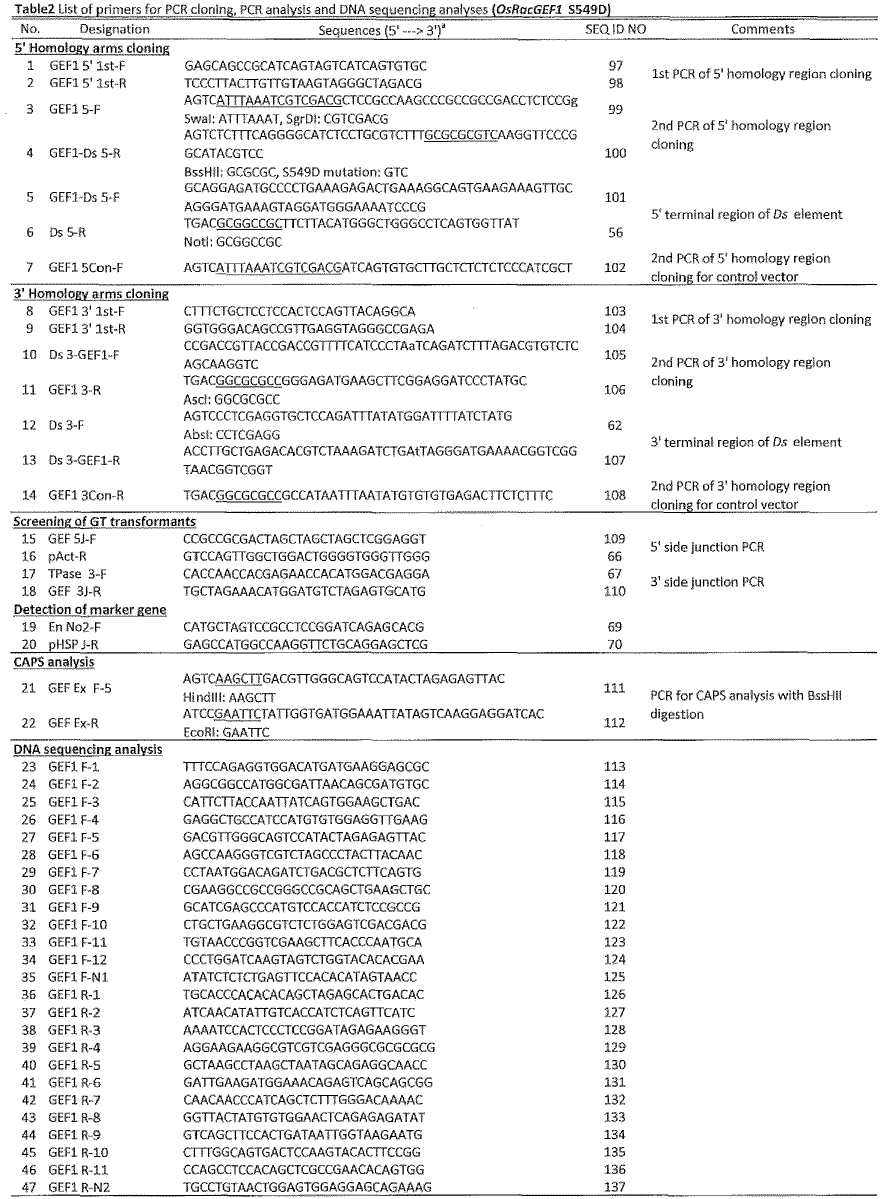

### <Transformation of rice for gene targeting>

"Nipponbare" and "Kinmaze" were used to knock out (KO) OsClpP5 and to introduce an S549D mutation to OsRacGEF1, respectively. Transformation mediated by large-scale Agrobacterium was basically performed according to prior reports (Terada R. et al., Nat Biotechnol, 20(10):1030-1034 (2002), Terada R. et al., Plant Physiol, 144(2):846-856 (2007)), but the following change was added. Germ disc-derived embryogenic calli were induced from 250 to 500 mature seeds on an N6D medium. In this case, embryogenic calli means calli that maintain a high regeneration potency. After Agrobacterium was removed, rice calli were cultured in an N6DNU medium for ten days to ensure that the Agrobacterium was removed. The rice calli were then transferred to an N6DSE-H40 medium and GT candidates were selected by positive-negative selection. Proliferative callus lines were subjected to PCR screening using an appropriate primer (Tables 1 and 2, Figures **5B** and **9C**) to identify GT callus lines. The selected callus lines were individually subcultured and subjected to heat-shock treatment at 42°C for 40, 60, or 90 minutes, thereby inducing Ac/Ds-mediated marker excision. As described below, introduction of a desired mutation and excision of a marker in the callus lines were confirmed by PCR and DNA sequencing analysis. The selected callus lines were transferred to an MSRE medium to regenerate T₀ plants. T₁ segregants obtained by self-pollination of marker-free T₀ plants were subjected to further analysis. Table 3 shows the general composition of the culture media for plant tissue used in the Examples.

**Table 3 The plant tissue culture media used in this study**

| Medium | | N6D^{*1)} | N6DNU | N6DSE-H40 | MSRE | 1/2MS |
|---|---|---|---|---|---|---|
| Application | | Callus proliferation | Agrobacterium elimination | Selection of rice calli | Regeneration of T₀ plants | Rooting of T₀ plants and seedling germination |
| Basal medium | | N6D | N6D | N6D | MS^{*2)} | 1/2MS |
| Antibiotics | Vancomycin | - | 100mg/L | 100mg/L | - | - |
| | Meropenem | - | 12.5mg/L | 12.5mg/L | - | - |
| | Hygromycin | - | - | 40mg/L | - | - |
| Gelling agent | Gelrite | 4g/L | 4g/L | 4g/L | 4g/L | 4g/L |

| | | | | | | |
|---|---|---|---|---|---|---|
| 1. Toki (1997) Rapid and efficint Agrobacterium-mediated transformation of rice. Plant Mol Biol Rep (15) 16-21 doi: 10.1007/BF02772109 2. Murashige and Skoog (1962) A Revised Medium for Rapid Growth and Bio Assays with Tobacco Cultures. Phisiol Plantar (15) 437-497 doi: 10.1111/j.1399-3054.1962.tb08052.x | | | | | | |

### <PCR and DNA sequencing analysis of GT plants>

A nucleic acid manipulation method comprising preparation of genome DNA and RNA, PCR, CAPS, and DNA sequencing analysis was performed in the same manner as a previously described method (Shimatani Z. et al., Mol Genet Genomics. 281(3):329-344 (2009), Shimatani Z. et al., Nat Biotechnol, 35(5):441-443 (2017)). The primers used in the Examples are shown in Tables 1 and 2.

First, GT candidates having an OsClpP5 KO mutation were screened by junction PCR analysis. A 4.1 kb 5' junction fragment comprising a promoter region was amplified with Clp 5J-F and pAct-R that are primers (Figure **4C** and Table 1) by using a Tks Gflex DNA polymerase (Takara Bio) according to the instructions of the manufacturer. An equimolar mixture of pOsClpP5KO-Con5 and the "Nipponbare" genome DNA that imitates the state of heterozygosis was used as a control DNA sample. Similarly, a 5.6- kb 3' junction fragment comprising a region encoding OsClpP5 was detected by PCR analysis using TPase 3-F and Clp 3J-R that are primers (Figure **4C** and Table 1). pOsClpP5KO-Con3 was used as a control. Next, the locus of a target was analyzed by CAPS analysis, and the presence of a restriction enzyme site in OsClpP5 introduced by gene targeting was confirmed. A 920 bp DNA fragment was amplified by PCR using Clp Ex F-6 and Clp Ex-R that are primers and digested using PvuII. The above-described DNA fragment was also used for direct DNA sequencing analysis to confirm a desired KO mutation in OsClpP5 and cloning sequencing analysis using Zero Blunt TOPO PCR cloning kit (Thermo Fisher Scientific) to confirm Ac/Ds-mediated positive marker excision.

Similarly, junction PCR analysis was performed to screen the callus lines of GT candidates having an S549D mutation of OsRacGEF1. Primer pairs of GEF 5J-F/pAct-R and TPase 3-F/GEF 3J-R were used to amplify a 4.2 kb 5' junction fragment and a 6.1 kb 3' junction fragment, respectively. A 980 bp DNA fragment amplified by PCR using primers GEF EX F-5 and GEF Ex-R was used for DNA sequencing analysis and CAPS analysis using BssHII.

### Example 1: An inducible-type autonomous marker excision system using artificially manipulated maize Ac/Ds

In order to evaluate the function of a heat-shock inducible Ac/Ds-mediated marker excision system, pZEN30NC and pZEN31E, which are plasmid vectors for a GUS reporter assay, were prepared (Figure **1**). These vectors were introduced to rice calli by using transformation mediated by Agrobacterium, thereby making 74 and 196 independent gene recombinant callus lines, respectively (Table 4). Furthermore, about 50 gene recombinant callus lines having pZEN30PC were also made as a control group that constitutively expresses GUSPlus. Subsequently, each callus line having pZEN30NC was analyzed as to whether Ds has been excised. As a result of PCR analysis using primers Ds Ex-F/Ds Ex-R (Table 5), no excision of Ds was detected in all gene recombinant callus lines (Table 4 and Figure **2C**). Similarly, while almost no GUS-positive signal was found in the callus lines introduced with pZEN30NC, almost all of the callus lines introduced with pZEN30PC exhibited a GUS signal (Table 4 and Figure **2D**). This result suggests that a DNA-type transposon system which has a functional compatibility with the Ac/Ds system and is activated by a tissue culture process does not exist in the rice genome.

The callus lines introduced with pZEN31E were divided into two groups. One of the groups was cultured at normal temperature (31.5°C) as a control while the other group was placed in a heated state at 42°C for 90 minutes. After nurse culture under normal conditions (31.5°C) for one week, the genome DNA of each gene recombinant callus line was extracted, and excision of Ds was detected by PCR analysis using primers Ds Ex-F/Ds Ex-R (Table 5). As a result, amplification of a 650 bp fragment exhibiting excision of Ds was observed in 159 (81.1%) and 187 (95.1%) callus lines in the control group and the heat-shock treatment group, respectively (Table 4 and Figure **2C**).

Furthermore, a GUS staining assay was performed to analyze the frequency and temporal and spatial occurrence of Ds excision by AcTPase4xOs under the control of a heat-shock promoter. The result indicates that AcTPase4xOs has a function of removing a synthesized Ds element in a rice callus. Although a Ds element was excised at a low frequency even under the normal conditions, it was revealed that heat-shock treatment increases the frequency of Ds excision mediated by AcTPase4xOs (Table 4 and Figure **2D**).

[Table 4]

**Table4 Evaluation of heat-shock inducible Ac/Ds mediated marker excision system**

| Vector | Analized | Heat-shock treatment | GUS-positive | (%) | Removal unit excision | (%) |
|---|---|---|---|---|---|---|
| pZEN30NC | 74 | - | 4 | 5.4 | 0 | 0 |
| pZEN31E | 196 | - | 182 | 92.9 | 159 | 81.1 |
| | | 42°C, 90min | 191 | 97.4 | 187 | 95.1 |

[Table 5]

**Table5 List of primers for Ac/Ds excision assay**

| No. | Designation | Sequences (5' ---> 3')^{a} | SEQ ID NO |
|---|---|---|---|
| **Ac/Ds excision assay** | | | |
| 1 | Ds Ex-F | AGTCAAGCTTCCGGAAACCTCCTCGGATTCCATTGCCCAG | 138 |
| 2 | Ds Ex-R | TGACGAATTCGACGCCATTGAGGTCGAAGACGCCACGGG | 139 |
| 3 | GUSPlus-F | ATGGTAGATCTGAGGGTAAATTTCTAGTTT | 140 |
| 4 | GUSPlus-R | TCACACGTGATGGTGATGGTGATGGCTAGC | 141 |
| 5 | hpt GT F-1 | GATATGTCCTGCGGGTAAATAGCTGCGCCG | 142 |
| 6 | t35S-R New | GGTACCCCTGGATTTTGGTTTTAGGAATTA | 143 |
| 7 | EnSpm No2-R | CGTGCTCTGATCCGGAGGCGACTAGCATG | 144 |

### Example 2: Design-based modification of a target gene by gene targeting

OsClpP5 is a rice endogenous gene encoding a P5 subunit of an ATP-dependent caseinolytic protease, and disruption of homozygosis causes a chloroplast dysfunction which leads to a leaf color mutation as in pale yellow leaves (Tsugane K. et al., Plant J. 45(1):46-57 (2006)). OsClpP5 was selected as a model to be combined with an autonomous Ac/Ds-mediated marker excision system to technically demonstrate design-based modification by gene targeting.

A gene targeting vector, pOsClpP5KO-AcDs, was designed to be introduced with a mutation in a splice site and a new PvuII site by introducing a nucleotide substitution in the 5' terminal of intron 1 of OsClpP5 (Figures **4A** and **B**). In primary modification, a "removal unit" comprising a transcription terminator (ΔEn), a positive marker (hpt) (the sequence set forth in SEQ ID NO: 44), and a visual marker (EGFP) (the sequence set forth in SEQ ID NO: 49) was inserted to OsClpP5 intron 1 via homologous recombination (Figure **4C**). In the subsequent secondary modification, the removal unit was excised out from a target region by an autonomous Ac/Ds system (Figures **5A** and **B**). The obtained transcriptional product of OsClpP5 was expected to comprise intron 1 that has not been subjected to splicing, which results in a premature termination codon. pOsClpP5KO-AcDs was introduced to embryogenic calli derived from a seed of Nipponbare via large-scale Agrobacterium transformation. Callus lines of GT candidates were selected from 67.5 g calli after positive-negative selection for three to four weeks.

In order to screen true GT (TGT) callus lines having OsClpP5 that is properly modified by homologous recombination, primer pairs (Clp 5J-F/pAct-R and TPase 3-F/Clp 3J- R, respectively) were used to perform junction PCR analysis (Figures **4C** and **D**). Among 297 GT candidates, 71 callus lines were found as a true gene target. Thus, the frequency of GT in the OsClpP5 locus was estimated to be about 23.9% per live callus (Table 6).

Sequencing analysis of a 5' junction fragment of each GT callus line revealed that a desired mutation was introduced to the position of a target of OsClpP5 together with a removal unit. Further, fluorescence microscope analysis was performed to confirm 67 GT callus lines (67/71, 94.4%) expressing an EGFP signal (Table 6 and Figure **4E**).

| Table6 Summary of GT experiments and target gene modification target:OsClpP5 (Os03g0308100) (Cultivar:Nipponbare) | | | | | | | | | | | |
|---|---|---|---|---|---|---|---|---|---|---|---|
| Number of experiments | Mutation type | Marker excision system | Fluorescent signal | Plant material for GT transformation | | Number of PN callus lines | PCR analysis | | EGFP positive | Sequencing analysis | |
| | | | | Seeds for callus | Fresh weight of calli (g) | | TGT callus lines | Frequency TGT/PN (%) | | Desired mutation | WT |
| 1 | KO | *Ac*/*Ds* | *EGFP* | 250 | 67.5 | 297 | 71 | 23.9 | 67 | ND | ND |

| target:OsRacGEF1 (OS09g0544800) (Cultivar:Kinmaze) | | | | | | | | | | | |
|---|---|---|---|---|---|---|---|---|---|---|---|
| Number of experiments | Mutation type | Marker excision system | Fluorescent signal | Plant material for GT transformation | | Number of PN callus lines | PCR analysis | | EGFP positive | Sequencing analysis | |
| | | | | Seeds for callus | Fresh weight of calli (g) | | TGT callus lines | Frequency TGT/PN (%) | | Desired mutation | WT |
| 3 | S594D | *Ac*/*Ds* | *EGFP* | 1322 | 252.5 | 574 | 59 | 10.3 | ND | ND | ND |
| 1 | S549D | *Ds* | *EGFP* | 481 | 92.3 | 113 | 13 | 11.5 | ND | ND | ND |

### Example 3: Heat-shock inducible Ac/Ds-mediated marker excision in TGT callus lines

The sturdiest 15 TGT callus lines having modified OsClpP5 were used for the following analysis in order to optimize the heat-shock condition for inducing Ac/Ds-mediated marker gene excision.

Each GT callus line was equally divided and treated at 42°C for 0, 40, 60, or 90 minutes based on the results of the above-described preliminary experiment (Figures **2A** to **D**). The treated callus lines were cultured in an N6D medium for four weeks and the frequency of secondary modification was analyzed. It was expected that EGFP expression would function as an indicator of excision of a "removal unit" in a specific cell line. It was considered that when the unit is excised out, expression of EGFP would be removed from corresponding cells and their progeny. Such a cell line, which is a product resulting from the secondary modification, can be distinguished from surrounding EGFP-expressing cells. As expected, all EGFP-positive cells were confirmed to have a "removal unit" by PCR analysis (Figure **5D**). Meanwhile, some EGFP-negative cell lines appeared in 13 or 14 TGT callus lines after treatment with heat-shock for a corresponding exposure time (Table 7 and Figure **5C**). A part of the EGFP-negative calli of each GT line was selected for PCR analysis. As a result, successful removal of a "removal unit" was confirmed in all of the analyzed EGFP-negative calli (Table 7 and Figure **5D**). These results demonstrated the secondary modification of OsClpP5 to remove a positive marker using a heat-shock inducible Ac/Ds system.

The GT callus lines that were treated or not treated with heat shock were transferred to an MSRE medium and cultured for four to six weeks until T₀ plants are regenerated. The regeneration rate of the heat-shock treated group was within the range from 56.3% to 62.5%, while the regeneration rate of the heat-shock untreated control group was 68.8% (Table 7). A sufficient number of T₀ plants were obtained from each group. These results suggest that the heat-shock step is not harmful to plant regeneration.

The regenerated T₀ plants were individually proliferated on a 1/2 MS medium to be subjected to further analysis. CAPS and DNA sequencing analysis indicate that a desired mutation has been introduced to the majority of the T₀ plants derived from the GT callus lines treated with heat shock (Figures **6A** and **B**). Furthermore, it was confirmed that almost all of such T₀ plants do not have a "removal unit" by PCR analysis and EGFP expression in root tissue (Table 8, Figures **6C** and **D**). Meanwhile, it was confirmed that the frequency of excision of a "removal unit" is clearly low in the T₀ plants derived from the heat-shock untreated group (Table 8).

Next, the influence of gene targeting and heat-shock inducible Ac/Ds-mediated marker excision on a rice plant body was studied. In order to evaluate the growth of T₀ plants, the plant height of the plants in the ear emergence period was evaluated. As a result, the plant height of T₀ plants regenerated from marker-free callus lines was confirmed to be relatively low (Figures **7A** and **B**). It is considered that this is probably due to a somatic cell mutation that occurred in the gene targeting process, and is barely associated with heat-shock treatment because the plant height of the T₀ plants regenerated from the callus lines was suppressed as compared to WT plants regardless of the presence or absence of the heat-shock treatment (Figure **7B**). Meanwhile, it seems that the plant height of the plants treated with heat shock affects the fertility of the T₀ plants. T₀ plants derived from the GT callus lines after heat-shock treatment at 42°C for 90 minutes exhibited a relatively low fertility (Figure **7C**). Thus, it was assumed that the heat-shock condition for inducing Ac/Ds-mediated marker excision is 42°C for 40 minutes that decreases the stress on rice calli as much as possible.

The above-described results demonstrated efficient design-based gene modification in rice calli by gene targeting in combination with an Ac/Ds-mediated autonomous marker excision system. In the next example, progeny of self-pollination of T₀ plants was studied to study the stability and heredity of a desired mutation.

**[Table 7]**

| **Table7** Heat-shock inducible *Ac*/*Ds* -mediated marker excision from TGT call us lines carrying modified *OsClpP5* | | | | | | |
|---|---|---|---|---|---|---|
| Treatment time | Analyzed | Marker gene excision | | | Regenerated | Frequency (%) |
| | | EGFP-negative | PCR-negative | Frequency (%) | | |
| 0 | 16 | 0 | 0 | 0.0 | 11 | 68.8 |
| 40 | | 13 | 13 | 81.3 | 9 | 56.3 |
| 60 | | 13 | 13 | 81.3 | 10 | 62.5 |
| 90 | | 14 | 14 | 87.5 | 10 | 62.5 |

**[Table 8]**

| **Table8** Genotyping of T₀ plants carrying modified *OsClpP5* | | | | | |
|---|---|---|---|---|---|
| Treatment time (min) | TGT callus lines | Analyzed | KO mutation | Marker excised | |
| | | | CAPS-positive | Marker excised | Frequency (%) |
| 0 | 7 | 73 | 63 | 14 | 28.6 |
| 40 | 5 | 69 | 66 | 66 | 100 |
| 60 | 5 | 39 | 37 | 33 | 89.2 |
| 90 | 5 | 53 | 45 | 44 | 97.8 |

### Example 4: Analysis of marker-free T₁ progeny having an OsClpP5 KO mutation

Marker-free plants of the T₀ generation obtained by two-stage mutation has an OsClpP5 KO mutation as a heterozygous recessive mutation. Thus, for the phenotype of their T₁ progeny, it was expected that the normal phenotype and the albino phenotype would be segregated at a ratio of 3:1. 1/4 of the T₁ progeny derived from each T₀ plant actually exhibited the albino phenotype (Table 9, Figures **8A** and **B**). Furthermore, the result of genotyping by CAPS and DNA sequencing analysis was consistent with the phenotype of each T₁ plant (Figures **8D** and **E**). These results demonstrated the accuracy and efficiency of modification of a target gene through gene targeting and Ac/Ds-mediated marker excision.

**[Table 9]**

| **Table9** Segregation of modified *OsClpP5* in T₁ generation | | | | |
|---|---|---|---|---|
| Line | Analyzed | Normal | | Albino |
| | | WT | Hetero | null |
| 156-2 | 18 | 5 | 7 | 6 |
| 193-19 | 18 | 3 | 10 | 5 |
| 209-6 | 19 | 7 | 7 | 4 |
| 156-5 | 20 | 8 | 8 | 4 |
| 156-1 | 19 | 7 | 10 | 2 |

### Example 5: A footprint sequence after Ac/Ds-mediated marker excision

The genome sequence of albino plants segregated in the T₁ generation was analyzed to confirm whether a target locus was properly edited after two-stage modification by gene targeting after Ac/Ds-mediated marker excision. A footprint sequence generated in association with Ac/Ds excision was observed as 1 bp insertion or 2 to 6 bp deletion (Figure **8F**). It was revealed that the plants derived from the same GT callus line share the same footprint sequence. This result means that a removal unit was excised at the very early stage after heat-shock treatment, and the T₀ plants were regenerated from proliferated calli in the same cell line.

### Example 6: Modification of OsRacGEF1

There are many receptor kinases to recognize pathogenic infection on a cell membrane of a plant. OsRacGEF1 was identified as an important component of "defensome" of rice. OsRacGEF1 is critically involved in the early stage of a chitin-driven immune response and plays an important role in a signaling pathway. In rice, chitin elicitor receptor kinase 1 (OsCERK1) and a chitin elicitor-binding protein (OsCEBiP) form a receptor complex to recognize and activate a downstream signaling pathway. OsRacGEF1 functions as a relay point of the signaling pathway. OsRacGEF1 directly interacts with OsCERK1 and is activated by phosphorylation of S549 at the C terminal thereof, thereby activating OsRac1 which is a main control factor of rice immunity. OsRacGEF1 S549D, which is a mutation that imitates the phosphorylation, was found to increase the resistance to Magnaporthe oryzae via activation of OsRac1 (Akamatsu A. et al., Cell Host Microbe, 13(4):465-476(2013)).

A gene targeting vector of OsRacGEF1 was designed so as to introduce a nucleotide substitution causing a silent mutation that causes an S549D mutation and a new BssHII site (Figures **9A** and **B**). In primary modification, a "removal unit" comprising a transcription terminator (ΔEn) and a visual marker (EGFP) was inserted to the 3'UTR region of OsRacGEF1 via homologous recombination (Figure **9B**). In the subsequent secondary modification, the removal unit is excised out from the target region by an autonomous Ac/Ds system. It was confirmed that obtained OsRacGEF1 has an S549D mutation (Figures **10A** and **B,** and Figure **11**). Furthermore, deletion of the "removal unit" without leaving a trace was confirmed (Figure **11**).

Large-scale Agrobacterium transformation and positive-negative selection were performed to introduce pGEF1S549D-AcDs to embryogenic calli derived from a seed of "Kinmaze". After repeating for three times, 1322 GT candidate callus lines were obtained from 252.5 g calli. In order to screen true GT (TGT) callus lines having OsRacGEF1 which is properly modified by HR, primer pairs (GEF 5-J-F/pAct-R and TPase 3-F/GEF 3J-R, respectively) were used to perform 5'- and 3'-junction PCR analysis (Figure **9C** and Table 2). As a result, 59 callus lines were found as a true gene target. Thus, the GT frequency in the OsRacGEF1 locus was estimated to be about 10.3% per live callus (Table 6).

As described above, pGEF1S549D-Ds was also introduced to Kinmaze. 13 callus lines among 113 candidates were confirmed to be TGT by 5'- and 3'-junction PCR analysis. The frequency of GT was 11.5% relative to the positive-negative-selected callus lines (Table 6). A desired mutation and EGFP expression were also observed.

Excision of an Ac/Ds-mediated maker from the modified OsRacGEF1 locus was subsequently performed as described above. 16 TGT callus lines having a desired mutation were equally divided and treated at 42°C for 40, 60, or 90 minutes. After nurse culture for four weeks, EGFP-negative cell lines were obtained (Figure **10C**). These calli were selected for regeneration of T₀ plants. The regeneration frequency was 37.5 to 62.5% (Table 10). As a result of PCR and CAPS analysis of T₀ plants, successful removal of a removal unit and introduction of a desired mutation to a targeted position in OsRacGEF1 were confirmed (Table 11, Figures **10D** and **E**). These T₀ plants were grown to confirm a desired mutation heredity segregation in the T₁ generation through self-pollination.

**[Table 10]**

| **Table 10** Heat-shock inducible *Ac*/*Ds*-mediated marker excision from TGT callus lines carrying modified *OsRacGEF1* | | | | | | |
|---|---|---|---|---|---|---|
| Treatment time | Analyzed | Marker gene excision | | | Regenerated | Frequency (%) |
| | | EGFP-negative | PCR-negative | Frequency (%) | | |
| 0 | 15 | 0 | ND | ND | 3 | 20.0 |
| 40 | | 15 | ND | ND | 10 | 66.7 |
| 60 | | 15 | ND | ND | 10 | 66.7 |
| 90 | | 15 | ND | ND | 6 | 40.0 |

**[Table 11]**

| **Table11** Genotyping of T₀ plants carrying modified *OsRacGEF1* | | | | | | | |
|---|---|---|---|---|---|---|---|
| Treatment time (min) | TGT callus lines | Analyzed | KO mutation | | | Marker excised | |
| | | | CAPS-positive | KO mutation | Frequency (%) | Marker excised | Frequency (%) |
| 0 | 3 | 157 | ND | ND | | 2 | 1.3 |
| 40 | 7 | 56 | 13 | ND | | 13 | 23.2 |
| 60 | 5 | 41 | 20 | ND | | 18 | 43.9 |
| 90 | 3 | 57 | 1 | ND | | 1 | 1.8 |

### Example 7: Analysis of a transcriptional product in an OsClpP5 KO variant

OsClpP5 modification in this example was designed to cause gene function disruption by splicing inhibition via base substitution. Specifically, a splicing donor site positioned on the 5' side terminal of the OsClpP5 first intron was modified to thereby modify GT to CT. It was expected that an immature termination codon would consequently appear by a frame shift mutation or the like due to the first intron sequence in the mRNA derived from the OsClpP5 gene, and a normal protein would not be translated, which would lead to disruption of the function. Thus, in order to confirm the accurate edition of a target gene and the function modification thereby, T₁ plant bodies were developed for two lines that had experienced successful OsClpP5 modification by the method of Example 2 and classified into a wild type, a heterozygosis type, and an osclpp5 homozygosis type which is an albino mutant based on the phenotype and the genotype of each individual, and the transcriptional products thereof were analyzed (Figures **14A** to **C** and Figures **15A** to **D**). First, an mRNA was isolated from each plant body, and a cDNA was obtained by a reverse transcription reaction using a primer set (Clp 5UTR-F No1; ACCACCCTCTCCCGGATAAGAGGCGCAACC (SEQ ID NO: 157) and Clp 3UTR-R No1; TGTGGAATCGCAAAACTATCTTGCCAAGCT (SEQ ID NO: 158)) set in the 5' UTR and 3' UTR regions of the OsClpP5 gene. Since a cDNA derived from the wild-type OsClpP5 is about 1.0 kb but a cDNA in the albino mutant comprises a 83 bp sequence corresponding to the first intron, the cDNA should be detected as an about 1.1 kb DNA fragment (Figures **14A****, B,** and **C**). As a result of fractionation of the OsClpP5 cDNA of each plant body by agarose gel electrophoresis, an about 1.0 kb DNA fragment and an about 1.1 kb DNA fragment were detected from the wild-type plant body and the osclpp5 homozygosis-type plant body, respectively as expected, while both DNA fragments were observed in the heterozygosis-type plant body (Figure **14C**)**.** Cloning sequence analysis was subsequently performed for the obtained cDNAs to observe a splicing out of the first intron in the mRNA derived from OsClpP5 in the wild-type individual (Figures **15A** and **B**). Meanwhile, it was confirmed that about 80 bases derived from the first intron remain in the mRNA derived from the osclpp5 homozygosis-type plant bodies (Figures **15C** and **D**). Further, appearance of an immature termination codon due to the first intron sequence was observed.

The above results demonstrated the modification of a splicing pattern of a target gene by gene targeting and the function disruption thereby.

### [Industrial Applicability]

The present invention enables minute and quick target gene modification as a plant variety improving technique. Unlike the conventional genome editing techniques, the present invention enables modification of a wide range (to a few kb), and also enables Cis genesis, knock-in modification and the like, wherein an artificial sequence such as a marker gene is autonomously removed after establishment of modification of a target gene. Furthermore, it is also possible to perform "pyramiding" for accumulating useful genes in a short period of time. Accordingly, the present invention can be expected to be applied as a practical breeding technique in variety improvement of higher plants.

The present application is on the basis of Japanese Patent Application No. 2019-030971 filed in Japan (filing date: February 22, 2019), whose entire content is encompassed herein.

## Claims

1. A nucleic acid comprising:
(1) a transposon; and
(2) a sequence homologous to at least a part of a target region of a double-stranded DNA of a plant cell,
wherein the transposon comprises a marker gene and a gene encoding a transposase operably linked to an inducible promoter.

2. The nucleic acid of claim 1, wherein the transposon is derived from a transposon selected from the group consisting of a Ds element, an Ac element, an Spm-s element, an En1 element, an Spm-I8 (dSpm) element, an Mu element, a Tam1 element, a Tam2 element, a Tam3 element, an nDart element, a Dart element, and a PiggyBac element.

3. The nucleic acid of claim 1 or 2, wherein the transposon is derived from a Ds element or an Ac element, and the transposase is AcTPase or a variant thereof.

4. The nucleic acid of any one of claims 1 to 3, wherein the marker gene includes a gene encoding a fluorescent protein.

5. The nucleic acid of any one of claims 1 to 4, wherein the inducible promoter is a heat-shock inducible promoter.

6. A method for producing plant cells that have a mutation in a target region of a double-stranded DNA and do not have any of an exogenous transposon and a gene encoding a transposase, the method comprising:
(1) the step of selecting plant cells introduced with the nucleic acid of any one of claims 1 to 5;
(2) the step of culturing the plant cells selected in step (1) under a condition wherein the inducible promoter is activated; and
(3) the step of selecting a cell in which expression of the marker protein is eliminated from a population comprising the plant cells cultured in step (2).

7. The method of claim 6, wherein the plant cells are cells of a monocotyledon.

8. The method of claim 7, wherein the monocotyledon is a gramineous plant.

9. A plant cell obtained by the method of any one of claims 6 to 8 or a plant body comprising the cell.
